(19) Europäisches Patentamt
European Patent Office
Office européen des brevets

(11) EP 2 481 816 A1

(12) EUROPEAN PATENT APPLICATION

(43) Date of publication:
**01.08.2012 Bulletin 2012/31**

(51) Int Cl.:
***C12Q 1/68*** *(2006.01)*

(21) Application number: **12153511.6**

(22) Date of filing: **01.02.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **01.02.2011 IT RM20110044**

(71) Applicant: **Consiglio Nazionale Delle Ricerche**
**00185 Roma (IT)**

(72) Inventors:
- **Gatti, Maurizio**
  **00040 Monte Porzio Catone, RM (IT)**
- **Somma, Maria Patrizia**
  **00191 Roma, RM (IT)**
- **Di Cunto, Ferdinando**
  **10127 Torino, TO (IT)**
- **Provero, Paolo**
  **10090 Cinzano, TO (IT)**
- **Damasco, Christian**
  **12042 Bra, CN (IT)**
- **Lembo, Antonio**
  **12038 Savigliano, CN (IT)**

(74) Representative: **Capasso, Olga et al**
**De Simone & Partners S.p.A.**
**Via Vincenzo Bellini, 20**
**00198 Roma (IT)**

# (54) Markers to predict survival of breast cancer patients and uses thereof

(57) The present invention relates to a method to predict the mortality risk of a subject (p) affected of breast cancer comprising measuring the expression level of 105 specific genes in a biological sample, obtaining the prognostic score, S(p), that indicates the expression levels of said genes in said subject (p) affected of cancer, and predicting the mortality risk of said subj ect (p) affected of cancer.



Printed by Jouve, 75001 PARIS (FR)

## Description

### Field of invention

The present invention relates to the construction of a gene expression signature that is highly predictive of survival in breast cancer.

### Background art

A reliable prediction of the outcome of a breast cancer is extremely valuable information for deciding a therapeutic strategy. The analysis of gene expression profiles obtained with microarrays has allowed identification of gene sets, or genetic "signatures", that are strongly predictive of poor prognosis (see [1,2] for a recent survey). In the past few years, two types of cancer signatures have been developed commonly designated as "bottom-up" or "top-down". In top-down (or supervised) signatures, the risk-predicting genes are selected by correlating the tumor's gene expression profiles with the patients' clinical outcome. One of the most powerful top-down signatures is the so-called 70-gene signature, which includes genes regulating cell cycle, invasion, metastasis and angiogenesis [3]. This signature outperforms standard clinical and histological criteria in predicting the likelihood of distant metastases within five years [4]. Although highly predictive of cancer outcome, top-down signatures have the drawback of including different gene types, thereby preventing precise definition of the biological processes altered in the tumor.

Bottom-up (or unsupervised) signatures are developed using sets of genes thought to be involved in specific cancer-related processes and do not rely on patients' gene expression data. Examples of these signatures are the "Wound signature" that includes genes expressed in fibroblasts after serum addition with a pattern reminiscent of the wound healing process [5,6], the "Hypoxia signatures" that contains genes involved in the transcriptional response to hypoxia [7-9], and the "Proliferation signatures" that include genes expressed in actively proliferating cells [10,11]. Other bottom-up signatures are the "ES signature" [12], the proliferation, immune response and RNA splicing modules signature [13] (henceforth abbreviated as "Module signature") the "invasiveness gene signature" (IGS) [14] and the chromosomal instability signature (CIN) [15]. The "ES signature" is based on the assumption that cells with tumor-initiating capability derive from normal stem cells. This signature reflects the gene expression pattern of embryonic stem cells (ES) and includes genes that are preferentially expressed or repressed in this type of cells [12]. The "Module signature" was generated by selecting gene sets that were enriched in nine pre-existing signatures, and consists of gene modules involved in 11 different processes including the immune response, cell proliferation, RNA splicing, focal adhesion, and apoptosis [13]. The IGS signature includes genes that are differentially expressed in tumorigenic breast cancer cells compared to normal breast-epithelium cells; the 186 genes of this signature are involved in a large variety of cellular functions and processes [14]. The CIN signature has features of both top-down and bottom-up signatures; it was developed by selecting genes with variations in the expression level correlated with the overall chromosomal aneuploidy of tumor samples [15].

Tumors are characterized by frequent mitotic divisions and chromosome instability. The authors thus reasoned that genes required for mitotic cell division and genes involved in the maintenance of chromosome integrity could be used to develop a new cancer signature.

In a recent RNAi-based screen performed in *Drosophila* S2 cells [16], the authors of the instant invention identified 44 genes required to prevent spontaneous chromosome breakage and 98 genes that control mitotic division. Thus, considering the strong phylogenetic conservation of the mitotic process, rather than relying on functional annotation databases, the authors used the 142 *Drosophila* genes identified in the screen [16] to develop a new bottom-up signature that includes genes involved in cell division but not yet annotated in the literature. 108 of these 142 *Drosophila* genes have unambiguous human orthologs [17]. Here the authors show that these 108 human genes constitute an excellent signature to predict breast cancer outcome. This *Drosophila mitotic signature*, or "DM signature", has minimal overlap with pre-existing gene signatures and outperforms them in predictive power.

### Description of the invention

The classification of patients with breast cancer into risk groups represents a very valuable tool for the identification of subjects who would benefit from an aggressive systemic therapy. The analysis of microarray's data allowed to generate many signatures of gene expression improving the diagnosis and allowing the risk assessment. There is also evidence that specific genes of a proliferative state would have an high predictive value within these signatures.

Thus, the authors thus constructed a gene expression signature (the DM signature) using the human orthologues of 108 *Drosophila melanogaster* genes required for either the maintenance of chromosome integrity (36 genes) or mitotic division (72 genes). The DM signature has minimal overlap with the extant signatures and is highly predictive of survival in 5 large breast cancer datasets. In addition, the authors show that the DM signature outperforms other widely used cancer signatures in predictive power, and performs comparably to other proliferation-based signatures. For most genes of the DM signature, an increased expression is negatively correlated with patient survival. The genes that provide the highest contribution to the predictive power of the DM signature are those involved in cytokinesis. This finding highlights cytokinesis as an important marker in breast cancer prognosis and as a possible target for antimitotic therapies.

It is therefore, an obj ect of the invention a method to predict the mortality risk of a subj ect (p) affected of breast cancer comprising:

a) measuring the expression level of the genes *C15orf44*, *CASP7*, *CNOT3*, *CTPS*, *CUL4B*, *CWC15*, *DCAKD*, *DDB1*, *FRGI*, *MSH6*, *ORC5L*, *PCNA*, *PIAS1*, *POLA1*, *PRIM2*, *PRPF3*, *RAD54L*, *RFC2*, *RPA1*, *RRM2*, *SART1*, *SF3A3*, *SMC1A*, *TAF6*, *TFDP2*, *TK2*, *TPR*, *TYMS*, *WBP11*, *WDR46*, *WDR75*, *XAB2*, *XRN2*, *ZMYM4*, *MCM3*, *MCM7*, *SMC3*, *NCAPD2*, *NCAPG*, *SMC4*, *SMC2*, *MASTL*, *ORC2L*, *TOP2A*, *CDT1*, *BUB3*, *KNTC1*, *ZW10*, *ASCC3L1*, *CCNB1*, *CDC40*, *DHX8*, *KIAA1310*, *LSM2*, *PRPF31*, *SF3A1*, *SF3A2*, *SF3B1*, *SF3B2*, *SF3B14*, *SLU7*, *SNRPA1*, *SNRPE*, *TXNL4A*, *U2AF1*, *U2AF2*, *ANAPC5*, *ANAPC10*, *CDC20*,, *KIN*, *PSMC1*, *SFRS15*. *CKAP5*, *EIF3A*, *EIF3D*, *EIF3E*, *EIF31*, *GTF3C3*, *MAPRE3*, *NOC3L*, *RRP1B*, *TBK1*, *THOC2*, *TUBB2C*, *WDR82*, *TRRAP*, *TUBGCP4*, *TUBG2*, *ASPM*, *CENPJ*, *MKI671P*, *PPP1R8*, *CDC2*, *KIFC1*, *KIF11*, *KIF18A*, *AURKC*, *RBBP7*, *PLK1*, *ECT2*, *KIF23*, *PRC1*, *RACGAP1*, *ANLN*, *CIT* in a biological sample, obtaining the prognostic score, S(p), that indicates the expression levels of said genes in said subj ect (p) affected of cancer, and

b) predicting the mortality risk of said subject (p) affected of cancer comparing said prognostic score, S(p), to a cut off value (cut off threshold).

Preferably the expression level of said genes is measured by means of quantitative detection of the transcript sequences selected from the group SEQ ID No 1 to SEQ ID No. 217.

Still preferably the expression level of said genes is detected by means of microarray. In a preferred embodiment the biological sample is selected from the group of: blood, tumour cell, frozen or fixed tissue sections, biopsy, biological fluid. In a still preferred embodiment the mortality risk is assigned as follows:

i) to the class "low risk" if the prognostic score, S(p), is lower than the cut off threshold, or

ii) to the class "high risk" if the prognostic score, S(p), is greater than the cut off threshold, and optionally

iii) to the class "intermediate" if the prognostic score, S(p), is between two cut off threshold values.

Still preferably the prognostic score, S(p), is calculated according to the following formula:

$$S(p) = \sum_g x(g,p) z(g)$$

wherein

*x(g,p)* is the expression level expressed in logarithmic base 2 of the probeset g in the patient *p*;

*z(g)* is the z-score of the probeset g calculated in the Pawitan dataset;

wherein the probeset g comprises a group of 217 probes, each one being specific and selective for one of the gene transcript belonging to the group of SEQ ID No. 1 to SEQ ID No. 217.

Yet preferably the z-score for each probe is the one calculated in the Pawitan database reported in table II.

It is a further object of the invention a kit to detect the transcript expression level of genes *C15orf44*, *CASP7*, *CNOT3*, *CTPS*, *CUL4B*, *CWC15*, *DCAKD*, *DDB1*, *FRG1*, *MSH6*, *ORC5L*, *PCNA*, *PIAS1*, *POLA1*, *PRIM2*, *PRPF3*, *RAD54L*, *RFC2*, *RPA1*, *RRM2*, *SART1*, *SF3A3*, *SMC1A*, *TAF6*, *TFDP2*, *TK2*, *TPR*, *TYMS*, *WBP11*, *WDR46*, *WDR75*, *XAB2*, *XRN2*, *ZMYM4*, *MCM3*, *MCM7*, *SMC3*, *NCAPD2*, *NCAPG*, *SMC4*, *SMC2*, *MASTL*, *ORC2L*, *TOP2A*, *CDT1*, *BUB3*, *KNTC1*, *ZW10*, *ASCC3L1*, *CCNB1*, *CDC40*, *DHX8*, *KIAA1310*, *LSM2*, *PRPF31*, *SF3A1*, *SF3A2*, *SF3B1*, *SF3B2*, *SF3B14*, *SLU7*, *SNRPA1*, *SNRPE*, *TXNL4A*, *U2AF1*, *U2AF2*, *ANAPC5*, *ANAPC10*, *CDC20*,, *KIN*, *PSMC1*, *SFRS15*. *CKAP5*, *EIF3A*, *EIF3D*, *EIF3E*, *EIF3I*, *GTF3C3*, *MAPRE3*, *NOC3L*, *RRP1B*, *TBKI*, *THOC2*, *TUBB2C*, *WDR82*, *TRRAP*, *TUBGCP4*, *TUBG2*, *ASPM*, *CENPJ*, *MKI67IP*, *PPP1R8*, *CDC2*, *KIFC1*, *KIF11*, *KIF18A*, *AURKC*, *RBBP7*, *PLK1*, *ECT2*, *KIF23*, *PRC1*, *RACGAP1, ANLN, CIT,*
comprising:

- for each of said genes, sequence specific amplification means to obtain amplified nucleic acids having sequences comprised in the transcribed region thereof;
- quantitative detection means of said amplified nucleic acids;
- appropriate reagents.

Preferably said amplified nucleic acids consist of :

for *C15orf44*, SEQ ID No. 145; for *CASP7*, SEQ ID No. 189; for *CNOT3*, SEQ ID No. 66 and/or SEQ ID No. 138 and/or SEQ ID No.167; for *CTPS,* SEQ ID No. 39; for *CUL4B*, SEQ ID No. 113 and/or SEQ ID No. 152 and/or SEQ ID No. 165 and/or SEQ ID No. 212; for *CWC15*, SEQ ID No. 159; for *DCAKD*, SEQ ID No. 126 and/or SEQ ID No. 140 and/or SEQ ID No. 190; for *DDB1*, SEQ ID No. 38; for *FRG1*, SEQ ID No. 195; for *MSH6,* SEQ ID No. 46 and/or SEQ ID No. 61 and /or SEQ ID No. 153 and/or SEQ ID No. 187; for *ORC5L*, SEQ ID No. 70 and/or SEQ ID No. 79 and/or SEQ ID No. 109; for *PCNA*, SEQ ID No. 51; for *PIAS1*, SEQ ID No. 211 and/or SEQ ID No. 216 and/or SEQ ID No. 217; for *POLA1*, SEQ ID No. 147; for *PRIM2*, SEQ ID No. 43 and/or SEQ ID No. 56 and/or SEQ ID No. 88; for *PRPF3*, SEQ ID No. 170; for *RAD54L*, SEQ ID No. 75; for *RFC2,* SEQ ID No. 42 and/or SEQ ID No. 48; for *RPA1*, SEQ ID No. 64 and/or SEQ ID No. 103; for *RRM2*, SEQ ID No. 3 and/or SEQ ID No. 9; for *SART1*, SEQ ID No. 124; for *SF3A3,* SEQ ID No. 201; for *SMC1A*, SEQ ID No. 115 and/or SEQ ID No. 179 and/or SEQ ID No. 207; for *TAF6*, SEQ ID No. 68;

for *TFDP2*, SEQ ID No. 86 and/or SEQ ID No. 118 and/or SEQ ID No. 210; for *TK2*, SEQ ID No. 37 and/or SEQ ID No. 156 and/or SEQ ID No. 171 and/or SEQ ID No. 172; for *TPR,* SEQ ID No. 99 and/or SEQ ID No. 108 and/or SEQ ID No. 182 and/or SEQ ID No. 204; for *TYMS,* SEQ ID No. 32 and/or SEQ ID No. 125; for *WBP11,* SEQ ID No. 65 and/or SEQ ID No. 67; for *WDR46,* SEQ ID No. 93; for *WDR75,* SEQ ID No. 158; for *XAB2,* SEQ ID No. 180; for *XRN2,* SEQ ID No. 81 and/or SEQ ID No. 84; for *ZMYM4,* SEQ ID No. 192 and/or SEQ ID No. 196 and/or SEQ ID No. 213; for *MCM3*, SEQ ID No. 34; for *MCM7*, SEQ ID No. 28 and/or SEQ ID No. 52; for *SMC3,* SEQ ID No. 185 and/or SEQ ID No. 193 and/or SEQ ID No. 209; for *NCAPD2,* SEQ ID No. 106; for *NCAPG,* SEQ ID No. 22 and/or SEQ ID No. 24; for *SMC4*, SEQ ID No. 33 and/or SEQ ID No. 54 and/or SEQ ID No. 141; for *SMC2*, SEQ ID No. 45 and/or SEQ ID No. 127; for *MASTL*, SEQ ID No. 11; for *ORC2L*, SEQ ID No. 104; for *TOP2A*, SEQ ID No. 20 and/or SEQ ID No. 62 and/or SEQ ID No. 96; for *CDT1,* SEQ ID No. 2 and/or SEQ ID No. 36; *for BUB3,* SEQ ID No. 57 and/or SEQ ID No. 139 and/or SEQ ID No. 148 and/or SEQ ID No. 174 and/or SEQ ID No. 178; for *KNTC1,* SEQ ID No. 35; for *ZW10,* SEQ ID No. 143; for *ASCC3L1,* SEQ ID No. 55 and/or SEQ ID No. 135 and/or SEQ ID No. 150; for *CCNB1,* SEQ ID No. 7 and/or SEQ ID No. 14; for *CDC40,* SEQ ID No. 100 and/or SEQ ID No. 177; for *DHX8,* SEQ ID No. 58 and/or SEQ ID No. 120 and/or SEQ ID No. 121; for *KIAA1310,* SEQ ID No. 160 and/or SEQ ID No. 183 and/or SEQ ID No. 188; for *LSM2,* SEQ ID No. 137; for *PRPF31,* SEQ ID No. 60 and/or SEQ ID No. 91 and/or SEQ ID No. 184; for *SF3A1,* SEQ ID No. 98 and/or SEQ ID No. 119 and/or SEQ ID No. 162 and/or SEQ ID No. 173; for *SF3A2,* SEQ ID No. 169 and/or SEQ ID No. 176; for *SF3B1,* SEQ ID No. 194 and/or SEQ ID No. 203 and/or SEQ ID No. 208 and/or SEQ ID No. 214; for *SF3B2,* SEQ ID No. 77; for *SF3B14,* SEQ ID No. 10; for *SLU7,* SEQ ID No. 149 and/or SEQ ID No. 151; for *SNRPA1,* SEQ ID No. 23 and/or SEQ ID No. 49 and/or SEQ ID No. 71 and/or SEQ ID No. 181; for *SNRPE,* SEQ ID No. 72 and/or SEQ ID No. 136; for *TXNL4A,* SEQ ID No. 26 and/or SEQ ID No. 134; for *U2AF1,* SEQ ID No. 30 and/or SEQ ID No. 82 and/or SEQ ID No. 102 and/or SEQ ID No. 131; for *U2AF2,* SEQ ID No. 94 and/or SEQ ID No. 146 and/or SEQ ID No. 155 and/or SEQ ID No. 161; for *ANAPC5,* SEQ ID No. 85 and/or SEQ ID No. 95 and/or SEQ ID No. 97 and/or SEQ ID No. 112 and/or SEQ ID No. 117; for *ANAPC10,* SEQ ID No. 129; for *CDC20,* SEQ ID No. 17; for *KIN,* SEQ ID No. 111 and/or SEQ ID No. 144; for *PSMC1,* SEQ ID No. 25; for *SFRS15,* SEQ ID No. 50 and/or SEQ ID No. 63 and/or SEQ ID No. 80 and/or SEQ ID No. 142 and/or SEQ ID No. 197; for *CKAP5,* SEQ ID No. 21; for *EIF3A,* SEQ ID No. 175 and/or SEQ ID No. 186 and/or SEQ ID No. 202; *for EIF3D,* SEQ ID No. 101; for *EIF3E,* SEQ ID No. 154; for *EIF3I,* SEQ ID No. 114; for *GTF3C3,* SEQ ID No. 74 and/or SEQ ID No. 163; for *MAPRE3,* SEQ ID No. 116 and/or SEQ ID No. 128 and/or SEQ ID No. 130 and/or SEQ ID No. 133; for *NOC3L,* SEQ ID No. 164; for *RRP1B,* SEQ ID No. 105 and/or SEQ ID No. 123; for *TBK1,* SEQ ID No. 198; for *THOC2,* SEQ ID No. 110 and/or SEQ ID No. 132 and/or SEQ ID No. 199 and/or SEQ ID No. 205; for *TUBB2C,* SEQ ID No. 4 and/or SEQ ID No. 5; for *WDR82,* SEQ ID No. 191; for *TRRAP,* SEQ ID No. 69 and/or SEQ ID No. 73; for *TUBGCP4,* SEQ ID No. 76 and/or SEQ ID No. 215; for *TUBG2,* SEQ ID No. 157; for *ASPM,* SEQ ID No. 6 and/or SEQ ID No. 47 and/or SEQ ID No. 53; for *CENPJ,* SEQ ID No. 87 and/or SEQ ID No. 92 and/or SEQ ID No. 107; for *MKI67IP,* SEQ ID No. 41 and/or SEQ ID No. 89 and/or SEQ ID No. 200; for *PPP1R8,* SEQ ID No. 168; for *CDC2,* SEQ ID No. 15 and/or SEQ ID No. 16 and/or SEQ ID No. 31 and/or SEQ ID No. 206; for *KIFC1,* SEQ ID No. 19; for *KIF11,* SEQ ID No. 29; for *KIF18A,* SEQ ID No. 18; for *AURKC,* SEQ ID No. 90; for *RBBP7,* SEQ ID No. 166; for *PLK1,* SEQ ID No. 27; for *ECT2,* SEQ ID No. 40 and/or SEQ ID No. 59 and/or SEQ ID No. 83; for *KIF23,* SEQ ID No. 8 and/or SEQ ID No. 44; for *PRC1,* SEQ ID No. 13; for *RACGAP1,* SEQ ID No. 12; *for ANLN,* SEQ ID No. 1; for *CIT,* SEQ ID No. 78 and/or SEQ ID No. 122.

Still preferably, the kit further comprises sequence specific amplification means to obtain amplified nucleic acids having sequences comprised in the transcribed region of genes H3F3A and/or PPAN-P2RY11 and/or KIF4.

It is a further object of the invention a microarray consisting of:

a) solid supporting means, and

b) for each of the genes *C15orf44*, *CASP7*, *CNOT3*, *CTPS*, *CUL4B*, *CWC15*, *DCAKD*, *DDB1*, *FRG1*, *MSH6*, *ORC5L*, *PCNA*, *PIAS1*, *POLA1*, *PRIM2*, *PRPF3*, *RAD54L*, *RFC2*, *RPA1*, *RRM2*, *SART1*, *SF3A3*, *SMC1A*, *TAF6*, *TFDP2*, *TK2*, *TPR*, *TYMS*, *WBP11*, *WDR46*, *WDR75*, *XAB2*, *XRN2*, *ZMYM4*, *MCM3*, *MCM7*, *SMC3*, *NCAPD2*, *NCAPG*, *SMC4*, *SMC2*, *MASTL*, *ORC2L*, *TOP2A*, *CDT1*, *BUB3*, *KNTC1*, *ZW10*, *ASCC3L1*, *CCNB1*, *CDC40*, *DHX8*, *KIAA1310*, *LSM2*, *PRPF31*, *SF3A1*, *SF3A2*, *SF3B1*, *SF3B2*, *SF3B14*, *SLU7*, *SNRPA1*, *SNRPE*, *TXNL4A*, *U2AF1*, *U2AF2*, *ANAPC5*, *ANAPC10*, *CDC20*, *KIN*, *PSMC1*, *SFRS15*. *CKAP5*, *EIF3A*, *EIF3D*, *EIF3E*, *EIF3I*, *GTF3C3*, *MAPRE3*, *NOC3L*, *RRP1B*, *TBKI*, *THOC2*, *TUBB2C*, *WDR82*, *TRRAP*, *TUBGCP4*, *TUBG2*, *ASPM*, *CENPJ*, *MKI67IP*, *PPP1R8*, *CDC2*, *KIFC1*, *KIF11*, *KJF18A*, *AURKC*, *RBBP7*, *PLK1*, *ECT2*, *KIF23*, *PRC1*, *RACGAP1*, *ANLN*, *CIT*, at least one oligonucleotide able to specifically hybridize to a sequence comprised in the transcribed region thereof. Preferably wherein the sequences comprised in the transcribed region of said genes consist of: for *C15orf44*, SEQ ID No. 145; for *CASP7*, SEQ ID No. 189; for *CNOT3*, SEQ ID No. 66 and/or SEQ ID No. 138 and/or SEQ ID No.167; for *CTPS*, SEQ ID No. 39; for *CUL4B*, SEQ ID No. 113 and/or SEQ ID No. 152 and/or SEQ ID No. 165 and/or SEQ ID No. 212; for *CWC15*, SEQ ID No. 159; for *DCAKD*, SEQ ID No. 126 and/or SEQ ID No. 140 and/or SEQ ID No. 190; for *DDB1*, SEQ ID No. 38; for *FRG1*, SEQ ID No. 195; for *MSH6*, SEQ ID No. 46 and/or SEQ ID No. 61 and /or SEQ ID No. 153 and/or SEQ ID No. 187; for *ORC5L*, SEQ ID No. 70 and/or SEQ ID No. 79 and/or SEQ ID No. 109; for *PCNA*, SEQ ID No. 51; for *PIAS1*, SEQ ID No. 211 and/or SEQ ID No. 216 and/or SEQ ID No. 217; for *POLA1*, SEQ ID No. 147; for *PRIM2,* SEQ ID No. 43 and/or SEQ ID No. 56 and/or SEQ ID No. 88; for *PRPF3*, SEQ ID No. 170; for *RAD54L*, SEQ ID No. 75; for *RFC2*, SEQ ID No. 42 and/or SEQ ID No. 48; for *RPA1*, SEQ ID No. 64 and/or SEQ ID No. 103; for RRM2, SEQ ID No. 3 and/or SEQ ID No. 9; for *SART1*, SEQ ID No. 124; for *SF3A3,* SEQ ID No. 201; for *SMC1A*, SEQ ID No. 115 and/or SEQ ID No. 179 and/or SEQ ID No. 207; for *TAF6*, SEQ ID No. 68; for *TFDP2*, SEQ ID No. 86 and/or SEQ ID No. 118 and/or SEQ ID No. 210; for *TK2*, SEQ ID No. 37 and/or SEQ ID No. 156 and/or SEQ ID No. 171 and/or SEQ ID No. 172; for *TPR*, SEQ ID No. 99 and/or SEQ ID No. 108 and/or SEQ ID No. 182 and/or SEQ ID No. 204; for *TYMS*, SEQ ID No. 32 and/or SEQ ID No. 125; for *WBP11*, SEQ ID No. 65 and/or SEQ ID No. 67; for *WDR46*, SEQ ID No. 93; for *WDR75*, SEQ ID No. 158; for *XAB2*, SEQ ID No. 180; for *XRN2*, SEQ ID No. 81 and/or SEQ ID No. 84; for *ZMYM4*, SEQ ID No. 192 and/or SEQ ID No. 196 and/or SEQ ID No. 213; for *MCM3*, SEQ ID No. 34; for *MCM7*, SEQ ID No. 28 and/or SEQ ID No. 52; for *SMC3*, SEQ ID No. 185 and/or SEQ ID No. 193 and/or SEQ ID No. 209; for *NCAPD2*, SEQ ID No. 106; for *NCAPG*, SEQ ID No.22 and/or SEQ ID No. 24; for *SMC4*, SEQ ID No. 33 and/or SEQ ID No. 54 and/or SEQ ID No. 141; for *SMC2*, SEQ ID No. 45 and/or SEQ ID No. 127; for *MASTL,* SEQ ID No. 11; for *ORC2L*, SEQ ID No. 104; for *TOP2A*, SEQ ID No. 20 and/or SEQ ID No. 62 and/or SEQ ID No. 96; for *CDT1,* SEQ ID No. 2 and/or SEQ ID No. 36; for *BUB3*, SEQ ID No. 57 and/or SEQ ID No. 139 and/or SEQ ID No. 148 and/or SEQ ID No. 174 and/or SEQ ID No. 178; for *KNTC1,* SEQ ID No. 35; for *ZW10*, SEQ ID No. 143; for *ASCC3L1*, SEQ ID No. 55 and/or SEQ ID No. 135 and/or SEQ ID No. 150; for *CCNB1*, SEQ ID No. 7 and/or SEQ ID No. 14; for *CDC40*, SEQ ID No. 100 and/or SEQ ID No. 177; for *DHX8*, SEQ ID No. 58 and/or SEQ ID No. 120 and/or SEQ ID No. 121; for *KIAA1310*, SEQ ID No. 160 and/or SEQ ID No. 183 and/or SEQ ID No. 188; for *LSM2*, SEQ ID No. 137; for *PRPF31*, SEQ ID No. 60 and/or SEQ ID No. 91 and/or SEQ ID No. 184; for *SF3A1*, SEQ ID No. 98 and/or SEQ ID No. 119 and/or SEQ ID No. 162 and/or SEQ ID No. 173; for *SF3A2*, SEQ ID No. 169 and/or SEQ ID No. 176; for *SF3B1*, SEQ ID No. 194 and/or SEQ ID No. 203 and/or SEQ ID No. 208 and/or SEQ ID No. 214; for *SF3B2*, SEQ ID No. 77; for *SF3B14*, SEQ ID No. 10; for *SLU7*, SEQ ID No. 149 and/or SEQ ID No. 151; for *SNRPA1*, SEQ ID No. 23 and/or SEQ ID No. 49 and/or SEQ ID No. 71 and/or SEQ ID No. 181; for *SNRPE*, SEQ ID No. 72 and/or SEQ ID No. 136; for *TXNL4A*, SEQ ID No. 26 and/or SEQ ID No. 134; for *U2AF1*, SEQ ID No. 30 and/or SEQ ID No. 82 and/or SEQ ID No. 102 and/or SEQ ID No. 131; for *U2AF2*, SEQ ID No. 94 and/or SEQ ID No. 146 and/or SEQ ID No. 155 and/or SEQ ID No. 161; for *ANAPC5*, SEQ ID No. 85 and/or SEQ ID No. 95 and/or SEQ ID No. 97 and/or SEQ ID No. 112 and/or SEQ ID No. 117; for *ANAPC10*, SEQ ID No. 129; for *CDC20*, SEQ ID No. 17; for *KIN*, SEQ ID No. 111 and/or SEQ ID No. 144; for *PSMC1*, SEQ ID No. 25; for *SFRS15*, SEQ ID No. 50 and/or SEQ ID No. 63 and/or SEQ ID No. 80 and/or SEQ ID No. 142 and/or SEQ ID No. 197; for *CKAP5*, SEQ ID No. 21; for *EIF3A*, SEQ ID No. 175 and/or SEQ ID No. 186 and/or SEQ ID No. 202; *for EIF3D,* SEQ ID No. 101; for *EIF3E*, SEQ ID No. 154; for *EIF3I*, SEQ ID No. 114; for *GTF3C3*, SEQ ID No. 74 and/or SEQ ID No. 163; for *MAPRE3*, SEQ ID No. 116 and/or SEQ ID No. 128 and/or SEQ ID No. 130 and/or SEQ ID No. 133; for *NOC3L*, SEQ ID No. 164; for *RRP1B*, SEQ ID No. 105 and/or SEQ ID No. 123; for *TBK1*, SEQ ID No. 198; for *THOC2*, SEQ ID No. 110 and/or SEQ ID No. 132 and/or SEQ ID No. 199 and/or SEQ ID No. 205; for *TUBB2C*, SEQ ID No. 4 and/or SEQ ID No. 5; for *WDR82*, SEQ ID No. 191; for *TRRAP*, SEQ ID No. 69 and/or SEQ ID No. 73; for *TUBGCP4,* SEQ ID No. 76 and/or SEQ ID No. 215; for *TUBG2*, SEQ ID No. 157; for *ASPM*, SEQ ID No. 6 and/or SEQ ID No. 47 and/or SEQ ID No. 53; for *CENPJ*, SEQ ID No. 87 and/or SEQ ID No. 92 and/or SEQ ID No. 107; for *MKI67IP*, SEQ ID No. 41 and/or SEQ ID No. 89 and/or SEQ ID No. 200; for *PPP1R8*, SEQ ID No. 168; for *CDC2,* SEQ ID No. 15 and/or SEQ ID No. 16 and/or SEQ ID No. 31 and/or SEQ ID No. 206; for *KIFC1*, SEQ ID No. 19; for *KIF11*, SEQ ID No. 29; for *KIF18A*, SEQ ID No. 18; for *AURKC*, SEQ ID No. 90; for *RBBP7*, SEQ ID No. 166; for *PLK1*, SEQ ID No. 27; for *ECT2*, SEQ ID No. 40 and/or SEQ ID No. 59 and/or SEQ ID No. 83; for *KIF23,* SEQ ID No. 8 and/or SEQ ID No. 44; for *PRC1*, SEQ ID No. 13; for *RACGAP1*, SEQ ID No. 12; for *ANLN*, SEQ ID No. 1; for *CIT*, SEQ ID No. 78 and/or SEQ ID No. 122.

Preferably the microarray further comprises at least one oligonucleotide able to specifically hybridize to a sequence comprised in the transcribed region of genes H3F3A and/or PPAN-P2RY11 and/or KIF4.

In the present invention the method to predict the mortality risk of a subject affected of breast cancer is also a method to predict the survival of a subject affected of breast cancer. Further the genes of the DM signature could be merged with those of other signatures to further improve risk stratification.

In the present invention, 3 cutoff values are provided, corresponding to 90%, 70% and 50% sensitivity on Miller dataset.

The cut off threshold on the prognostic score were calculated on the Miller dataset (a dataset independent from that used to develop the signature, but built on a consecutive series of patients and therefore representative of the population), and corresponds, on this dataset, to 90%, 70% and 50% sensitivity. Sensitivity is defined as the fraction of high-risk patients correctly identified by the predictor. For each cut off, the specificity is reported. The specificity was calculated on the Miller dataset and is defined as the fraction of low-risk patients correctly identified by the predictor. The cut off of 90% sensitivity = 798 (32% specificity), the cut off of 70% sensitivity = 921.8 (57% specificity) and the cut off of 50% sensitivity = 928.5 (73% specificity). These values are non-limitative example and may vary.

The present invention is illustrated by the following non limiting examples and figures.

**Fig. 1 - Predictive power of the DM signature.** Kaplan-Meier analysis using the DM signature shows significant differences in survival of patients from five independents breast cancer datasets. The curves represent the cumulative chances of survival of patients classified within two groups by the hierarchic clustering algorithm based on the correlation coefficient: lower curve - high risk patients; top curve - low risk patients.

**Fig. 2 - Predictive power of the mitotic and chromosome-integrity genes of the DM signature.** Kaplan-Meier survival analysis was performed on five breast cancer datasets using either the 34 chromosome integrity genes or the 71 mitotic genes of the DM signature represented in the Affymetrix platform. The curves represent the cumulative probabilities of survival of patients classified within two groups by the hierarchic clustering algorithm based on the correlation coefficient: lower curve - high risk patients; top curve - low risk patients.

**Fig. 3 - The DM signature outperforms 9 major signatures in predictive power.** The predictive power of signature is expressed with *P*; *P* is the P-value of the log-rank test for difference in survival probability of the two groups of patients obtained by hierarchical clustering using the genes of each signature. Colours correspond to the statistical significance: red, $P >= 0.05$; yellow, $0.05>P >= 0.01$; green, $P<0.01$. The signatures compared (DM; Proliferation of Starmans et al. [11], Module [13], CIN [15], Hypoxia of Sung et al. [8], Hypoxia of Winter et al. [9], ES [12]; 70-gene [3]; IGS [14]; Wound [5,6] are described in the text.

**Fig. 4 - Distribution of the z-scores of the genes of the DM signature compared to the distribution of z-scores of all genes represented in five breast cancer datasets.** Density = ratio between the number of the genes in a given *z*-score and the total number of genes.

**Fig. 5 - Comparative evaluation of the prognostic score of the DM signature.** The prognostic score of the DM signature is compared to those obtained from the CIN [15], Proliferation [11], IGS [14], Hypoxia [9], 70-gene [3], and Wound [5] signatures in the three datasets not used for training. The scores are used to predict outcome at five years. The bars show the areas under the ROC curves (AUC).

**Fig. 6 - Predictive power of the DM signature on a dataset of lung cancer** [18]: Kaplan-Meier survival analysis. The curves represent the cumulative probabilities of survival of patients classified within two groups by the hierarchic clustering algorithm based on the correlation coefficient: lower curve - high risk patients; top curve - low risk patients.

**Fig. 7 - Predictive power of the DM signature on a dataset of glioma** [19]: Kaplan-Meier survival analysis. The curves represent the cumulative probabilities of survival of patients classified within two groups by the hierarchic clustering algorithm based on the correlation coefficient: lower curve - high risk patients; top curve - low risk patients.

## Materials and methods

### Definition of the DM signature

The 142 *D. melanogaster* mitotic genes described in [16] were first converted into Entrez gene ids (file gene_info.gz downloaded from the Entrez Gene ftp site in June 2008). The authors then used Homologene, build 62, to obtain the 108 human orthologues that compose the DM signature. The authors considered only one-to-one orthology relationships reported in Homologene. This criterion led to the exclusion from the DM signature of several human genes that are commonly considered homologous to the *Drosophila* genes. However, the degree of homology between these human genes and their *Drosophila* counterparts was not sufficient for inclusion in Homologene.

## Breast cancer datasets

The authors used the following publicly available breast cancer datasets: NKI [4]; Pawitan [20] - Gene Expression Omnibus (GEO-) series GSE1456; Miller [21] - GEO series GSE3494; Wang [22] - GEO series GSE2034; Desmedt [23] - GEO series GSE7390; and Sotiriou [24] - GEO series GSE2990. The authors used relapse-free survival times when available, and overall survival times otherwise. Since the Sotiriou, Desmedt and Miller datasets have some patients in common, the authors merged the Sotiriou and Desmedt datasets in a single dataset, from which the authors removed the patients included in the Miller dataset. The authors refer to this combined dataset as the Sotiriou-Desmedt dataset. Normalized expression data and clinical data for the NKI dataset were obtained from http://www.rii.com/publications/2002/nejm.html. For the Affymetrix-based datasets, the authors obtained gene expression values from the raw data, using MAS 5.0 algorithm as implemented in the simpleaffy [25] package of Bioconductor [26]. For all datasets the authors considered only the probesets unambiguously assigned to one Entrez Gene ID in the platform annotation. For the Affymetrix platform, the authors used the annotation provided by the manufacturer, version 25, which allowed them to identify single or multiple probesets for 105 of the 108 DM signature genes. For the NKI dataset the authors used the annotation file provided in the website mentioned above; the correspondence between sequence accession number and Entrez gene was obtained from the Entrez gene ftp site; 98 of the 108 DM genes were thus associated with one or multiple probes.

## Dataset of patients with lung glandular cancer and of patients with glioma.

The expression data of patient with lung glandular cancer [18] were obtained from the caArray database, (https://array.nci.nih.gov/caarray) identification "jacobs-00182". The expression data of patients with glioma [19] were obtained by the GEO database, accession GSE4271. In both cases data were treated as described for the breast cancer dataset on Affymetrix platform.

The Large lung cancer dataset refers to bibliographic reference [18]. Other lung cancer dataset and also ovarian cancer refer to bibliographic reference [27].

## Determination of the predictive power of the genes in the DM signatures by clustering analysis

To determine whether the expression profiles of the genes included in the DM signature are significantly and robustly correlated with the disease outcome the authors used the following procedure on the datasets mentioned above: (a) select the microarray probes unambiguously associated to the signature genes; (b) creating two groups of patients by Pearson correlation-based hierarchical clustering, using only the expression profiles of the probes selected in step a; (c) determining by a standard log-rank test, as implemented in the *survival* library of R, whether the cumulative probability of survival is significantly different between the two groups.

## Determination of prognostic scores

For all datasets the authors divided the patients into two groups (good- and poor-outcome) based on their status at five years. The authors then calculated the prognostic scores for outcome prediction at five years using the following procedures. For the 70-gene signature, the score of a patient is the cosine-correlation of the expression profile of genes with good-prognosis found in http://www.rii.com/publications/2002/nejm.html [4]. The genes in the signature, given at as accession numbers, were translated into Entrez gene IDs and then into Affymetrix probesets using Affymetrix annotation files, version 25. The authors obtained 76 probesets for the HG-U133A platform, and 109 probesets for the HG-U133A and HG-U133B platforms considered together. Probesets corresponding to the same gene were assigned the same coefficient in the good-prognosis profile.

For the Wound and IGS signatures, the score of a patient is given by the Pearson correlation of the expression profile of the signature genes. For the Wound signature the core serum response centroid is available at http://microarray-pubs.stanford.edu/wound [5]. The genes in the signature were translated into Entrez gene ids and then into Affymetrix probesets using the procedure described above. The authors obtained 493 probesets for the HG-U133A platform, and 667 probesets for the HG-U133A and HG-U133B platforms considered together. Probesets corresponding to the same gene were assigned the same expression value in the core serum response centroid. The centroid for the IGS signature is directly given in Affymetrix probesets [14].

For the CIN [15], Proliferation [11] and Hypoxia [9] signatures, the score of a patient is the sum of the logarithmic expression of the signature genes in the patient sample. For the CIN and Proliferation signatures, the gene symbols, were translated first into Entrez gene ids and then into Affymetrix probesets as described above. The Hypoxia signature is directly given in terms of Affymetrix probesets.

For the DM signature, the prognostic score of a patient is given by:

$$S(p) = \sum_g x(g, p) z(g)$$

where the sum is over all the probesets associated to the signature, z(g) is the z-score of probeset g computed in the Pawitan dataset and *x(g,p)* is the logarithmic expression level of probeset *g* in patient *p*. The Affymetrix probesets that comprise the DM signature together with their z-scores are reported in Table II.

The authors used ROC curves to compare the scalable scores on three datasets (Miller, Wang and Sotiriou-Desmedet). The area under the curves and the related standard error were computed using the Hmisc library and programs available at http://biostat.mc.vanderbilt.edu/s/Hmisc. The Pawitan and NKI datasets were not used in this comparison because they were involved in the training of the DM and 70-gene signatures, respectively.

**Contribution of specific gene classes to the predictive power of the signature**

The contribution of each probeset g to the difference in score between poor- and good-prognosis patients is defined as:

$$\Delta s(g) = z(g)\big(P(g) - G(g)\big)$$

where *P(g) (G(g))* is the logarithmic expression of the probeset averaged on all poor (good) prognosis patients and z(g) is the z-score of the probeset. Given a subset of the DM signature (e.g. cytokinesis-related genes), the authors used a Mann-Whitney U test to compare the contribution of the probesets included in the subset to the contribution of all the other probesets.

**mRNA amplification**

The methods for obtaining and amplifying mRNA are known in the art and described for example in Sambrook et al., Molecular Cloning - A laboratory manual (2nd Ed.), vols. 1-3, Cold Spring Harbor Laboratory, Cold Spring Harbor, New York (1989) and Ausubel et al. Current Protocols in Molecular Biology vol.2, Current Protocol Publishing, New York (1994).

The RNA can be isolated from samples of tumor tissue, frozen or fixed tumor tissue sections, biopsy, biological fluid or tumor cell.

In the method, the sequence can be in any part of the transcript as indicated in Table II.

**Results**

**Generation of the DM signature**

The authors have recently carried out an RNAi-based screen to detect *Drosophila* genes required for chromosome integrity and for the fidelity of mitotic division [16]. Since these types of genes tend to be transcriptionally co-expressed, the authors first used a co-expression-based bioinformatic procedure to select a group of 1,000 genes highly enriched in mitotic functions. The authors then performed RNAi against each of these genes in *Drosophila* S2 cultured cells. Phenotypic analysis of dsRNA-treated cells allowed the identification of 142 genes representative of the entire spectrum of functions required for proper transmission of genetic information. 44 of these genes were required to prevent spontaneous chromosome breakage. The remaining 98 genes specified a variety of mitotic functions including those required for spindle assembly, chromosome segregation and cytokinesis [16]. Based on the observed RNAi phenotypes, these 142 genes were subdivided into 18 phenoclusters [16].

To construct the DM signature the authors identified the human homologues of these *Drosophila* genes, according to Homologene [17]. Both the genes required for chromosome integrity and those involved in the mitotic process turned out to be highly conserved in humans. 36 of the 44 chromosome-integrity genes and 72 of the 98 mitotic genes had clear human orthologues. These 108 human genes, and their classification according to the phenotypes associated with RNAi-mediated silencing of their *Drosophila* counterparts, are listed in Tables I and II.

**Table I.** Classification of the 108 genes of the DM signature according to the RNAi phenotypes of their *Drosophila* orthologues. The phenoclusters, indicated in bold characters, are described in detail in [16]

| RNAi phenotypes elicited by the *Drosophila* genes | Names of the human orthologues |
|---|---|
| **Chromosome integrity genes** | |
| Chromosome aberrations **(CA)** | *C15orf44*, *CASP7*, *CNOT3*, *CTPS*, *CUL4B*, *CWC15*, *DCAKD*, *DDB1*, *FRGI*, *H3F3A*, *MSH6*, *ORC5L*, *PCNA*, *PIAS1*, *PPAN-P2RY11*, *POLA1*, *PRIM2*, *PRPF3*, *RAD54L*, *RFC2*, *RPA1*, *RRM2*, *SART1*, *SF3A3*, *SMC1A*, *TAF6*, *TFDP2*, *TK2*, *TPR*, *TYMS*, *WBP11*, |
| | *WDR46*, *WDR75*, *XAB2*, *XRN2*, *ZMYM4*. |
| **Mitotic genes** | |
| Abnormal chromosome structure. **CC1,** loss of sister chromatid cohesion in heterochromatin; **CC2** and **CC3,** defective lateral and longitudinal chromosome condensation, respectively | **CC1:** *MCM3*, *MCM7*, *SMC3*.<br>**CC2:** *NCAPD2*, *NCAPG*, *SMC4*, *SMC2*.<br>**CC3:** *MASTL*, *ORC2L*, *TOP2A*. |
| Abnormal chromosome segregation. **CS1,** defective chromosome duplication; **CS2,** precocious sister chromatid separation; **CS3** and **CS4,** lack of sister chromatid separation; **CS5,** defective chromosome segregation during anaphase | **CS1:** *CDT1*.<br>**CS2:** *BUB3*, *KNTC1*, *ZW10*.<br>**CS3** and **CS4:** *ASCC3L1*, *CCNB1*, *CDC40*, *DHX8*, *KIM1310*, *LSM2*, *PRPF31*, *SF3A1*, *SF3A2*, *SF3B1*, *SF3B2*, *SF3B14*, *SLU7*, *SNRPA1*, *SNRPE*, *TXNL4A*, *U2AF1*, *U2AF2*.<br>***CS5*:** *ANAPC5*, *ANAPC10*, *CDC20*, *KIF4A*, *KIN, PSMC1, SFRS15*. |
| Abnormal spindle morphology: **SA1,** short spindles; **SA2,** spindles with a low MT density; **SA3,** poorly focused spindle poles, **SA4** miscellaneous spindle defects | **SA1:** *CKAP5, EIF3A, EIF3D, EIF3E, EIF3I, GTF3C3, MAPRE3, NOC3L, RRP1B, TBK1, THOC2, TUBB2C, WDR82.*<br>**SA2:** *TRRAP, TUBGCP4, TUBG2.*<br>**SA3:** *ASPM, CENPJ, MKI67IP, PPP1R8.*<br>**SA4:** *CDC2, KIFC1, KIF11, KIF18A.* |
| Abnormal spindle and chromosome structure: SC1, defective chromosome condensation and cytokinesis; SC2, multiple mitotic defects | SC1: *AURKC*, *RBBP7.*<br>SC2: *PLK1.* |
| Frequent cytokinesis failures: **CY1** and **CY2,** defective in early and late cytokinesis, respectively | **CY1:** *ECT2, KIF23, PRC1, RACGAP1.*<br>**CY2:** *ANLN, CIT.* |

**Table II - Ranking of the Affymetrix probesets of the DM signature according to their *z*-scores.** The Affymetrix probesets associated with the DM signature genes are ranked according to their Cox z-score computed on the training dataset (Pawitan). The contribution to the difference in score between poor and good prognosis patients in the other datesets is also reported. The phenoclusters associated with the *Drosophila* genes [16] are abbreviated as follows: CA, chromosome aberrations; CC1, loss of sister chromatid cohesion in heterochromatin; CC2 aberrant lateral chromosome condensation; CC3, aberrant longitudinal chromosome condensation; CS1, defective chromosome duplication; CS2, precocious sister chromatid separation; CS3 and CS4, lack of sister chromatid separation; CS5, defective chromosome segregation during anaphase; SA1, short spindles; SA2, spindles with a low MT density; SA3, poorly focused spindle poles; SA4 miscellaneous spindle defects; SC1, defective chromosome condensation and cytokinesis; SC2, multiple mitotic defects; SC1, defective in early cytokinesis; SC2, defective in late cytokinesis. The relative transcripts of the gene of the DM signature are also indicated according to their SEQ ID No.

| | | | | | | | | **Contribution to the difference in score** | | |
|---|---|---|---|---|---|---|---|---|---|---|
| ***Drosophila* gene symbol** | **Phenocluster** | **Human gene symbol** | **Human Gene name** | **Entrez Gene ID** | **Probeset** | **Transcript sequence ID No.** | **Pawitan z-score** | **Miller** | **Sotiriou-Desmedt** | **Wang** |
| scra | CY2 | ANLN | Anillin, actin binding protein | 54443 | 222608_s_at | SEQ ID No. 1 | 4,39 | 2,35 | - | - |
| dup | CS1 | CDT1 | Chromatin licensing and DNA replication factor 1 | 81620 | 228868_x_at | SEQ ID No. 2 | 4,17 | 2,54 | - | - |
| RnrS | CA | RRM2 | Ribonucleotide reductase M2 polypeptide | 6241 | 209773_s_at | SEQ ID No. 3 | 4,12 | 2,26 | 2,35 | 1,8 |
| betaTub 56D | SA1 | TUBB2C | Tubulin, beta 2C | 10383 | 213726_x_at | SEQ ID No. 4 | 4,06 | 0,69 | 0,54 | 0,34 |
| betaTub 56D | SA1 | TUBB2C | Tubulin, beta 2C | 10383 | 208977_x_at | SEQ ID No. 5 | 4,06 | 0,47 | 0,49 | 0,27 |
| asp | SA3 | ASPM | Asp (abnormal spindle) homolog, microcephaly associated (Drosophila) | 259266 | 219918_s_at | SEQ ID No. 6 | 3,99 | 2,56 | 1,66 | 1,93 |
| CycB | CS3 & CS4 | CCNB1 | Cyclin B1 | 891 | 214710_s_at | SEQ ID No. 7 | 3,95 | 2,43 | 2,32 | 1,18 |
| pav | CY1 | KIF23 | Kinesin family member 23 | 9493 | 204709_s_at | SEQ ID No. 8 | 3,91 | 2,51 | 1,23 | 0,98 |

(continued)

| | | | | | | | | Contribution to the difference in score | | |
|---|---|---|---|---|---|---|---|---|---|---|
| ***Drosophila* gene symbol** | **Phenocluster** | **Human gene symbol** | **Human Gene name** | **Entrez Gene ID** | **Probeset** | **Transcript sequence ID No.** | **Pawitan z-score** | **Miller** | **Sotiriou-Desmedt** | **Wang** |
| RnrS | CA | RRM2 | Ribonucleotide reductase M2 polypeptide | 6241 | 201890_at | SEQ ID No. 9 | 3,91 | 2,7 | 2,64 | 1,95 |
| CG13298 | CS3 & CS4 | SF3B 14 | Splicing factor 3B, 14 kDa subunit | 51639 | 223416_at | SEQ ID No. 10 | 3,85 | 0,38 | - | - |
| gwl | CC3 | MASTL | Microtubule associated serine/threonine kinase-like | 84930 | 228468_at | SEQ ID No. 11 | 3,73 | 0,69 | - | - |
| tum | CY1 | RACGAP 1 | Rac GTPase activating protein 1 | 29127 | 222077_s_at | SEQ ID No. 12 | 3,69 | 1,88 | 1,6 | 1,7 |
| feo | CY1 | PRC1 | Protein regulator of cytokinesis 1 | 9055 | 218009_s_at | SEQ ID No. 13 | 3,68 | 1,65 | 2,11 | 1,45 |
| CycB | CS3 &CS4 | CCNB1 | Cyclin B1 | 891 | 228729_at | SEQ ID No. 14 | 3,64 | 2,7 | - | - |
| cdc2 | SA4 | CDC2 | Cell division cycle 2, G1 to S and G2 to M | 983 | 210559_s_at | SEQ ID No. 15 | 3,57 | 1,76 | 2,08 | 0,77 |
| cdc2 | SA4 | CDC2 | Cell division cycle 2, G1 to S and G2 to M | 983 | 203213_ at | SEQ ID No. 16 | 3,55 | 2,79 | 1,9 | 1,36 |
| fzy | CS5 | CDC20 | Cell division cycle 20 homolog (S. cerevisiae) | 991 | 202870_s_at | SEQ ID No. 17 | 3,52 | 2,14 | 2,04 | 1,38 |
| Klp67A | SA4 | KIF18A | Kinesin family member 18A | 81930 | 221258_s_at | SEQ ID No. 18 | 3,49 | 2,6 | 0,51 | 0,56 |
| ncd | SA4 | KIFC1 | Kinesin family member C1 | 3833 | 209680_s_at | SEQ ID No. 19 | 3,43 | 2,36 | 0,54 | 1,08 |

(continued)

| | | | | | | | | Contribution to the difference in score | | |
|---|---|---|---|---|---|---|---|---|---|---|
| *Drosophila* gene symbol | Phenocluster | Human gene symbol | Human Gene name | Entrez Gene ID | Probeset | Transcript sequence ID No. | Pawitan z-score | Miller | Sotiriou-Desmedt | Wang |
| Top2 | CC3 | TOP2A | Topoisomerase (DNA) II alpha 170kDa | 7153 | 201292_at | SEQ ID No. 20 | 3,43 | 1,54 | 1,99 | 1,4 |
| msps | SA1 | CKAP5 | Cytoskeleton associated protein 5 | 9793 | 212832_s_at | SEQ ID No. 21 | 3,38 | 0,11 | 0,26 | 0,31 |
| CG34438 | CC2 | NCAPG | Non-SMC condensin I complex, subunit G | 64151 | 218662_s_at | SEQ ID No. 22 | 3,37 | 1,54 | 1,79 | 0,72 |
| U2A | CS3 & CS4 | SNRPA1 | Small nuclear ribonucleoprotein polypeptide A' | 6627 | 216977_x_at | SEQ ID No. 23 | 3,36 | -0,09 | 0,82 | 0,43 |
| CG34438 | CC2 | NCAPG | Non-SMC condensin I complex, subunit G | 64151 | 218663_ at | SEQ ID No. 24 | 3,36 | 2,03 | 1,57 | 1,06 |
| Pros26.4 | CS5 | PSMC1 | Proteasome (prosome, macropain) 26S subunit, ATPase, 1 | 5700 | 204219_s_at | SEQ ID No. 25 | 3,35 | 0,36 | 0,37 | 0,18 |
| CG3058 | CS3 & CS4 | TXNL4A | Thioredoxin-like 4A | 10907 | 202836_s_at | SEQ ID No. 26 | 3,27 | 0,91 | 0,52 | 0,12 |
| polo | SC2 | PLK1 | Polo-like kinase 1 (Drosophila) | 5347 | 202240 at | SEQ ID No. 27 | 3,14 | 1,18 | 0,94 | 0,4 |
| Mcm7 | CC1 | MCM7 | Minichromosome maintenance complex component 7 | 4176 | 208795_s_at | SEQ ID No. 28 | 3,13 | 0,51 | 0,6 | 0,38 |
| Klp61F | SA4 | KIF11 | Kinesin family member 11 | 3832 | 204444_at | SEQ ID No. 29 | 3,12 | 1,46 | 1,25 | 0,91 |

(continued)

| *Drosophila* gene symbol | Phenocluster | Human gene symbol | Human Gene name | Entrez Gene ID | Probeset | Transcript sequence ID No. | Pawitan z-score | Contribution to the difference in score: Miller | Sotiriou-Desmedt | Wang |
|---|---|---|---|---|---|---|---|---|---|---|
| U2af38 | CS3 & CS4 | U2AF1 | U2 small nuclear RNA auxiliary factor 1 | 7307 | 202858_at | SEQ ID No. 30 | 3,08 | 0,94 | 0,33 | 0,23 |
| cdc2 | SA4 | CDC2 | Cell division cycle 2, G1 to S and G2 to M | 983 | 203214_x_at | SEQ ID No. 31 | 3,04 | 1,29 | 1,6 | 0,61 |
| Ts | CA | TYMS | Thymidylate synthetase | 7298 | 202589_ at | SEQ ID No. 32 | 3,04 | 1,07 | 1,24 | 0,56 |
| glu | CC2 | SMC4 | Structural maintenance of chromosomes 4 | 10051 | 201663_s_at | SEQ ID No. 33 | 2,98 | 0,08 | 1,06 | 0,69 |
| Mcm3 | CC1 | MCM3 | Minichromosome maintenance complex component 3 | 4172 | 201555_at | SEQ ID No. 34 | 2,90 | 0,45 | 0,33 | -0,12 |
| rod | CS2 | KNTC1 | Kinetochore associated 1 | 9735 | 206316_s_at | SEQ ID No. 3 5 | 2,89 | -0,29 | 0,49 | 0,7 |
| dup | CS1 | CDT1 | Chromatin licensing and DNA replication factor 1 | 81620 | 209832_s_at | SEQ ID No. 36 | 2,87 | 0,64 | 0,62 | 0,24 |
| dnk | CA | TK2 | Thymidine kinase 2, mitochondrial | 7084 | 204227_s_at | SEQ ID No.37 | 2,82 | -0,13 | -0,13 | 0,04 |
| DDB1 | CA | DDB1 | Damage-specific DNA binding protein 1, 127kDa | 1642 | 208619_ at | SEQ ID No. 3 8 | 2,81 | 0,29 | -0,11 | 0,17 |
| CG6854 | CA | CTPS | CTP synthase | 1503 | 202613_ at | SEQ ID No. 39 | 2,80 | 0,34 | 0,79 | 0,46 |

(continued)

| | | | | | | | | Contribution to the difference in score | | |
|---|---|---|---|---|---|---|---|---|---|---|
| *Drosophila* gene symbol | Phenocluster | Human gene symbol | Human Gene name | Entrez Gene ID | Probeset | Transcript sequence ID No. | Pawitan z-score | Miller | Sotiriou-Desmedt | Wang |
| pbl | CY1 | ECT2 | Epithelial cell transforming sequence 2 oncogene | 1894 | 234992_x_at | SEQ ID No. 40 | 2,79 | 1,42 | - | - |
| CG6937 | SA3 | MKI67IP | MKI67 (FHA domain) interacting nucleolar phosphoprotein | 84365 | 224714_at | SEQ ID No. 41 | 2,73 | 0,41 | - | - |
| RfC40 | CA | RFC2 | Replication factor C (activator 1) 2, 40kDa | 5982 | 203696_s_at | SEQ ID No. 42 | 2,70 | 0,24 | 0,19 | 0,01 |
| DNAprim | CA | PRIM2 | Primase, DNA, polypeptide 2 (58kDa) | 5558 | 205628_at | SEQ ID No. 43 | 2,68 | 1 | 0,02 | 0,14 |
| pav | CY1 | KIF23 | Kinesin family member 23 | 9493 | 244427_at | SEQ ID No. 44 | 2,49 | 0,31 | - | - |
| SMC2 | CC2 | SMC2 | Structural maintenance of chromosomes 2 | 10592 | 204240_s_at | SEQ ID No. 45 | 2,46 | 0,22 | 0,92 | 0,28 |
| CG7003 | CA | MSH6 | MutS homolog 6 (E. coli) | 2956 | 202911_at | SEQ ID No. 46 | 2,38 | 0,24 | 0,64 | 0,29 |
| asp | SA3 | ASPM | Asp (abnormal spindle) homolog, microcephaly associated (Drosophila) | 259266 | 239002_at | SEQ ID No. 47 | 2,37 | 1,35 | - | - |

(continued)

| | | | | | | | | Contribution to the difference in score | | |
|---|---|---|---|---|---|---|---|---|---|---|
| ***Drosophila* gene symbol** | **Phenocluster** | **Human gene symbol** | **Human Gene name** | **Entrez Gene ID** | **Probeset** | **Transcript sequence ID No.** | **Pawitan z-score** | **Miller** | **Sotiriou-Desmedt** | **Wang** |
| RfC40 | CA | RFC2 | Replication factor C (activator 1) 2, 40kDa | 5982 | 1053_at | SEQ ID No. 48 | 2,33 | 0,11 | 0,22 | -0,28 |
| U2A | CS3 & CS4 | SNRPA1 | Small nuclear ribonucleoprotein polypeptide A’ | 6627 | 215722_s_at | SEQ ID No. 49 | 2,33 | 0,13 | 0,57 | 0,12 |
| CG4266 | CS5 | SFRS 15 | Splicing factor, arginine/serine-rich 15 | 57466 | 226082_s_at | SEQ ID No. 50 | 2,27 | 0,28 | - | - |
| mus209 | CA | PCNA | Proliferating cell nuclear antigen | 5111 | 201202_at | SEQ ID No.51 | 2,27 | 0,38 | 0,67 | 0,64 |
| Mcm7 | CC1 | MCM7 | Minichromosome maintenance complex component 7 | 4176 | 210983_s_at | SEQ ID No. 52 | 2,25 | 0,18 | 0,52 | -0,07 |
| asp | SA3 | ASPM | Asp (abnormal spindle) homolog, microcephaly associated (Drosophila) | 259266 | 232238_at | SEQ ID No. 53 | 2,19 | 1,03 | - | - |
| glu | CC2 | SMC4 | Structural maintenance of chromosomes | 10051 | 201664_at | SEQ ID No. 54 | 2,19 | 0,32 | 0,62 | 0,62 |
| CG5931 | CS3 & CS4 | ASCC3L1 | Activating signal cointegrator 1 complex subunit 3-like 1 | 23020 | 200058_s_at | SEQ ID No. 55 | 2,19 | 0,2 | 0,02 | -0,09 |

(continued)

| | | | | | | | | Contribution to the difference in score | | |
|---|---|---|---|---|---|---|---|---|---|---|
| *Drosophila* gene symbol | Phenocluster | Human gene symbol | Human Gene name | Entrez Gene ID | Probeset | Transcri pt sequence ID No. | Pawitan z-score | Miller | Sotiriou-Desmedt | Wang |
| DNAprim | CA | PRIM2 | Primase, DNA, polypeptide 2 (58kDa) | 5558 | 215708_s_at | SEQ ID No. 56 | 2,16 | 0,06 | 0,11 | -0,06 |
| Bub3 | CS2 | BUB3 | BUB3 budding uninhibited by benzimidazoles 3 homolog (yeast) | 9184 | 201457_x_at | SEQ ID No. 57 | 2,13 | 0,49 | -0,02 | -0,02 |
| CG8241 | CS3 & CS4 | DHX8 | DEAH (Asp-Glu-Ala-His) box polypeptide 8 | 1659 | 231184_at | SEQ ID No. 58 | 1,94 | -0,05 | - | - |
| pbl | CY1 | ECT2 | Epithelial cell transforming sequence 2 oncogene | 1894 | 219787_s_at | SEQ ID No. 59 | 1,94 | 0,73 | 0,55 | 0,48 |
| CG6876 | CS3 &CS4 | PRPF31 | PRP31 pre-mRNA processing factor 31 homolog (S. cerevisiae) | 26121 | 202407_s_at | SEQ ID No. 60 | 1,90 | 0,33 | 0,37 | -0,4 |
| CG7003 | CA | MSH6 | MutS homolog 6 (E. coli) | 2956 | 211450_s_at | SEQ ID No. 61 | 1,88 | 0,29 | 0,61 | -0,12 |
| Top2 | CC3 | TOP2A | Topoisomerase (DNA) II alpha 170kDa | 7153 | 201291_s_at | SEQ ID No. 62 | 1,78 | 1,1 | 1,3 | 0,81 |
| CG4266 | CS5 | SFRS 15 | Splicing factor, arginine/serine-rich 15 | 57466 | 233753_at | SEQ ID No. 63 | 1,76 | 0,33 | - | - |
| RpA-70 | CA | RPA1 | Replication protein A1, 70kDa | 6117 | 201529_s_at | SEQ ID No. 64 | 1,73 | -0,24 | -0,06 | -0,21 |

(continued)

| | | | | | | | | Contribution to the difference in score | | |
|---|---|---|---|---|---|---|---|---|---|---|
| ***Drosophila* gene symbol** | **Phenocluster** | **Human gene symbol** | **Human Gene name** | **Entrez Gene ID** | **Probeset** | **Transcript sequence ID No.** | **Pawitan z-score** | **Miller** | **Sotiriou-Desmedt** | **Wang** |
| CG2685 | CA | WBP11 | WW domain binding protein 11 | 51729 | 217821_s_at | SEQ ID No.65 | 1,72 | -0,43 | 0,05 | -0,24 |
| 1(2)NC1 36 | CA | CNOT3 | CCR4-NOT transcription complex, subunit 3 | 4849 | 211141_s_at | SEQ ID No. 66 | 1,68 | 0,5 | -0,03 | 0,01 |
| CG2685 | CA | WBP11 | WW domain binding protein 11 | 51729 | 217822_ at | SEQ ID No. 67 | 1,64 | -0,03 | 0,04 | 0,14 |
| Taf6 | CA | TAF6 | TAF6 RNA polymerase II, TATA box binding protein (TBP)-associated factor, 80kDa | 6878 | 203572_s_at | SEQ ID No. 68 | 1,62 | -0,06 | -0,05 | 0,06 |
| Nipped-A | SA2 | TRRAP | Transformation/ transcription domain-associated protein | 8295 | 202642_s_at | SEQ ID No. 69 | 1,61 | 0,02 | 0,01 | 0,28 |
| Orc5 | CA | ORC5L | Origin recognition complex, subunit 5-like (yeast) | 5001 | 211212_s_at | SEQ ID No. 70 | 1,58 | -0,02 | 0,08 | 0,07 |
| U2A | CS3 & CS4 | SNRPA1 | Small nuclear ribonucleoprotein polypeptide A' | 6627 | 206055_s_at | SEQ ID No. 71 | 1,56 | 0,24 | 0,24 | 0,3 |
| CG1859 1 | CS3 & CS4 | SNRPE | Small nuclear ribonucleoprotein polypeptide E | 6635 | 203316_s_at | SEQ ID No. 72 | 1,54 | 0,28 | 0,05 | 0,16 |

(continued)

| | | | | | | | | Contribution to the difference in score | | |
|---|---|---|---|---|---|---|---|---|---|---|
| ***Drosophila* gene symbol** | **Phenocluster** | **Human gene symbol** | **Human Gene name** | **Entrez Gene ID** | **Probeset** | **Transcript sequence ID No.** | **Pawitan z-score** | **Miller** | **Sotiriou-Desmedt** | **Wang** |
| Nipped-A | SA2 | TRRAP | Transformation/ transcription domain-associated protein | 8295 | 214908_s_at | SEQ ID No. 73 | 1,52 | -0,14 | -0,05 | -0,14 |
| CG8950 | SA1 | GTF3C3 | General transcription factor IIIC, polypeptide 3, 102kDa | 9330 | 218343_s_at | SEQ ID No. 74 | 1,50 | 0,07 | 0,15 | 0,06 |
| okr | CA | RAD54L | RAD54-like (S. cerevisiae) | 8438 | 204558_at | SEQ ID No. 75 | 1,49 | 0,05 | 0,26 | 0,64 |
| Grip75 | SA2 | TUBGCP4 | Tubulin, gamma complex associated protein 4 | 27229 | 211337_s_at | SEQ ID No. 76 | 1,47 | -0,1 | 0,22 | 0,01 |
| CG3605 | CS3 &CS4 | SF3B2 | Splicing factor 3b, subunit 2, 145kDa | 10992 | 200619_at | SEQ ID No. 77 | 1,44 | 0,07 | 0,05 | 0,07 |
| sti | CY2 | CIT | Citron (rho-interacting, serine/threonine kinase 21) | 11113 | 212801_at | SEQ ID No. 78 | 1,43 | 0,01 | 0,02 | 0,07 |
| Orc5 | CA | ORC5L | Origin recognition complex, subunit 5-like (yeast) | 5001 | 204957_at | SEQ ID No. 79 | 1,36 | 0,05 | 0,12 | 0,1 |
| CG4266 | CS5 | SFRS 15 | Splicing factor, arginine/serine-rich 15 | 57466 | 222311_s_at | SEQ ID No. 80 | 1,36 | -0,1 | 0,12 | 0,02 |

(continued)

| | | | | | | | | Contribution to the difference in score | | |
|---|---|---|---|---|---|---|---|---|---|---|
| ***Drosophila*** **gene symbol** | **Phenocluster** | **Human gene symbol** | **Human Gene name** | **Entrez Gene ID** | **Probeset** | **Transcript sequence ID No.** | **Pawitan z-score** | **Miller** | **Sotiriou-Desmedt** | **Wang** |
| CG1035 4 | CA | XRN2 | 5'-3' exoribonuclease 2 | 22803 | 233878_s_at | SEQ ID No. 81 | 1,30 | -0,08 | - | - |
| U2af3 8 | CS3 & CS4 | U2AF1 | U2 small nuclearRNA auxiliary factor 1 | 7307 | 242499_at | SEQ ID No. 82 | 1,27 | 0,11 | - | - |
| pbl | CY1 | ECT2 | Epithelial cell transforming sequence 2 oncogene | 1894 | 237241_at | SEQ ID No. 83 | 1,23 | 0,05 | - | - |
| CG1035 4 | CA | XRN2 | 5'-3' exoribonuclease 2 | 22803 | 223002_s_at | SEQ ID No. 84 | 1,21 | -0,07 | - | - |
| ida | CS5 | ANAPC5 | Anaphase promoting complex subunit 5 | 51433 | 208721_s_at | SEQ ID No. 85 | 1,14 | 0,03 | 0,09 | -0,15 |
| Dp | CA | TFDP2 | Transcription factor Dp-2 (E2F dimerization partner 2) | 7029 | 203588_s_at | SEQ ID No. 86 | 1,14 | 0,16 | 0,24 | -0,13 |
| Sas-4 | SA3 | CENPJ | Centromere protein J | 55835 | 220885_s_at | SEQ ID No. 87 | 1,12 | 0,03 | 0,05 | -0,05 |
| DNApri m | CA | PRIM2 | Primase, DNA, polypeptide 2 (58kDa) | 5558 | 215709_at | SEQ ID No. 88 | 1,10 | 0,02 | -0,01 | 0,04 |
| CG6937 | SA3 | MKI67IP | MKI67 (FHA domain) interacting nucleolar phosphoprotein | 84365 | 224713_at | SEQ ID No. 89 | 1,04 | 0,06 | - | - |

(continued)

| *Drosophila* gene symbol | Phenocluster | Human gene symbol | Human Gene name | Entrez Gene ID | Probeset | Transcript sequence ID No. | Pawitan z-score | Contribution to the difference in score: Miller | Sotiriou-Desmedt | Wang |
|---|---|---|---|---|---|---|---|---|---|---|
| ial | SC1 | AURKC | Aurora kinase C | 6795 | 211107_s_at | SEQ ID No. 90 | 1,02 | 0,1 | -0,02 | -0,08 |
| CG6876 | CS3 & CS4 | PRPF31 | PRP31 pre-mRNA processing factor 31 homolog (S. cerevisiae) | 26121 | 202408_s_at | SEQ ID No. 91 | 1,01 | 0,29 | 0,1 | 0,08 |
| Sas-4 | SA3 | CENPJ | Centromere protein J | 55835 | 223513_at | SEQ ID No. 92 | 0,94 | 0,03 | - | - |
| CG2260 | CA | WDR46 | WD repeat domain 46 | 9277 | 209196_at | SEQ ID No. 93 | 0,91 | 0,21 | 0,01 | -0,15 |
| U2af50 | CS3 & CS4 | U2AF2 | U2 small nuclear RNA auxiliary factor 2 | 11338 | 218382_s_at | SEQ ID No. 94 | 0,82 | 0,07 | 0,02 | -0,21 |
| ida | CS5 | ANAPC5 | Anaphase promoting complex subunit 5 | 51433 | 200098_s_at | SEQ ID No. 95 | 0,82 | 0,01 1 | 0,02 | 0,09 |
| Top2 | CC3 | TOP2A | Topoisomerase (DNA) II alpha 170kDa | 7153 | 237469_at | SEQ ID No. 96 | 0,79 | 0,24 | - | - |
| ida | CS5 | ANAPC5 | Anaphase promoting complex subunit 5 | 51433 | 208722_s_at | SEQ ID No. 97 | 0,77 | -0,1 | -0,01 | 0,06 |
| CG1694 1 | CS3 & CS4 | SF3A1 | Splicing factor 3a, subunit 1, 120kDa | 10291 | 201357_s_at | SEQ ID No. 98 | 0,71 | -0,09 | -0,01 | -0,11 |

(continued)

| | | | | | | | | Contribution to the difference in score | | |
|---|---|---|---|---|---|---|---|---|---|---|
| ***Drosophila* gene symbol** | **Phenocluster** | **Human gene symbol** | **Human Gene name** | **Entrez Gene ID** | **Probeset** | **Transcript sequence ID No.** | **Pawitan z-score** | **Miller** | **Sotiriou-Desmedt** | **Wang** |
| Mtor | CA | TPR | Translocated promoter region (to activated MET oncogene) | 7175 | 215220_s_at | SEQ ID No. 99 | 0,68 | -0,16 | -0,02 | -0,12 |
| CG6015 | CS3 &CS4 | CDC40 | Cell division cycle 40 homolog (S. cerevisiae) | 51362 | 203377_s_at | SEQ ID No. 100 | 0,65 | 0,02 | 0 | 0,1 |
| eIF-3p66 | SA1 | EIF3D | Eukaryotic translation initiation factor 3, subunit D | 8664 | 200005_at | SEQ ID No. 101 | 0,63 | 0,05 | -0,04 | -0,04 |
| U2af3 8 | CS3 & CS4 | U2AF1 | U2 small nuclear RNA auxiliary factor 1 | 7307 | 232141_at | SEQ ID No. 102 | 0,58 | 0,03 | - | - |
| RpA-70 | CA | RPA1 | Replication protein A1, 70kDa | 6117 | 201528_at | SEQ ID No. 103 | 0,57 | -0,03 | 0,01 | 0,07 |
| Orc2 | CC3 | ORC2L | Origin recognition complex, subunit 2-like (yeast) | 4999 | 204853_at | SEQ ID No. 104 | 0,56 | 0,05 | 0,01 | 0,03 |
| Nnp-1 | SA1 | RRP1B | Ribosomal RNA processing 1 homolog B (S. cerevisiae) | 23076 | 212844_at | SEQ ID No. 105 | 0,54 | 0,05 | 0,04 | 0,01 |
| CAP-D2 | CC2 | NCAPD2 | Non-SMC condensin I complex, subunit D2 | 9918 | 201774_s_at | SEQ ID No. 106 | 0,52 | 0,03 | 0,12 | -0,03 |
| Sas-4 | SA3 | CENPJ | Centromere protein J | 55835 | 234023_s_at | SEQ ID No. 107 | 0,44 | 0,06 | - | - |

(continued)

| | | | | | | | | Contribution to the difference in score | | |
|---|---|---|---|---|---|---|---|---|---|---|
| ***Drosophila*** **gene symbol** | **Phenocluster** | **Human gene symbol** | **Human Gene name** | **Entrez Gene ID** | **Probeset** | **Transcript sequence ID No.** | **Pawitan z-score** | **Miller** | **Sotiriou-Desmedt** | **Wang** |
| Mtor | CA | TPR | Translocated promoter region (to activated MET oncogene) | 7175 | 201731_s_at | SEQ ID No. 108 | 0,44 | 0 | -0,07 | 0,01 |
| Orc5 | CA | ORC5L | Origin recognition complex, subunit 5-like (yeast) | 5001 | 211213_at | SEQ ID No. 109 | 0,44 | 0,09 | 0 | -0,01 |
| tho2 | SA1 | THOC2 | THO complex 2 | 57187 | 226628_at | SEQ ID No. 110 | 0,40 | 0 | - | - |
| kin17 | CS5 | KIN | KIN, antigenic determinant of recA protein homolog (mouse) | 22944 | 205664_at | SEQ ID No. 111 | 0,34 | 0,03 | 0,04 | 0,01 |
| ida | CS5 | ANAPC5 | Anaphase promoting complex subunit 5 | 51433 | 211036_x_at | SEQ ID No. 112 | 0,33 | -0,02 | 0 | 0,06 |
| cul-4 | CA | CUL4B | Cullin 4B | 8450 | 210257_x_at | SEQ ID No. 113 | 0,29 | -0,03 | 0,03 | 0 |
| Trip1 | SA1 | EIF3I | Eukaryotic translation initiation factor 3, subunit I | 8668 | 208756_at | SEQ ID No. 114 | 0,26 | 0,01 | -0,01 | -0,04 |
| SMC1 | CA | SMC1A | Structural maintenance of chromosomes 1A | 8243 | 201589_at | SEQ ID No. 115 | 0,26 | 0,03 | 0,05 | 0,05 |

(continued)

| | | | | | | | | Contribution to the difference in score | | |
|---|---|---|---|---|---|---|---|---|---|---|
| ***Drosophila* gene symbol** | **Phenocluster** | **Human gene symbol** | **Human Gene name** | **Entrez Gene ID** | **Probeset** | **Transcri pt sequence ID No.** | **Pawitan z-score** | **Miller** | **Sotiriou-Desmedt** | **Wang** |
| Eb1 | SA1 | MAPRE3 | Microtubule-associated protein, RP/EB family, member 3 | 22924 | 203842_s _at | SEQ ID No. 116 | 0,25 | -0,01 | 0,01 | -0,02 |
| ida | CS5 | ANAPC5 | Anaphase promoting complex subunit 5 | 51433 | 239651_at | SEQ ID No. 117 | 0,25 | -0,01 | - | - |
| Dp | CA | TFDP2 | Transcription factor Dp-2 (E2F dimerization partner 2) | 7029 | 203589_s _at | SEQ ID No. 118 | 0,25 | -0,03 | 0,01 | -0,01 |
| CG1694 1 | CS3 & CS4 | SF3A1 | Splicing factor 3a, subunit 1, 120kDa | 10291 | 227516_at | SEQ ID No. 119 | 0,19 | -0,06 | - | - |
| CG8241 | CS3 &CS4 | DHX8 | DEAH (Asp-Glu-Ala-His) box polypeptide 8 | 1659 | 227079 at | SEQ ID No. 120 | 0,15 | 0 | - | - |
| CG8241 | CS3 & CS4 | DHX8 | DEAH (Asp-Glu-Ala-His) box polypeptide 8 | 1659 | 203334_at | SEQ ID No. 121 | 0,08 | -0,03 | 0 | 0 |
| sti | CY2 | CIT | Citron (rho-interacting, serine/threonine kinase 21) | 11113 | 242872_at | SEQ ID No. 122 | 0,07 | 0,02 | - | - |
| Nnp-1 | SA1 | RRP1B | Ribosomal RNA processing 1 homolog B (S. cerevisiae) | 23076 | 212846_at | SEQ ID No. 123 | 0,06 | 0,02 | 0,01 | 0,01 |

(continued)

| | | | | | | | | Contribution to the difference in score | | |
|---|---|---|---|---|---|---|---|---|---|---|
| *Drosophila* gene symbol | Phenocluster | Human gene symbol | Human Gene name | Entrez Gene ID | Probeset | Transcript sequence ID No. | Pawitan z-score | Miller | Sotiriou-Desmedt | Wang |
| CG6686 | CA | SART1 | Squamous cell carcinoma antigen recognized by T cells | 9092 | 200051_at | SEQ ID No. 124 | 0,02 | 0 | 0 | |
| Ts | CA | TYMS | Thymidylate synthetase | 7298 | 217684_at | SEQ ID No. 125 | 0,01 | 0 | 0 | 0 |
| CG1939 | CA | DCAKD | Dephospho-CoA kinase domain containing | 79877 | 221225_at | SEQ ID No. 126 | 0,01 | 0 | 2,29E-05 | 0 |
| SMC2 | CC2 | SMC2 | Structural maintenance of chromosomes 2 | 10592 | 213253_ at | SEQ ID No. 127 | -0,03 | -0,01 | -0,01 | 0 |
| Eb1 | SA1 | MAPRE3 | Microtubule-associated protein, RP/EB family, member 3 | 22924 | 214270_s _at | SEQ ID No. 128 | -0,05 | 0,01 | 0 | 0 |
| CG1141 9 | CS5 | ANAPC1 0 | Anaphase promoting complex subunit 10 | 10393 | 207845_s _at | SEQ ID No. 129 | -0,07 | 0 | -0,01 | -0,01 |
| Eb1 | SA1 | MAPRE3 | Microtubule-associated protein, RP/EB family, member 3 | 22924 | 203841_x _at | SEQ ID No. 130 | -0,08 | 0,01 | 0 | 0 |
| U2af38 8 | CS3 & CS4 | U2AF1 | U2 small nuclear RNA auxiliary factor 1 | 7307 | 231904_at | SEQ ID No. 131 | -0,10 | -0,01 | - | - |
| tho2 | SA1 | THOC2 | THO complex 2 | 57187 | 226626_ at | SEQ ID No. 132 | -0,11 | 0 | - | - |

(continued)

| | | | | | | | | Contribution to the difference in score | | |
|---|---|---|---|---|---|---|---|---|---|---|
| *Drosophila* gene symbol | Phenocluster | Human gene symbol | Human Gene name | Entrez Gene ID | Probeset | Transcript sequence ID No. | Pawitan z-score | Miller | Sotiriou-Desmedt | Wang |
| Eb1 | SA1 | MAPRE3 | Microtubule-associated protein, RP/EB family, member 3 | 22924 | 229682_at | SEQ ID No. 133 | -0,13 | -0,03 | - | - |
| CG3058 | CS3 & CS4 | TXNL4A | Thioredoxin-like 4A | 10907 | 202835_ at | SEQ ID No. 134 | -0,13 | -0,02 | -0,01 | -0,01 |
| CG5931 | CS3 & CS4 | ASCC3L 1 | Activating signal cointegrator 1 complex subunit 3-like 1 | 23020 | 232931_at | SEQ ID No. 135 | -0,15 | -0,02 | - | - |
| CG1859 1 | CS3 & CS4 | SNRPE | Small nuclear ribonucleoprotein polypeptide E | 6635 | 231112_at | SEQ ID No. 136 | -0,15 | 0,03 | - | - |
| CG1041 8 | CS3 & CS4 | LSM2 | LSM2 homolog, U6 small nuclear RNA associated (S. cerevisiae) | 57819 | 209449_at | SEQ ID No. 137 | -0,19 | -0,01 | -0,01 | 0,01 |
| 1(2)NC1 36 | CA | CNOT3 | CCR4-NOT transcription complex, subunit 3 | 4849 | 203239_s _at | SEQ ID No. 138 | -0,20 | -0,03 | 0,01 | 0,01 |
| Bub3 | CS2 | BUB3 | BUB3 budding uninhibited by benzimidazoles 3 homolog (yeast) | 9184 | 209974_s _at | SEQ ID No. 139 | -0,21 | -0,05 | 0,01 | -0,03 |
| CG1939 | CA | DCAKD | Dephospho-CoA kinase domain containing | 79877 | 221224_s _at | SEQ ID No. 140 | -0,24 | 0,06 | -0,01 | 0,09 |
| glu | CC2 | SMC4 | Structural maintenance of chromosomes 4 | 10051 | 215623_x _at | SEQ ID No. 141 | -0,24 | -0,02 | -0,03 | -0,05 |

(continued)

| | | | | | | | | Contribution to the difference in score | | |
|---|---|---|---|---|---|---|---|---|---|---|
| ***Drosophila*** **gene symbol** | **Phenocluster** | **Human gene symbol** | **Human Gene name** | **Entrez Gene ID** | **Probeset** | **Transcript sequence ID No.** | **Pawitan z-score** | **Miller** | **Sotiriou-Desmedt** | **Wang** |
| CG4266 | CS5 | SFRS 15 | Splicing factor, arginine/serine-rich 15 | 57466 | 243759_at | SEQ ID No. 142 | -0,28 | -0,02 | - | - |
| mit(1)15 | CS2 | ZW10 | ZW10, kinetochore associated, homolog (Drosophila) | 9183 | 204812_at | SEQ ID No. 143 | -0,32 | 0,01 | 0,01 | 0 |
| kin17 | CS5 | KIN | KIN, antigenic determinant of recA protein homolog (mouse) | 22944 | 236887_at | SEQ ID No. 144 | -0,34 | 0,03 | - | - |
| CG4785 | CA | C15orf44 | Chromosome 15 open reading frame 44 | 81556 | 221265_s_at | SEQ ID No. 145 | -0,34 | -0,02 | 0 | 0 |
| U2af50 | CS3 & CS4 | U2AF2 | U2 small nuclear RNA auxiliary factor 2 | 11338 | 229508_at | SEQ ID No. 146 | -0,35 | -0,08 | - | - |
| DNApol alpha18 0 | CA | POLA1 | Polymerase (DNA directed), alpha 1, catalytic subunit | 5422 | 204835_at | SEQ ID No. 147 | -0,37 | 0,05 | -0,03 | -0,01 |
| Bub3 | CS2 | BUB3 | BUB3 budding uninhibited by benzimidazoles 3 homolog (yeast) | 9184 | 229827_at | SEQ ID No. 148 | -0,37 | -0,09 | - | - |
| CG1420 | CS3 & CS4 | SLU7 | SLU7 splicing factor homolog (S. cerevisiae) | 10569 | 231718_at | SEQ ID No. 149 | -0,38 | -0,02 | - | - |

(continued)

| | | | | | | | | Contribution to the difference in score | | |
|---|---|---|---|---|---|---|---|---|---|---|
| ***Drosophila* gene symbol** | **Phenocluster** | **Human gene symbol** | **Human Gene name** | **Entrez Gene ID** | **Probeset** | **Transcript sequence ID No.** | **Pawitan z-score** | **Miller** | **Sotiriou-Desmedt** | **Wang** |
| CG5931 | CS3 & CS4 | ASCC3L1 | Activating signal cointegrator 1 complex subunit 3-like 1 | 23020 | 214982_at | SEQ ID No. 150 | -0,38 | 0,1 | -0,01 | -0,02 |
| CG1420 | CS3 & CS4 | SLU7 | SLU7 splicing factor homolog (S. cerevisiae) | 10569 | 227990_at | SEQ ID No. 151 | -0,41 | 0,03 | - | - |
| cul-4 | CA | CUL4B | Cullin 4B | 8450 | 202213_s_at | SEQ ID No. 152 | -0,43 | 0,03 | -0,02 | 0,01 |
| CG7003 | CA | MSH6 | MutS homolog 6 (E. coli) | 2956 | 240148_at | SEQ ID No. 153 | -0,45 | -0,05 | - | - |
| Int6 | SA1 | EIF3E | Eukaryotic translation initiation factor 3, subunit E | 3646 | 208697_s_at | SEQ ID No. 154 | -0,45 | -0,03 | -0,03 | -0,03 |
| U2af50 | CS3 & CS4 | U2AF2 | U2 small nuclear RNA auxiliary factor 2 | 11338 | 214171_s_at | SEQ ID No. 155 | -0,48 | 0,05 | -0,01 | 0,02 |
| dnk | CA | TK2 | Thymidine kinase 2, mitochondrial | 7084 | 204277_s_at | SEQ ID No. 156 | -0,49 | 0,11 | 0,01 | 0,07 |
| gamma Tub23C | SA2 | TUBG2 | Tubulin, gamma 2 | 27175 | 203894_at | SEQ ID No. 157 | -0,53 | 0,02 | 0 | 0,01 |
| CG1205 0 | CA | WDR75 | WD repeat domain 75 | 84128 | 224721_at | SEQ ID No. 158 | -0,54 | -0,02 | - | - |
| C12.1 | CA | CWC15 | CWC15 homolog (S. cerevisiae) | 51503 | 223067_at | SEQ ID No. 159 | -0,55 | -0,03 | - | - |
| CG8233 | CS3 & CS4 | KIAA1310 | KIAA1310 | 55683 | 224318_s_at | SEQ ID No. 160 | -0,56 | 0,05 | - | - |

(continued)

| | | | | | | | | Contribution to the difference in score | | |
|---|---|---|---|---|---|---|---|---|---|---|
| *Drosophila* gene symbol | Phenocluster | Human gene symbol | Human Gene name | Entrez Gene ID | Probeset | Transcript sequence ID No. | Pawitan z-score | Miller | Sotiriou-Desmedt | Wang |
| U2af50 | CS3 & CS4 | U2AF2 | U2 small nuclear RNA auxiliary factor 2 | 11338 | 218381_s_at | SEQ ID No. 161 | -0,56 | -0,14 | 0 | -0,08 |
| CG1694 1 | CS3 & CS4 | SF3A1 | Splicing factor 3a, subunit 1, 120kDa | 10291 | 216457_s_at | SEQ ID No. 162 | -0,56 | 0,05 | 0 | -0,01 |
| CG8950 | SA1 | GTF3C3 | General transcription factor IIIC, polypeptide 3, 102kDa | 9330 | 222604_at | SEQ ID No. 163 | -0,57 | 0,01 | - | - |
| CG1234 | SA1 | NOC3L | Nucleolar complex associated 3 homolog (S. cerevisiae) | 64318 | 218889_at | SEQ ID No. 164 | -0,57 | -0,04 | -0,02 | 0,03 |
| cul-4 | CA | CUL4B | Cullin 4B | 8450 | 215997_s_at | SEQ ID No. 165 | -0,63 | 0 | -0,01 | 0 |
| Caf1 | SC1 | RBBP7 | Retinoblastoma binding protein 7 | 5931 | 201092_at | SEQ ID No. 166 | -0,64 | -0,08 | -0,07 | -0,06 |
| 1(2)NC1 36 | CA | CNOT3 | CCR4-NOT transcription complex, subunit 3 | 4849 | 229143_at | SEQ ID No. 167 | -0,71 | -0,07 | - | - |
| NiPp1 | SA3 | PPP1R8 | Protein phosphatase 1, regulatory (inhibitor) subunit 8 | 5511 | 207830_s_at | SEQ ID No. 168 | -0,71 | 0,02 | 0 | 0 |
| CG1075 4 | CS3 & CS4 | SF3A2 | Splicing factor 3a, subunit 2, 66kDa | 8175 | 209381_x_at | SEQ ID No. 169 | -0,73 | -0,14 | 0,06 | 0,07 |

(continued)

| | | | | | | | | Contribution to the difference in score | | |
|---|---|---|---|---|---|---|---|---|---|---|
| *Drosophila* gene symbol | Phenocluster | Human gene symbol | Human Gene name | Entrez Gene ID | Probeset | Transcript sequence ID No. | Pawitan z-score | Miller | Sotiriou-Desmedt | Wang |
| CG7757 | CA | PRPF3 | PRP3 pre-mRNA processing factor 3 homolog (S. cerevisiae) | 9129 | 202251_at | SEQ ID No. 170 | -0,74 | 0,13 | -0,04 | -0,11 |
| dnk | CA | TK2 | Thymidine kinase 2, mitochondrial | 7084 | 240300_at | SEQ ID No. 171 | -0,76 | -0,04 | - | - |
| dnk | CA | TK2 | Thymidine kinase 2, mitochondrial | 7084 | 204276_at | SEQ ID No. 172 | -0,76 | 0,17 | 0,02 | 0,05 |
| CG1694 1 | CS3 & CS4 | SF3A1 | Splicing factor 3a, subunit 1, 120kDa | 10291 | 201356_at | SEQ ID No. 173 | -0,77 | 0,11 | -0,01 | 0,04 |
| Bub3 | CS2 | BUB3 | BUB3 budding uninhibited by benzimidazoles 3 homolog (yeast) | 9184 | 201458_s_at | SEQ ID No. 174 | -0,83 | -0,06 | 0,04 | -0,08 |
| eIF3-S10 | SA1 | EIF3A | Eukaryotic translation initiation factor 3, subunit A | 8661 | 200595_s_at | SEQ ID No. 175 | -0,84 | 0 | 0,04 | 0 |
| CG1075 4 | CS3 &CS4 | SF3A2 | Splicing factor 3a, subunit 2, 66kDa | 8175 | 37462_i_at at | SEQ ID No. 176 | -0,84 | -0,09 | 0,1 | 0,02 |
| CG6015 | CS3 &CS4 | CDC40 | Cell division cycle 40 homolog (S. cerevisiae) | 51362 | 203376_at | SEQ ID No. 177 | -0,93 | 0 | 0,09 | -0,09 |

(continued)

| | | | | | | | | Contribution to the difference in score | | |
|---|---|---|---|---|---|---|---|---|---|---|
| *Drosophila* gene symbol | Phenocluster | Human gene symbol | Human Gene name | Entrez Gene ID | Probeset | Transcript sequence ID No. | Pawitan z-score | Miller | Sotiriou-Desmedt | Wang |
| Bub3 | CS2 | BUB3 | BUB3 budding uninhibited by benzimidazoles 3 homolog (yeast) | 9184 | 201456_s_at | SEQ ID No. 178 | -0,96 | -0,03 | 0,1 | -0,08 |
| SMC1 | CA | SMC1A | Structural maintenance of chromosomes 1A | 8243 | 239688_at | SEQ ID No. 179 | -1,01 | -0,21 | - | - |
| CG6197 | CA | XAB2 | XPA binding protein 2 | 56949 | 218110_at | SEQ ID No. 180 | -1,05 | -0,1 | 0,12 | 0,11 |
| U2A | CS3 & CS4 | SNRPA1 | Small nuclear ribonucleoprotein polypeptide A' | 6627 | 242146_at | SEQ ID No. 181 | -1,11 | 0,06 | - | - |
| Mtor | CA | TPR | Translocated promoter region (to activated MET oncogene) | 7175 | 228709_at | SEQ ID No. 182 | -1,16 | -0,03 | - | - |
| CG8233 | CS3 & CS4 | KIAA1310 | KIAA1310 | 55683 | 220950_s_at | SEQ ID No. 183 | -1,18 | -0,18 | 0,09 | 0,09 |
| CG6876 | CS3 & CS4 | PRPF31 | PRP31 pre-mRNA processing factor 31 homolog (S. cerevisiae) | 26121 | 214380_at | SEQ ID No. 184 | -1,19 | 0,17 | -0,04 | 0,01 |
| Cap | CC1 | SMC3 | Structural maintenance of chromosomes | 9126 | 209259_s_at | SEQ ID No. 185 | -1,26 | -0,04 | -0,09 | -0,09 |

(continued)

| | | | | | | | | Contribution to the difference in score | | |
|---|---|---|---|---|---|---|---|---|---|---|
| ***Drosop hila* gene symbol** | **Phenocluster** | **Human gene symbol** | **Human Gene name** | **Entrez Gene ID** | **Probeset** | **Transcri pt sequence ID No.** | **Pawitan z-score** | **Miller** | **Sotiriou-Desmedt** | **Wang** |
| eIF3-S10 | SA1 | EIF3A | Eukaryotic translation initiation factor 3, subunit A | 8661 | 200597_at | SEQ ID No. 186 | -1,36 | 0,06 | 0,14 | -0,03 |
| CG7003 | CA | MSH6 | MutS homolog 6 (E. coli) | 2956 | 211449_at | SEQ ID No. 187 | -1,38 | -0,2 | 0,01 | 0,02 |
| CG8233 | CS3 & CS4 | KIAA131 0 | KIAA1310 | 55683 | 223756_at | SEQ ID No. 188 | -1,39 | -0,27 | - | - |
| Dcp-1 | CA | CASP7 | Caspase 7, apoptosis-related cysteine peptidase | 840 | 207181_s _at | SEQ ID No. 189 | -1,47 | -0,11 | 0,15 | 0,14 |
| CG1939 | CA | DCAKD | Dephospho-CoA kinase domain containing | 79877 | 224522_s _at | SEQ ID No. 190 | -1,54 | 0,17 | - | - |
| CG1729 3 | SA1 | WDR82 | WD repeat domain 82 | 80335 | 201934_at | SEQ ID No. 191 | -1,55 | -0,1 | 0,24 | 0,02 |
| woc | CA | ZMYM4 | Zinc finger, MYM-type 4 | 9202 | 202049_s _at | SEQ ID No. 192 | -1,58 | 0,01 | 0,1 | 0,1 |
| Cap | CC1 | SMC3 | Structural maintenance of chromosomes 3 | 9126 | 209257_s _at | SEQ ID No. 193 | -1,62 | 0,18 | -0,1 | 0,04 |
| CG2807 | CS3 & CS4 | SF3B1 1 | Splicing factor 3b, subunit 1, 155kDa | 23451 | 201071_x _at | SEQ ID No. 194 | -1,63 | 0,16 | 0,03 | -0,02 |
| CG6480 | CA | FRG1 | FSHD region gene 1 | 2483 | 204145_at | SEQ ID No. 195 | -1,69 | -0,19 | -0,13 | -0,01 |
| WOC | CA | ZMYM4 | Zinc finger, MYM-type 4 | 9202 | 202050_s _at | SEQ ID No. 196 | -1,69 | 0,14 | 0,15 | -0,03 |
| CG4266 | CS5 | SFRS 15 | Splicing factor, arginine/serine-rich 15 | 57466 | 222310_at | SEQ ID No. 197 | -1,71 | -0,14 | -0,09 | -0,15 |

(continued)

| | | | | | | | | Contribution to the difference in score | | |
|---|---|---|---|---|---|---|---|---|---|---|
| *Drosophila* gene symbol | Phenocluster | Human gene symbol | Human Gene name | Entrez Gene ID | Probeset | Transcript sequence ID No. | Pawitan z-score | Miller | Sotiriou-Desmedt | Wang |
| ik2 | SA1 | TBK1 | TANK-binding kinase 1 | 29110 | 218520_at | SEQ ID No. 198 | -1,71 | 0,15 | -0,22 | -0,1 |
| tho2 | SA1 | THOC2 | THO complex 2 | 57187 | 212994_at | SEQ ID No. 199 | -1,75 | -0,01 | -0,1 | 0,29 |
| CG6937 | SA3 | MKI67IP | MKI67 (FHA domain) interacting nucleolar phosphoprotein | 84365 | 234167_at | SEQ ID No. 200 | -1,76 | 0,44 | - | - |
| noi | CA | SF3A3 | Splicing factor 3a, subunit 3, 60kDa | 10946 | 203818_s_at | SEQ ID No. 201 | -1,92 | 0,02 | -0,03 | 0,17 |
| eIF3-S10 | SA1 | EIF3A | Eukaryotic translation initiation factor 3, subunit A | 8661 | 200596_s_at | SEQ ID No. 202 | -1,95 | -0,09 | 0,09 | 0,34 |
| CG2807 | CS3 & CS4 | SF3B1 | Splicing factor 3b, subunit 1, 155kDa | 23451 | 214305_s_at | SEQ ID No. 203 | -2,14 | 0,13 | 0,1 | 0,11 |
| Mtor | CA | TPR | Translocated promoter region (to activated MET oncogene) | 7175 | 201730_s_at | SEQ ID No. 204 | -2,29 | -0,01 | 0,13 | -0,02 |
| tho2 | SA1 | THOC2 | THO complex 2 | 57187 | 222122_s_at | SEQ ID No. 205 | -2,29 | 0,06 | -0,23 | -0,03 |
| cdc2 | SA4 | CDC2 | Cell division cycle 2, G1 to S and G2 to M | 983 | 231534_at | SEQ ID No. 206 | -2,31 | -0,02 | - | - |
| SMC1 | CA | SMC1A | Structural maintenance of chromosomes 1A | 8243 | 217555_at | SEQ ID No. 207 | -2,31 | -0,11 | -0,05 | 0,06 |

(continued)

| | | | | | | | | Contribution to the difference in score | | |
|---|---|---|---|---|---|---|---|---|---|---|
| ***Drosophila* gene symbol** | **Phenocluster** | **Human gene symbol** | **Human Gene name** | **Entrez Gene ID** | **Probeset** | **Transcript sequence ID No.** | **Pawitan z-score** | **Miller** | **Sotiriou-Desmedt** | **Wang** |
| CG2807 | CS3 & CS4 | SF3B1 | Splicing factor 3b, subunit 1, 155kDa | 23451 | 211185_s_at | SEQ ID No. 208 | -2,57 | 0,08 | 0,06 | -0,03 |
| Cap | CC1 | SMC3 | Structural maintenance of chromosomes 3 | 9126 | 209258_s_at | SEQ ID No. 209 | -2,62 | -0,09 | -0,2 | 0,26 |
| Dp | CA | TFDP2 | Transcription factor Dp-2 (E2F dimerization partner 2) | 7029 | 226157_at | SEQ ID No. 210 | -2,67 | 0,5 | - | - |
| Su(var)2-10 | CA | PIAS1 | Protein inhibitor of activated STAT, 1 | 8554 | 217864_s_at | SEQ ID No. 211 | -2,71 | 0,13 | 0,05 | 0,36 |
| cul-4 | CA | CUL4B | Cullin 4B | 8450 | 202214_s_at | SEQ ID No. 212 | -2,96 | 0,29 | -0,17 | 0,27 |
| woc | CA | ZMYM4 | Zinc finger, MYM-type 4 | 9202 | 202051_s_at | SEQ ID No. 213 | -3,10 | -0,04 | 0,22 | -0,09 |
| CG2807 | CS3 &CS4 | SF3B1 | Splicing factor 3b, subunit 1, 155kDa | 23451 | 201070_x_at | SEQ ID No. 214 | -3,46 | 0,27 | 0,04 | 0,22 |
| Grip75 | SA2 | TUBGCP4 | Tubulin, gamma complex associated protein 4 | 27229 | 213266_at | SEQ ID No. 215 | -3,56 | 0,68 | 0,52 | -0,01 |
| Su(var)2-10 | CA | PIAS1 | Protein inhibitor of activated STAT, 1 | 8554 | 217862_at | SEQ ID No. 216 | -3,84 | 0,45 | 0,09 | 0,35 |
| Su(var)2-10 | CA | PIAS1 | Protein inhibitor of activated STAT, 1 | 8554 | 217863_at | SEQ ID No. 217 | -4,30 | 0,2 | 0 | -0,15 |

Collectively, the genes in Table I constitute the DM signature. The remaining 34 *Drosophila* genes identified in the screen [16] were not included in the DM signature because they did not have an unambiguous human homologue in Homologene (Release 62). The DM signature shares very few genes with pre-existing signatures. We considered the top-down 70-gene signature [3] and several bottom-up signatures based on various aspects of cancer biology: the Wound signature [5,6]: the ES signature [12]; the IGS signature [14] the Hypoxia signatures of Sung et al. [8] and Winter et al. [9]; the Proliferation signature of Starmans et al. [11]; the proliferation/immune response/RNA splicing (Module) signature [13] and the chromosomal instability (CIN) signature [15]. The number of genes that the DM signature shares with the 70-gene, ES, IGS, Wound and Hypoxia signatures is extremely small. The overlap is higher with the Module, Proliferation and CIN signatures, but none of these signatures shares more that 20% of its genes with the DM signature (Table III).

**Table III.** The DM signature shares very few genes with other major cancer signatures

| ***Signature*** | ***# of genes in the signature*** | ***Genes in common with the DM signature*** |
|---|---|---|
| Module | 261 | 18 (6.9%) |
| CIN | 71 | 14 (19.7) |
| ES | 1029 | 14 (1.4%) |
| Wound | 371 | 6 (1.6%) |
| Proliferation | 52 | 6 (11.5%) |
| 70-gene | 61 | 2 (3.3%) |
| Hypoxia (Winter) | 92 | 2 (2.2%) |
| IGS | 175 | 2 (1,1%) |
| Hypoxia (Sung) | 126 | 1 (0.8%) |

Of the 108 human genes, 25 are included in the list of genes periodically expressed during the cell cycle in HeLa cells {pmid: 12058064}, compared to 5.8 expected by chance (P=2.2E-10): therefore, as expected, the human orthologs of genes that display a mitotic phenotype in the fly tend to be regulated by the cell cycle also in human.

For each dataset and each signature the same analysis as the one shown in Fig. 1 was performed and the value of P log-rank was compared to that calculated for the DM signature. In agreement with previous studies, the vast majority of the signatures show a good predictive value in the majority of the datasets (Fig. 3). The signature DM has a higher performance (in terms of P-value in the log-rank test) when compared to all other signatures in the majority of datasets (Fig. 3). Further, the DM signature has a statistically significant predictive power in all datasets and the lower overall P-value.

**The prognostic value of the DM signature**

For assessment of the predictive power and robustness of the DM signature the authors used six publicly available breast cancer datasets: (i) NKI, which contains expression data from primary breast tumors for 295 consecutive, relatively young (age < 52 yrs) patients [4]; (ii) Pawitan, which includes data from 159 consecutive breast cancer patients [20]; (iii) Miller, with data from 251 patients selected from a consecutive series based on the quality of the material [21]; (iv) Desmedt and (v) Wang, which contains expression data from 198 and 286 lymph-node negative, systemically untreated patients, respectively [22,23]; (vi) Sotiriou, which includes 189 invasive breast carcinomas [24]. Due to the presence of common samples, the authors merged the Desmedt and Sotiriou datasets into a single one and removed from it the patients that were also included in the Miller dataset. All datasets contain both ER-positive and ER-negative samples.

Although most of these gene expression data were generated using the same microarray platform, and could in principle be merged in a single dataset as recently described [13], the authors evaluated the DM signature on the individual datasets. The authors chose this approach because the robustness of a gene signature on independent datasets is an important criterion for validation of its predictive power. In the authors' prognostic power analysis, they used relapse-free survival times when available, or overall survival times otherwise. Because three genes of the DM signature (*H3F3A, PPAN-P2RY11 and KIF4*) were not represented in the Affymetrix platform, the authors performed their analyses on 105 genes. For each dataset, patients were divided into two groups based on the expression profiles of the genes in the DM signature using hierarchical clustering. Differences in survival probability between the two groups were then evaluated with a standard log-rank test on Kaplan-Meier curves. Fig. 1 shows that the differences in survival are statistically significant for all datasets considered.

As mentioned above, the DM signature contains two broad classes of genes, namely 72 mitotic genes (71 in platform) and 36 genes required for the maintenance of chromosome integrity (34 in platform). To determine the relative contribution of these two gene classes to the predictive power of the DM signature, the authors performed the analysis using the two categories of genes separately. Both gene groups turned out to be independently predictive of survival (Fig. 2). However the predictive power of the global signature was higher in all cases.

The authors also asked whether the DM signature is predictive of survival in other tumors besides breast cancer. Using the hierarchical clustering approach described above, the authors found that the DM signature is predictive of survival in a large lung cancer dataset [18] (P=3e-6, Fig. 6) and in a glioma dataset [19] (P=0.0170, Fig. 7). However, the DM signature is not significantly predictive in other lung cancer [27] and glioma [28] datasets, or in renal [29] and ovarian [27] cancer datasets. The p-values of the log-rank tests for non-breast datasets are reported in Table IV.

**Table IV - Predictive power of the DM signature in cancers other than breast. The p-**values obtained from the log-rank test when comparing the cumulative probability of survival of clusters of patients in other types of cancer.

| dataset | Log (p-value) | |
|---|---|---|
| Glioma (Freij e) | 1,77 ** | * P > 0.05 |
| Glioma (Phillips) | 0,27 * | ** 0.05 > P > 0.01 |
| Lung (Bild) | 0,21 * | ***P<0.01 |
| Lung (Shedden) | 3,52 *** | |
| Ovarian (Bild) | 0,57 * | |
| Renal (Zhao) | 1,12 * | |

### Evaluation of a prognostic score for the DM signature

Subdivision of patients into risk groups using the unsupervised clustering-based approach described above allows assessment of the predictive power of a gene signature, but does not allow specificity (fraction of low-risk patients correctly classified) and sensitivity (fraction of high-risk patients correctly classified) to be tuned according to specific requirements. However, such tuning is important in clinical applications, because the misclassification of a high-risk patient is potentially more harmful than the misclassification of a low-risk patient. Indeed, the 70-gene signature [3], which is used in clinical practice, assigns a risk score to each patient; patients are then classified based on a score threshold that can be tuned to obtain the desired compromise between specificity and sensitivity. Scalable prognostic scores, each computed from gene expression data with a specific algorithm, have been previously defined also for the Wound [6], IGS [14], Proliferation [11], CIN [15] and Hypoxia [9] signatures.

The authors determined a scalable prognostic score for the DM signature, using a procedure similar to that employed by Wang and co-workers [22]. The authors define the DM prognostic score as the sum of the logarithmic expression values of the signature genes, each multiplied by its z-score. The Cox z-score measures the correlation between the expression pattern of a gene and survival of the patient. A positive (negative) z-score indicates negative (positive) correlation between the gene expression level and patient's survival time.

The authors used the Pawitan dataset as training set and computed the Cox z-scores for the Affymetrix probesets associated to the DM signature (the z-scores of all probesets are shown in Table II). The distribution of these z-scores is consistently shifted towards positive values compared to the distribution of the z-scores of all genes represented on the microarrays (P-values between 1.1e-6 and 3.3e-15 from one-sided Mann-Whitney U test) (Fig. 4). Thus, as expected for proliferation-related genes, for most genes in the DM signature an increased expression level is negatively correlated with survival.

The authors then compared the DM signature score with the scores of 6 other scalable signatures for performance in predicting cancer outcome at 5 years. For this analysis the authors used ROC curves generated with the Affymetrix datasets not employed for training (Miller, Sotiriou-Desmedt and Wang). The scores of the CIN [15], Proliferation [11], 70-gene [3], Wound [6], IGS [14], and Hypoxia [9] signatures were computed as described in the respective references, after mapping the genes to the Affymetrix platform (see Methods for details). As shown in Fig. 5, the predictive power of the 3 proliferation-based signatures (DM, CIN and Proliferation), measured by the Area Under ROC Curves (AUC), is very similar in all datasets and systematically higher than that of the 70-gene, Wound, IGS, or Hypoxia signature.

Since the DM signature and the two other proliferation-based signatures perform similarly in predicting outcome at 5 years, as shown by the AUC values in Fig. 5, the authors compared their performance in greater detail at three values of the sensitivity (percentage of poor-outcome patients that are classified correctly by the signature). The results are shown in Tab. V.

**Table V. Comparison of the performance of the proliferation-based signatures**

| *90% sensitivity* | DM | | CIN | | Proliferation | |
|---|---|---|---|---|---|---|
| | P value | Spec. | P value | Spec. | P value | Spec. |
| Miller | **2.26E-04** | 0.318 | 5.44E-04 | **0.352** | 4.89E-04 | **0.352** |
| Sotiriou-Desmedt | **4.44E-03** | **0.335** | 0.0312 | 0.329 | 0.0124 | 0.329 |
| Wang | **4.08E-03** | 0.226 | 0.0114 | **0.260** | 0.015 | 0.227 |
| *70% sensitivity* | DM | | CIN | | Proliferation | |
| | P value | Spec. | P value | Spec. | P value | Spec. |
| Miller | **1.77E-04** | **0.614** | 7.63E-03 | 0.523 | 3.02E-03 | 0.562 |
| Sotiriou-Desmedt | 4.51E-04 | **0.613** | **4.25E-04** | 0.600 | 1.24E-03 | 0.574 |
| Wang | **4.25E-04** | **0.547** | 5.58E-04 | **0.547** | 1.19E-03 | 0.536 |
| | | | | | | |
| *50% sensitivity* | DM | | CIN | | Proliferation | |
| | P value | Spec. | P value | Spec. | P value | Spec. |
| Miller | **3.91E-04** | **0.733** | 8.81E-04 | 0.705 | 1.42E-03 | 0.716 |
| Sotiriou-Desmedt | 0.138 | 0.697 | **0.134** | **0.722** | 0.161 | 0.690 |
| Wang | 6.85E-03 | 0.669 | **2.41E-03** | **0.691** | 0.022 | 0.641 |

Tab. V reports for each signature and each dataset the specificity (percentage of correct classifications among patients classified as poor-outcome), and the P-value of the log-rank test between the two groups of patients. These two parameters have different interpretations: while the specificity refers to the ability of the signature to predict the outcome specifically at the 5-years endpoint, the P-value takes into account the complete survival data, and thus measures the ability to stratify the patients over the whole time range.

The results show that the DM and CIN signatures tend to perform better than the Proliferation one at all tested sensitivity values. DM performs slightly better than CIN at higher sensitivities, especially in terms of P-value. These differences in performance between the three signatures are driven by percentages of discordantly classified patients ranging from ~2% to ~10%. The number of discordantly classified patients in the three datasets is reported in Table VI.

**Table VI: Cox multivariate analysis for various breast cancer datasets. The DM score is** a predictor of survival independent of clinical and histological parameters commonly used in patient stratification. The table shows the odd-ratio and P-value obtained from a Cox multivariate analysis of survival including the DM score and several other predictors of survival as covariates. **NKI dataset**

| **Covariate** | **Odd ratio (95% C.I.)** | **P-value** |
|---|---|---|
| DM score (range 0-10) | 1.27 (1.13-1.43) | 9,84E-005 |
| ER (pos=1, neg=0) | 0.56 (0.33-0.96) | 0,036 |
| St Gallen (1=low risk, 0-high risk) | 0.33 (0.04-2.51) | 0,28 |
| LN (positive =1, negative = 0) | 0.84 (0.53-1.32) | 0,45 |
| NIH (1=low risk, 0-high risk) | 0.60 (0.08-4.52) | 0,62 |

**Sotiriou-Desmedt dataset**

| **Covariate** | **Odd ratio (95% C.I.)** | **P-value** |
|---|---|---|
| DM score (range 0-10) | 1.25 (1.08-1.46) | 3,00E-003 |
| Size (cm) | 1.23 (0.97-1.57) | 0,093 |

(continued)

| Covariate | Odd ratio (95% C.I.) | P-value |
|---|---|---|
| Grade (1-3) | 0.79 (0.56-1.09) | 0,15 |
| ER (pos=1, neg=0) | 1.16 (0.67-2.00) | 0,58 |
| Age (years) | 1.00 (0.97-1.02) | 0,72 |
| LN (positive =1, negative = 0) | 1.13 (0.34-3.74) | 0,84 |
| **Wang dataset** | | |
| DM score (range 0-10) | 1.23 (1.09-1.38) | 9,80E-004 |
| ER (pos=1, neg=0) | 1.31 (0.81-2.11) | 0,27 |

The authors also performed multivariate Cox analysis to ascertain whether the DM score predicts survival independently of other molecular and histological tumor markers. In all datasets, the DM score is a predictor independent of the available clinical parameters. The results for the Miller dataset, which is the richest in clinical annotation, are reported in Table VII, and the ones for the other datasets in Tables VIII.

**Table VII:** Multivariate Cox analysis for Miller dataset

| Covariate | Odd ratio (95% C.I.) | P-value |
|---|---|---|
| LN (positive=1, negative=0) | 2.82 (1.53-5.21) | 8.95E-04 |
| DM score (range 0-10) | 1.32 (1.08-1.60) | 0.0057 |
| Size (mm) | 1.04 (1.01-1.06) | 0.0065 |
| ER (positive=1, negative=0) | 3.34 (1.11-10.00) | 0.031 |
| Age (years) | 1.02 (1.00-1.04) | 0.057 |
| PGR (positive=1, negative=0) | 0.53 (0.23-1.23)) | 0.14 |
| P53 (mutant=1, wt=0) | 0.97 (0.49-1.95)) | 0.95 |
| Grade (1-3) | 0.99 (0.56-1.75) | 0.96 |
| Multivariate Cox analysis for the Miller dataset shows that the DM score is predictive of survival independently of several other predictors. | | |

**Table VIII: Number of patients discordantly classified by the three proliferation-based** signatures. For each dataset and pair of proliferation-based signatures, the authors report the number of patients classified in different outcome groups, using score cutoffs corresponding to the same sensitivity.

**90% sensitivity**

| | DM | CIN | Proliferation |
|---|---|---|---|
| DM | 0 | 20 (7.25%) | 24 (8.7%) |
| CIN | | 0 | 14 (5.1%) |
| Proliferation | | | 0 |

**70% sensitivity**

| | DM | CIN | Proliferation |
|---|---|---|---|
| DM | 0 | 24 (8.7%) | 30 (10.9%) |
| CIN | | 0 | 24 (8.7%) |
| Proliferation | | | 0 |

(continued)

| 50% sensitivity | | | |
|---|---|---|---|
| | DM | CIN | Proliferation |
| DM | 0 | 10 (3.62%) | 23 (8.33%) |
| CIN | | 0 | 21 (7.61 %) |
| Proliferation | | | 0 |

Multivariate Cox analysis on the Miller dataset using the other proliferation-based signatures gives very similar results, shown in Table IX.

**Table IX: Cox multivariate analysis for other proliferation-based signatures.** For the CIN and proliferation signature we report the results of the Cox multivariate analysis using the signature score and various other predictors of survival as covariates.

| **CIN signature** | | |
|---|---|---|
| **Covariate** | **Odd ratio (95% C.I.)** | **P-value** |
| LN | 2.86 (1.54-5.29) | 8,64E-004 |
| Size | 1.04 (1.01-1.06) | 5,09E-003 |
| CIN score | 1.26 (1.07-1.49) | 6,29E-003 |
| ER | 3.26 (1.09-9.74) | 0,034 |
| Age | 1.02 (1.00-1.04) | 0,055 |
| PGR | 0.54 (0.23-1.26) | 0,16 |
| Grade | 1.01 (0.57-1.79) | 0,98 |
| P53 | 1.00 (0.51-1.99) | 0,99 |

| **Proliferation signature** | | |
|---|---|---|
| **Covariate** | **Odd ratio (95% C.I.)** | **P-value** |
| LN | 2.78 (1.51-5.15) | 1,08E-003 |
| Size | 1.04 (1.01-1.07) | 5,00E-003 |
| Proliferation score | 1.28 (1.07-1.53) | 6,65E-003 |
| ER | 3.39 (1.13-10.19) | 0,03 |
| Age | 1.02 (1.00-1.04) | 0,072 |
| PGR | 0.53 (0.23-1.24) | 0,14 |
| P53 | 0.97 (0.49-1.93) | 0,93 |
| Grade | 0.98 (0.55-1.75) | 0,95 |

Lymph-node negative patients are a group of particular clinical significance: therefore the authors computed the AUC under ROC curves for the DM signature as a predictor of 5-year survival in the Miller and Sotiriou-Desmedt datasets limited to this subgroup. In both cases the authors find AUC values similar to the ones found for the entire dataset (AUC resp. 0.616 and 0.678). The Wang dataset includes lymph-node negative patients only.

Contribution of specific genes and gene classes to the predictive power of the DM signature.

The authors next asked whether any of the phenotypic class identified by the RNAi screen (chromosome condensation, chromosome integrity, chromosome segregation, spindle assembly and cytokinesis) [6] is especially relevant in separating poor- from good-prognosis patients. The authors computed the contribution of each probeset in the DM signature to the difference in score between poor- and good-outcome patients (see Methods); the authors then compared the contribution of specific gene classes to the total score of the 105 genes of the DM signature. For the three Affymetrix datasets not used as training, the cytokinesis genes *(ANLN, CIT, ECT2, KIF23, PRC1, RACGAP1*) turned out to contribute, as a group, significantly more than other genes to the difference in score (P-values between 0.0025 and 0.012, two-sided Mann-Whitney U test). The function of these genes is highly conserved, as they are required for cytokinesis in both *Drosophila* and humans (reviewed in [30]). Interestingly, high z-scores were also observed for *ASPM, KIF18A* and *PLK1* (respectively, 3.99, 3.49 and 3.14). The *Drosophila* homologues of these genes (*asp, Klp67 and polo*) play role in multiple mitotic stages and are required for cytokinesis [30]. In addition there is evidence that *ASPM* and *PLK1* are involved in human cell cytokinesis [30]. Thus, it appears that cytokinesis genes have higher prognostic value than other mitotic genes and genes required for chromosome integrity.

In the DM signature, there are a few genes whose reduced expression is negatively correlated with survival (Table II). The gene with the most negative z-score is *PIASl* (*z* = - 4.07, averaged on two probesets), an E3 ligase involved in sumoylation of DNA repair proteins including BRCA1 [31]. Remarkably, the expression of this gene is substantially reduced in colon cancers [32].

The authors have shown that the DM signature is highly predictive of survival in five major breast cancer datasets. The DM signature contains two classes of genes required for cell proliferation: genes that maintain the integrity of mitotic chromosomes and genes that mediate mitotic division. Cell proliferation-associated genes have been previously used to construct several unsupervised signatures, and large subsets of this type of genes are included in most supervised signatures [33]. Thus, it has been suggested that genes required for cell proliferation may underlie the prognostic power of many cancer signatures [33].

In agreement with such expectations the authors found that the DM signature has a predictive power for breast cancer outcome similar to two other proliferation-based signatures (the CIN signature [15] and the Proliferation signature of Starmans et al. [11]), and outperforms 4 additional published signatures that contain different proportions of proliferation-related genes, including the supervised 70-gene signature, which is currently used in clinical practice for breast cancer patients [3]. Altogether, these results indicate that signatures enriched in proliferation genes are the most powerful predictors of breast cancer outcome.

High performance of the DM signature may reflect its specifically high content in genes truly involved in cell proliferation. The proliferation-associated genes in the other signatures have been selected on the basis of their periodic expression pattern during the cell cycle and include several genes that, although periodically expressed, are not involved in basic cell cycle processes [10,33]. In contrast, genes underlying either the maintenance of chromosome integrity or mitosis are expected to play essential roles in cell cycle progression and cell proliferation. Thus the DM signature is a strong predictor of survival in breast cancer because it contains a relatively undiluted sample of genes essential for cell proliferation. The expression of these genes should therefore reflect the cell proliferation rate within a cancer better than the gene sets of the other signatures. Consistent with this idea, the authors have shown that most of the DM signature genes with a high predictive power of poor outcome in patients display increased expression (Fig. 4).

The frequency of mitotic cells is one of the criteria used to classify breast cancers in low versus high grade. However, cytological analysis of mitosis proved to be a rather subjective assay with significant inter-observer variations [34]. The analysis of gene expression using the DM signature provides reliable quantitative information on cell proliferation within a breast cancer sample, allowing risk assessments in individual patients.

The authors have shown that a group of genes required for cytokinesis (*ANLN, CIT, ECT2, KIF23, PRC1, RACGAP1, ASPM, KJF18A* and *PLK1*) contributes to the predictive power of the DM signature significantly more than the other genes in the signature. All cytokinesis genes display high positive z-scores, indicating that an increased expression level of these genes is negatively correlated with survival. Strikingly, there is evidence that *ANLN, ECT2, PRC1, RACGAP1, ASPM,* and *PLK1* are upregulated in a variety of human cancers and that the overexpression levels of these genes often correlate with poor outcomes in patients (see for example [35-43] and references therein). In addition, it has been shown that two of these cytokinesis genes, *ETC2* and *ANLN,* are amplified in cancer cells [38,44]. These findings raise the questions of why cytokinesis genes have a higher prognostic value and tend to be more upregulated in cancers compared to other mitotic genes. It is possible that overexpression of cytokinesis genes is an oncogenic factor per se. However, the finding that *PRC1* overexpression does not result in cell growth enhancement [41] argues against this possibility. Another possibility is that cytokinesis proteins are limited in amount or stability compared to other mitotic proteins. That is, when cell proliferation is strongly enhanced, normal levels of gene transcription and translation would not be sufficient to produce the amounts of cytokinesis proteins required for proper execution of the process. As a result, cancers cell clones overexpressing cytokinesis genes would be favoured over clones in which these genes are normally expressed.

In conclusion, the present invention indicates that the DM signature improves risk stratification for breast cancer patients compared to the major extant signatures. In addition, the identification of new cancer prognostic genes with well-defined biological functions, such as those of the DM signature, provides new prognostic tools based on gene expression. For example, according to a previous approach [6,11,13] the genes of the DM signature could be merged with those of other signatures to further improve risk stratification. Finally, the authors' finding that cytokinesis genes tend to be overexpressed in patients with poor prognosis sets forth this class of genes and their protein products as targets for antimitotic therapies.

**References**

1. Dupuy A, Simon RM (2007) J Natl Cancer Inst 99: 147-157.

2. Wirapati P, et al. (2008) Breast Cancer Res 10: R65.
3. van't Veer LJ, et al. (2002) Nature 415: 530-536.
4. van de Vijver MJ, et al. (2002) N Engl J Med 347: 1999-2009.
5. Chang HY, et al. (2004) PLoS Biol 2: E7.
6. Chang HY, et al. (2005) Proc Natl Acad Sci USA 102: 3738-3743.
7. Chi JT, et al. (2006) PLoS Med 3: e47.
8. Sung FL, et al. (2007) Cancer Lett 253: 74-88.
9. Winter SC, et al. (2007) Cancer Res 67: 3441-3449.
10. Whitfield ML, et al. (2002) Mol Biol Cell 13: 1977-2000.
11. Starmans MH, et al. (2008) Br J Cancer 99: 1884-1890.
12. Ben-Porath I, et al. (2008) Nat Genet 40: 499-507.
13. Reyal F, et al. (2008) Breast Cancer Res 10: R93.
14. Liu R, et al. (2007) N Engl J Med 356: 217-226.
15. Carter SL, et al. (2006) Nat Genet 38: 1043-1048.
16. Somma MP, et al. (2008) PLoS Genet 4: e1000126.
17. Sayers EW, et al. (2010) Nucleic Acids Res 38: D5-16.
18. Shedden K, et al. (2008) Nat Med 14: 822-827.
19. Phillips HS, et al. (2006) Cancer Cell 9: 157-173.
20. Pawitan Y, et al. (2005) Breast Cancer Res 7: R953-964.
21. Miller LD, et al. (2005) Proc Natl Acad Sci USA 102: 13550-13555.
22. Wang Y, et al. (2005) Lancet 365: 671-679.
23. Desmedt C, et al. (2007) Clin Cancer Res 13: 3207-3214.
24. Sotiriou C, et al. (2006) J Natl Cancer Inst 98: 262-272.
25. Wilson CL,Miller CJ (2005) Bioinformatics 21: 3683-3685.
26. Gentleman RC, et al. (2004) Genome Biol 5: R80.
27. Bild AH, et al. (2006) Nature 439: 353-357.
28. Freije WA, et al. (2004) CancerRes 64: 6503-6510.
29. Zhao H, et al. (2006) PLoS Med 3: e13.
30. Eggert US, Mitchison TJ,Field CM (2006) Annu Rev Biochem 75: 543-566.
31. Galanty Y, et al. (2009) Nature 462: 935-939.
32. Coppola D, et al. (2009) J Cancer Res Clin Oncol 135: 1287-1291.
33. Whitfield ML, George LK, Grant GD,Perou CM (2006) Nat Rev Cancer 6: 99-106.
34. Paik S, et al. (2004) N Engl J Med 351: 2817-2826.
35. Suzuki C, et al. (2005) Cancer Res 65: 11314-11325.
36. Tamura K, et al. (2007) Cancer Res 67: 5117-5125.
37. Skrzypski M, et al. (2008) Clin Cancer Res 14: 4794-4799.
38. Fields AP, Justilien V (2009) Adv Enzyme Regul.
39. Horvath S, et al. (2006) Proc Natl Acad Sci USA 103: 17402-17407.
40. Lin SY, et al. (2008) Clin Cancer Res 14: 4814-4820.
41. Shimo A, et al. (2007) Cancer Sci 98: 174-181.
42. Pellegrino R, et al. (2009) Hepatology.
43. Schmit TL, et al. (2009) J Invest Dermatol 129: 2843-2853.
44. Shimizu S, et al. (2007) Oncol Rep 18: 1489-1497.

## Claims

1. A method to predict the mortality risk of a subject (p) affected of breast cancer comprising:

   a) measuring the expression level of the genes *C15orf44, CASP7, CNOT3, CTPS, CUL4B, CWC15, DCAKD, DDB1, FRGI, MSH6, ORC5L, PCNA, PIAS1, POLA1, PRIM2, PRPF3, RAD54L, RFC2, RPA1, RRM2, SART1, SF3A3, SMCIA, TAF6, TFDP2, TK2, TPR, TYMS, WBP11, WDR46, WDR75, XAB2, XRN2, ZMYM4, MCM3, MCM7, SMC3, NCAPD2, NCAPG, SMC4, SMC2, MASTL, ORC2L, TOP2A, CDT1, BUB3, KNTC1, ZW10, ASCC3L1, CCNB1, CDC40, DHX8, KIAA1310, LSM2, PRPF31, SF3A1, SF3A2, SF3B1, SF3B2, SF3B14, SLU7, SNRPA1, SNRPE, TXNL4A, U2AF1, U2AF2, ANAPC5, ANAPC10, CDC20" KIN, PSMC1, SFRS15. CKAP5, EIF3A, EIF3D, EIF3E, EIF3I, GTF3C3, MAPRE3, NOC3L, RRPIB, TBKI, THOC2, TUBB2C, WDR82, TRRAP, TUBGCP4, TUBG2, ASPM, CENPJ, MKI67IP, PPP1R8, CDC2, KIFC1, KJF11, KIF18A, AURKC, RBBP7, PLK1, ECT2, KIF23, PRC1, RACGAP1, ANLN, CIT* in a biological sample, obtaining the prognostic score, S(p), that indicates the expression levels of said genes in said subject (p) affected of cancer, and
   b) predicting the mortality risk of said subject (p) affected of cancer comparing said prognostic score, S(p), to a cut off value (cut off threshold).

2. The method according to claim 1 wherein the expression level of said genes is measured by means of quantitative detection of the transcript sequences selected from the group SEQ ID No 1 to SEQ ID No. 217.

3. The method according to any one of previous claim wherein the expression level of said genes is detected by means of microarray.

4. The method according to any one of previous claim wherein the biological sample is selected from the group of: blood, tumour cell, frozen or fixed tissue sections, biopsy, biological fluid.

**5.** The method according to any one of previous claim wherein the mortality risk is assigned as follows:

iv) to the class "low risk" if the prognostic score, S(p), is lower than the cut off threshold, or
v) to the class "high risk" if the prognostic score, S(p), is greater than the cut off threshold, and optionally
vi) to the class "intermediate" if the prognostic score, S(p), is between two cut off threshold values.

**6.** The method according to any one of previous claim wherein the prognostic score, S(p), is calculated according to the following formula:

$$S(p) = \sum_g x(g,p)z(g)$$

wherein
*x(g,p)* is the expression level expressed in logarithmic base 2 of the probeset *g* in the patient *p*;
*z*(g) is the *z*-score of the probeset g calculated in the Pawitan dataset;
wherein the probeset *g* comprises a group of 217 probes, each one being specific and selective for one of the gene transcript belonging to the group of SEQ ID No. 1 to SEQ ID No. 217.

**7.** The method according to claim 6 wherein the z-score for each probe is the one calculated in the Pawitan database reported in table II.

**8.** A kit to detect the transcript expression level of genes *C15orf44, CASP7, CNOT3, CTPS, CUL4B, CWC15, DCAKD, DDB1, FRG1, MSH6, ORC5L, PCNA, PIAS1, POLA1, PRIM2, PRPF3, RAD54L, RFC2, RPA1, RRM2, SART1, SF3A3, SMC1A, TAF6, TFDP2, TK2, TPR, TYMS, WBP11, WDR46, WDR75, XAB2, XRN2, ZMYM4, MCM3, MCM7, SMC3, NCAPD2, NCAPG, SMC4, SMC2, MASTL, ORC2L, TOP2A, CDT1, BUB3, KNTC1, ZW10, ASCC3L1, CCNB1, CDC40, DHX8, KIAA1310, LSM2, PRPF31, SF3A1, SF3A2, SF3B1, SF3B2, SF3B14, SLU7, SNRPA1, SNRPE, TXNL4A, U2AF1, U2AF2, ANAPC5, ANAPC10, CDC20" KIN, PSMC1, SFRS15. CKAP5, EIF3A, EIF3D, EIF3E, EIF3I, GTF3C3, MAPRE3, NOC3L, RRPIB, TBKI, THOC2, TUBB2C, WDR82, TRRAP, TUBGCP4, TUBG2, ASPM, CENPJ, MK167IP, PPP1R8, CDC2, KIFC1, KIF11, KIF18A, AURKC, RBBP7, PLK1, ECT2, KIF23, PRC1, RACGAP1, ANLN, CIT,*
comprising:

- for each of said genes, sequence specific amplification means to obtain amplified nucleic acids having sequences comprised in the transcribed region thereof;
- quantitative detection means of said amplified nucleic acids;
- appropriate reagents.

**9.** The kit according to claim 8 wherein said amplified nucleic acids consist of :
for *C15orf44,* SEQ ID No. 145; for *CASP7,* SEQ ID No. 189; for *CNOT3,* SEQ ID No. 66 and/or SEQ ID No. 138 and/or SEQ ID No.167; for *CTPS,* SEQ ID No. 39; for *CUL4B,* SEQ ID No. 113 and/or SEQ ID No. 152 and/or SEQ ID No. 165 and/or SEQ ID No. 212; for *CWC15,* SEQ ID No. 159; for *DCAKD,* SEQ ID No. 126 and/or SEQ ID No. 140 and/or SEQ ID No. 190; for *DDB1,* SEQ ID No. 38; for *FRG1,* SEQ ID No. 195; for *MSH6,* SEQ ID No. 46 and/or SEQ ID No. 61 and /or SEQ ID No. 153 and/or SEQ ID No. 187; for *ORC5L,* SEQ ID No. 70 and/or SEQ ID No. 79 and/or SEQ ID No. 109; for *PCNA,* SEQ ID No. 51; for *PIAS1,* SEQ ID No. 211 and/or SEQ ID No. 216 and/or SEQ ID No. 217; for *POLA1,* SEQ ID No. 147; for *PRIM2,* SEQ ID No. 43 and/or SEQ ID No. 56 and/or SEQ ID No. 88; for *PRPF3,* SEQ ID No. 170; for *RAD54L,* SEQ ID No. 75; for *RFC2,* SEQ ID No. 42 and/or SEQ ID No. 48; for *RPA1,* SEQ ID No. 64 and/or SEQ ID No. 103; for *RRM2*, SEQ ID No. 3 and/or SEQ ID No. 9; for *SART1,* SEQ ID No. 124; for *SF3A3,* SEQ ID No. 201; for *SMC1A,* SEQ ID No. 115 and/or SEQ ID No. 179 and/or SEQ ID No. 207; for *TAF6,* SEQ ID No. 68; for *TFDP2,* SEQ ID No. 86 and/or SEQ ID No. 118 and/or SEQ ID No. 210; for *TK2,* SEQ ID No. 37 and/or SEQ ID No. 156 and/or SEQ ID No. 171 and/or SEQ ID No. 172; for *TPR,* SEQ ID No. 99 and/or SEQ ID No. 108 and/or SEQ ID No. 182 and/or SEQ ID No. 204; for *TYMS,* SEQ ID No. 32 and/or SEQ ID No. 125; for *WBP11,* SEQ ID No. 65 and/or SEQ ID No. 67; for *WDR46,* SEQ ID No. 93; for *WDR75,* SEQ ID No. 158; for *XAB2,* SEQ ID No. 180; for *XRN2,* SEQ ID No. 81 and/or SEQ ID No. 84; for *ZMYM4,* SEQ ID No. 192 and/or SEQ ID No. 196 and/or SEQ ID No. 213; for *MCM3,* SEQ ID No. 34; for *MCM7,* SEQ ID No. 28 and/or SEQ ID No. 52; for *SMC3,* SEQ ID No. 185 and/or SEQ ID No. 193 and/or SEQ ID No. 209; for *NCAPD2,* SEQ ID No. 106; for *NCAPG,* SEQ ID No.22 and/or SEQ ID No. 24; for *SMC4,* SEQ ID No. 33 and/or SEQ ID No. 54 and/or SEQ ID No. 141; for *SMC2,* SEQ ID No. 45 and/or SEQ ID No. 127; for *MASTL,* SEQ ID No. 11; for *ORC2L,* SEQ ID No.

104; for *TOP2A,* SEQ ID No. 20 and/or SEQ ID No. 62 and/or SEQ ID No. 96; for *CDT1,* SEQ ID No. 2 and/or SEQ ID No. 36; for *BUB3,* SEQ ID No. 57 and/or SEQ ID No. 139 and/or SEQ ID No. 148 and/or SEQ ID No. 174 and/or SEQ ID No. 178; for *KNTC1,* SEQ ID No. 35; for *ZW10,* SEQ ID No. 143; for *ASCC3L1,* SEQ ID No. 55 and/or SEQ ID No. 135 and/or SEQ ID No. 150; for *CCNB1,* SEQ ID No. 7 and/or SEQ ID No. 14; for *CDC40,* SEQ ID No. 100 and/or SEQ ID No. 177; for *DHX8,* SEQ ID No. 58 and/or SEQ ID No. 120 and/or SEQ ID No. 121; for *KIAA1310,* SEQ ID No. 160 and/or SEQ ID No. 183 and/or SEQ ID No. 188; for *LSM2,* SEQ ID No. 137; for *PRPF31,* SEQ ID No. 60 and/or SEQ ID No. 91 and/or SEQ ID No. 184; for *SF3A1,* SEQ ID No. 98 and/or SEQ ID No. 119 and/or SEQ ID No. 162 and/or SEQ ID No. 173; for *SF3A2,* SEQ ID No. 169 and/or SEQ ID No. 176; for *SF3B1,* SEQ ID No. 194 and/or SEQ ID No. 203 and/or SEQ ID No. 208 and/or SEQ ID No. 214; for *SF3B2,* SEQ ID No. 77; for *SF3B14,* SEQ ID No. 10; for *SLU7,* SEQ ID No. 149 and/or SEQ ID No. 151; for *SNRPA1,* SEQ ID No. 23 and/or SEQ ID No. 49 and/or SEQ ID No. 71 and/or SEQ ID No. 181; for *SNRPE,* SEQ ID No. 72 and/or SEQ ID No. 136; for *TXNL4A,* SEQ ID No. 26 and/or SEQ ID No. 134; for *U2AF1,* SEQ ID No. 30 and/or SEQ ID No. 82 and/or SEQ ID No. 102 and/or SEQ ID No. 131; for *U2AF2,* SEQ ID No. 94 and/or SEQ ID No. 146 and/or SEQ ID No. 155 and/or SEQ ID No. 161; for *ANAPC5,* SEQ ID No. 85 and/or SEQ ID No. 95 and/or SEQ ID No. 97 and/or SEQ ID No. 112 and/or SEQ ID No. 117; for *ANAPC10,* SEQ ID No. 129; for *CDC20,* SEQ ID No. 17; for *KIN,* SEQ ID No. 111 and/or SEQ ID No. 144; for *PSMC1,* SEQ ID No. 25; for *SFRS15,* SEQ ID No. 50 and/or SEQ ID No. 63 and/or SEQ ID No. 80 and/or SEQ ID No. 142 and/or SEQ ID No. 197; for *CKAP5,* SEQ ID No. 21; for *EIF3A,* SEQ ID No. 175 and/or SEQ ID No. 186 and/or SEQ ID No. 202; for *EIF3D,* SEQ ID No. 101; for *EIF3E,* SEQ ID No. 154; for *EIF31,* SEQ ID No. 114; for *GTF3C3,* SEQ ID No. 74 and/or SEQ ID No. 163; for *MAPRE3,* SEQ ID No. 116 and/or SEQ ID No. 128 and/or SEQ ID No. 130 and/or SEQ ID No. 133; for *NOC3L,* SEQ ID No. 164; for *RRPIB,* SEQ ID No. 105 and/or SEQ ID No. 123; for *TBKI,* SEQ ID No. 198; for *THOC2,* SEQ ID No. 110 and/or SEQ ID No. 132 and/or SEQ ID No. 199 and/or SEQ ID No. 205; for *TUBB2C,* SEQ ID No. 4 and/or SEQ ID No. 5; for *WDR82,* SEQ ID No. 191; for *TRRAP,* SEQ ID No. 69 and/or SEQ ID No. 73; for *TUBGCP4,* SEQ ID No. 76 and/or SEQ ID No. 215; for *TUBG2,* SEQ ID No. 157; *for ASPM,* SEQ ID No. 6 and/or SEQ ID No. 47 and/or SEQ ID No. 53; for *CENPJ,* SEQ ID No. 87 and/or SEQ ID No. 92 and/or SEQ ID No. 107; for *MK167IP,* SEQ ID No. 41 and/or SEQ ID No. 89 and/or SEQ ID No. 200; for *PPPIR8,* SEQ ID No. 168; for *CDC2,* SEQ ID No. 15 and/or SEQ ID No. 16 and/or SEQ ID No. 31 and/or SEQ ID No. 206; for *KIFC1,* SEQ ID No. 19; for *KIF11,* SEQ ID No. 29; for *KIF18A,* SEQ ID No. 18; for *AURKC,* SEQ ID No. 90; for *RBBP7,* SEQ ID No. 166; for *PLK1,* SEQ ID No. 27; for *ECT2,* SEQ ID No. 40 and/or SEQ ID No. 59 and/or SEQ ID No. 83; for *KIF23,* SEQ ID No. 8 and/or SEQ ID No. 44; for *PRC1,* SEQ ID No. 13; for *RACGAP1,* SEQ ID No. 12; for *ANLN,* SEQ ID No. 1; for *CIT,* SEQ ID No. 78 and/or SEQ ID No. 122.

**10.** The kit according to claim 8 or 9 further comprising sequence specific amplification means to obtain amplified nucleic acids having sequences comprised in the transcribed region of genes H3F3A and/or PPAN-P2RY11 and/or KIF4.

**11.** A microarray consisting of:

a) solid supporting means, and

b) for each of the genes *C15orf44*, *CASP7*, *CNOT3*, *CTPS*, *CUL4B*, *CWC15*, *DCAKD*, *DDB1*, *FRG1*, *MSH6, ORC5L, PCNA, PIAS1, POLA1, PRIM2, PRPF3, RAD54L, RFC2, RPA1*, *RRM2, SART1, SF3A3, SMCIA, TAF6, TFDP2, TK2, TPR, TYMS, WBP11, WDR46, WDR75, XAB2, XRN2, ZMYM4, MCM3, MCM7, SMC3, NCAPD2, NCAPG, SMC4, SMC2, MASTL, ORC2L, TOP2A, CDT1, BUB3, KNTC1, ZW10, ASCC3L1, CCNB1, CDC40, DHX8, KIAA1310, LSM2, PRPF31, SF3A1, SF3A2, SF3B1, SF3B2, SF3B14, SL U7, SNRPA1, SNRPE, TXNL4A, U2AF1, U2AF2, ANAPC5, ANAPC10, CDC20" KIN, PSMC1, SFRS15. CKAP5, EIF3A, EIF3D, EIF3E, EIF3I, GTF3C3, MAPRE3, NOC3L, RRPIB, TBKI, THOC2, TUBB2C, WDR82, TRRAP, TUBGCP4, TUBG2, ASPM, CENPJ, MKI67IP, PPPIR8, CDC2, KIFC1, KJF11, KJF18A, AURKC, RBBP7, PLK1, ECT2, KIF23, PRC1, RACGAP1, ANLN, CIT,* at least one oligonucleotide able to specifically hybridize to a sequence comprised in the transcribed region thereof.

**12.** The microarray according to claim 11 wherein the sequences comprised in the transcribed region of said genes consist of : for *C15orf44,* SEQ ID No. 145; for *CASP7,* SEQ ID No. 189; for *CNOT3,* SEQ ID No. 66 and/or SEQ ID No. 138 and/or SEQ ID No.167; for *CTPS,* SEQ ID No. 39; for *CUL4B,* SEQ ID No. 113 and/or SEQ ID No. 152 and/or SEQ ID No. 165 and/or SEQ ID No. 212; for *CWC15,* SEQ ID No. 159; for *DCAKD,* SEQ ID No. 126 and/or SEQ ID No. 140 and/or SEQ ID No. 190; for *DDB1,* SEQ ID No. 38; for *FRG1,* SEQ ID No. 195; for *MSH6,* SEQ ID No. 46 and/or SEQ ID No. 61 and /or SEQ ID No. 153 and/or SEQ ID No. 187; for *ORC5L,* SEQ ID No. 70 and/or SEQ ID No. 79 and/or SEQ ID No. 109; for *PCNA,* SEQ ID No. 51; for *PIAS1,* SEQ ID No. 211 and/or SEQ ID No. 216 and/or SEQ ID No. 217; for *POLA1,* SEQ ID No. 147; for *PRIM2,* SEQ ID No. 43 and/or SEQ ID No. 56 and/or SEQ ID No. 88; for *PRPF3,* SEQ ID No. 170; for *RAD54L,* SEQ ID No. 75; for *RFC2,* SEQ ID No. 42 and/or SEQ ID No. 48; for *RPA1,* SEQ ID No. 64 and/or SEQ ID No. 103; for RRM2, SEQ ID No. 3 and/or SEQ ID No. 9; for *SART1,* SEQ ID No. 124; for *SF3A3,* SEQ ID No. 201; for *SMCIA,* SEQ ID No. 115 and/or SEQ ID No. 179 and/or SEQ ID No. 207; for *TAF6,* SEQ ID No. 68; for *TFDP2,* SEQ ID No. 86 and/or SEQ ID No. 118 and/or SEQ ID No. 210; for *TK2,* SEQ ID No. 37 and/or SEQ ID No. 156 and/or SEQ ID No. 171 and/or SEQ ID No. 172; for *TPR,* SEQ ID No. 99 and/or SEQ ID No. 108 and/or SEQ ID No. 182 and/or SEQ ID No. 204; for *TYMS,* SEQ ID No. 32 and/or SEQ ID No. 125; for *WBP11,* SEQ ID No. 65 and/or SEQ ID No. 67; for *WDR46,* SEQ ID No. 93; for *WDR75,* SEQ ID No. 158; for *XAB2,* SEQ ID No. 180; for *XRN2,* SEQ ID No. 81 and/or SEQ ID No. 84; for *ZMYM4,* SEQ ID No. 192 and/or SEQ ID No. 196 and/or SEQ ID No. 213; for *MCM3,* SEQ ID No. 34; for *MCM7,* SEQ ID No. 28 and/or SEQ ID No. 52; for *SMC3,* SEQ ID No. 185 and/or SEQ ID No. 193 and/or SEQ ID No. 209; for *NCAPD2,* SEQ ID No. 106; for *NCAPG,* SEQ ID No.22 and/or SEQ ID No. 24; for *SMC4,* SEQ ID No. 33 and/or SEQ ID No. 54 and/or SEQ ID No. 141; for *SMC2,* SEQ ID No. 45 and/or SEQ ID No. 127; for *MASTL,* SEQ ID No. 11; for *ORC2L,* SEQ ID No. 104; for *TOP2A,* SEQ ID No. 20 and/or SEQ ID No. 62 and/or SEQ ID No. 96; for *CDT1,* SEQ ID No. 2 and/or SEQ ID No. 36; for *BUB3,* SEQ ID No. 57 and/or SEQ ID No. 139 and/or SEQ ID No. 148 and/or SEQ ID No. 174 and/or SEQ ID No. 178; for *KNTC1,* SEQ ID No. 35; for *ZW10,* SEQ ID No. 143; for *ASCC3L1,* SEQ ID No. 55 and/or SEQ ID No. 135 and/or SEQ ID No. 150; for *CCNB1,* SEQ ID No. 7 and/or SEQ ID No. 14; for *CDC40,* SEQ ID No. 100 and/or SEQ ID No. 177; for *DHX8,* SEQ ID No. 58 and/or SEQ ID No. 120 and/or SEQ ID No. 121; for *KIAA1310,* SEQ ID No. 160 and/or SEQ ID No. 183 and/or SEQ ID No. 188; for *LSM2,* SEQ ID No. 137; for *PRPF31,* SEQ ID No. 60 and/or SEQ ID No. 91 and/or SEQ ID No. 184; for *SF3A1,* SEQ ID No. 98 and/or SEQ ID No. 119 and/or SEQ ID No. 162 and/or SEQ ID No. 173; for *SF3A2,* SEQ ID No. 169 and/or SEQ ID No. 176; for *SF3B1,* SEQ ID No. 194 and/or SEQ ID No. 203 and/or SEQ ID No. 208 and/or SEQ ID No. 214; for *SF3B2,* SEQ ID No. 77; for *SF3B14,* SEQ ID No. 10; for *SLU7,* SEQ ID No. 149 and/or SEQ ID No. 151; for *SNRPA1,* SEQ ID No. 23 and/or SEQ ID No. 49 and/or SEQ ID No. 71 and/or SEQ ID No. 181; for *SNRPE,* SEQ ID No. 72 and/or SEQ ID No. 136; for *TXNL4A,* SEQ ID No. 26 and/or SEQ ID No. 134; for *U2AF1,* SEQ ID No. 30 and/or SEQ ID No. 82 and/or SEQ ID No. 102 and/or SEQ ID No. 131; for *U2AF2,* SEQ ID No. 94 and/or SEQ ID No. 146 and/or SEQ ID No. 155 and/or SEQ ID No. 161; for *ANAPC5,* SEQ ID No. 85 and/or SEQ ID No. 95 and/or SEQ ID No. 97 and/or SEQ ID No. 112 and/or SEQ ID No. 117; for *ANAPC10,* SEQ ID No. 129; for *CDC20,* SEQ ID No. 17; for *KIN,* SEQ ID No. 111 and/or SEQ ID No. 144; for *PSMC1,* SEQ ID No. 25; for *SFRS15,* SEQ ID No. 50 and/or SEQ ID No. 63 and/or SEQ ID No. 80 and/or SEQ ID No. 142 and/or SEQ ID No. 197; for *CKAP5,* SEQ ID No. 21; for *EIF3A,* SEQ ID No. 175 and/or SEQ ID No. 186 and/or SEQ ID No. 202; *for EIF3D,* SEQ ID No. 101; for *EIF3E,* SEQ ID No. 154; for *EIF3I,* SEQ ID No. 114; for *GTF3C3,* SEQ ID No. 74 and/or SEQ ID No. 163; *for MAPRE3,* SEQ ID No. 116 and/or SEQ ID No. 128 and/or SEQ ID No. 130 and/or SEQ ID No. 133; for *NOC3L,* SEQ ID No. 164; for *RRP1B,* SEQ ID No. 105 and/or SEQ ID No. 123; for *TBK1,* SEQ ID No. 198; for *THOC2,* SEQ ID No. 110 and/or SEQ ID No. 132 and/or SEQ ID No. 199 and/or SEQ ID No. 205; for *TUBB2C,* SEQ ID No. 4 and/or SEQ ID No. 5; for *WDR82,* SEQ ID No. 191; for *TRRAP,* SEQ ID No. 69 and/or SEQ ID No. 73; for *TUBGCP4,* SEQ ID No. 76 and/or SEQ ID No. 215; for *TUBG2,* SEQ ID No. 157; for *ASPM,* SEQ ID No. 6 and/or SEQ ID No. 47 and/or SEQ ID No. 53; for *CENPJ,* SEQ ID No. 87 and/or SEQ ID No. 92 and/or SEQ ID No. 107; for *MKI67IP,* SEQ ID No. 41 and/or SEQ ID No. 89 and/or SEQ ID No. 200; for *PPP1R8,* SEQ ID No. 168; for *CDC2,* SEQ ID No. 15 and/or SEQ ID No. 16 and/or SEQ ID No. 31 and/or SEQ ID No. 206; for *KIFC1,* SEQ ID No. 19; for *KIF11,* SEQ ID No. 29; for *KIF18A,* SEQ ID No. 18; for *AURKC,* SEQ ID No. 90; for *RBBP7,* SEQ ID No. 166; for *PLK1,* SEQ ID No. 27; for *ECT2,* SEQ ID No. 40 and/or SEQ ID No. 59 and/or SEQ ID No. 83; for *KIF23,* SEQ ID No. 8 and/or SEQ ID No. 44; for *PRC1,* SEQ ID No. 13; for *RACGAP1,* SEQ ID No. 12; for *ANLN,* SEQ ID No. 1; for *CIT,* SEQ ID No. 78 and/or SEQ ID No. 122.

**13.** The microarray according to claim 11 or 12 further comprising at least one oligonucleotide able to specifically hybridize to a sequence comprised in the transcribed region of genes H3F3A and/or PPAN-P2RY11 and/or KIF4.

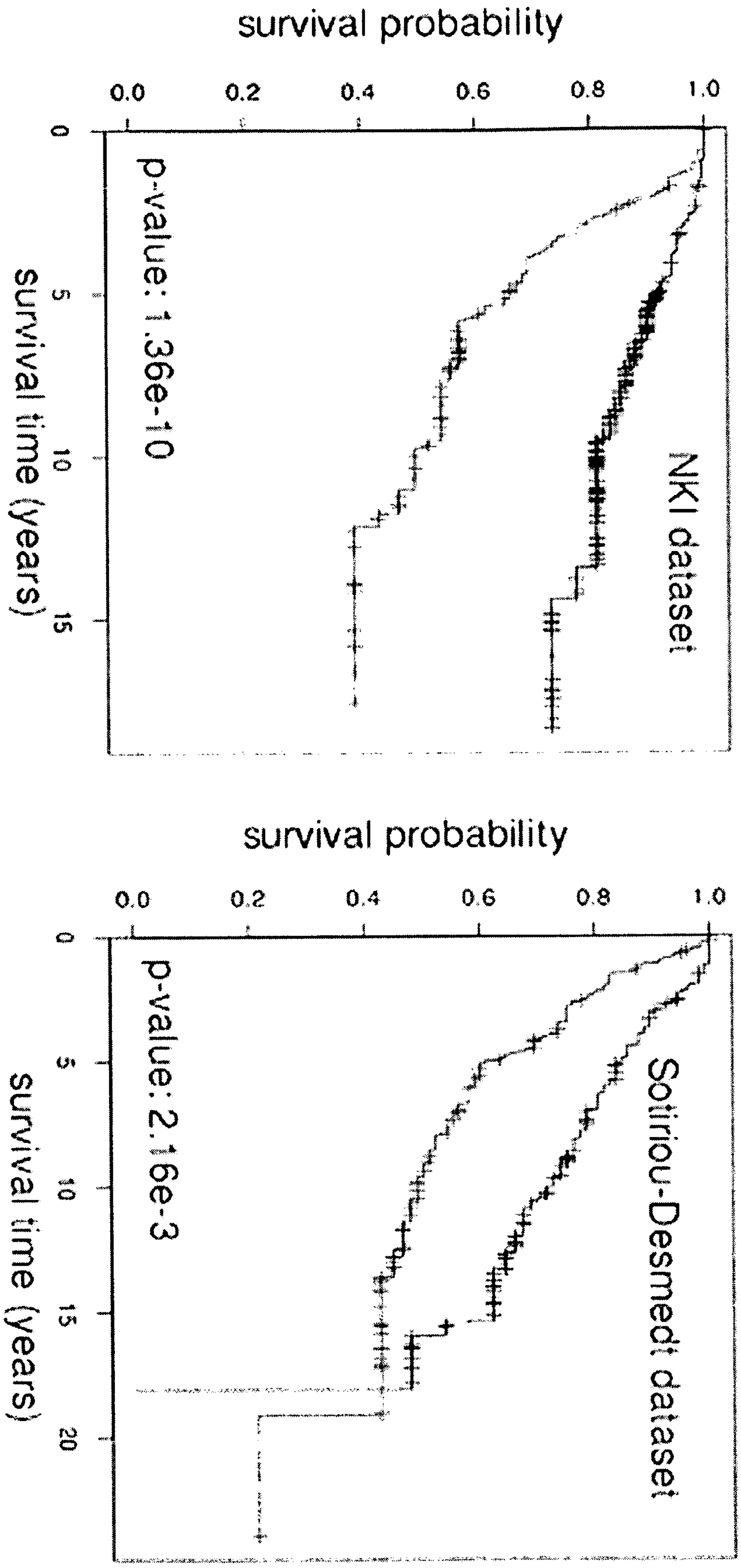
survival probability
0.0
0.2
0.4
0.6
0.8
1.0
0
5
10
15
survival time (years)
p-value: 1.36e-10
NKI dataset
survival probability
0.0
0.2
0.4
0.6
0.8
1.0
0
5
10
15
20
survival time (years)
p-value: 2.16e-3
Sotiriou-Desmedt dataset

Fig. 1

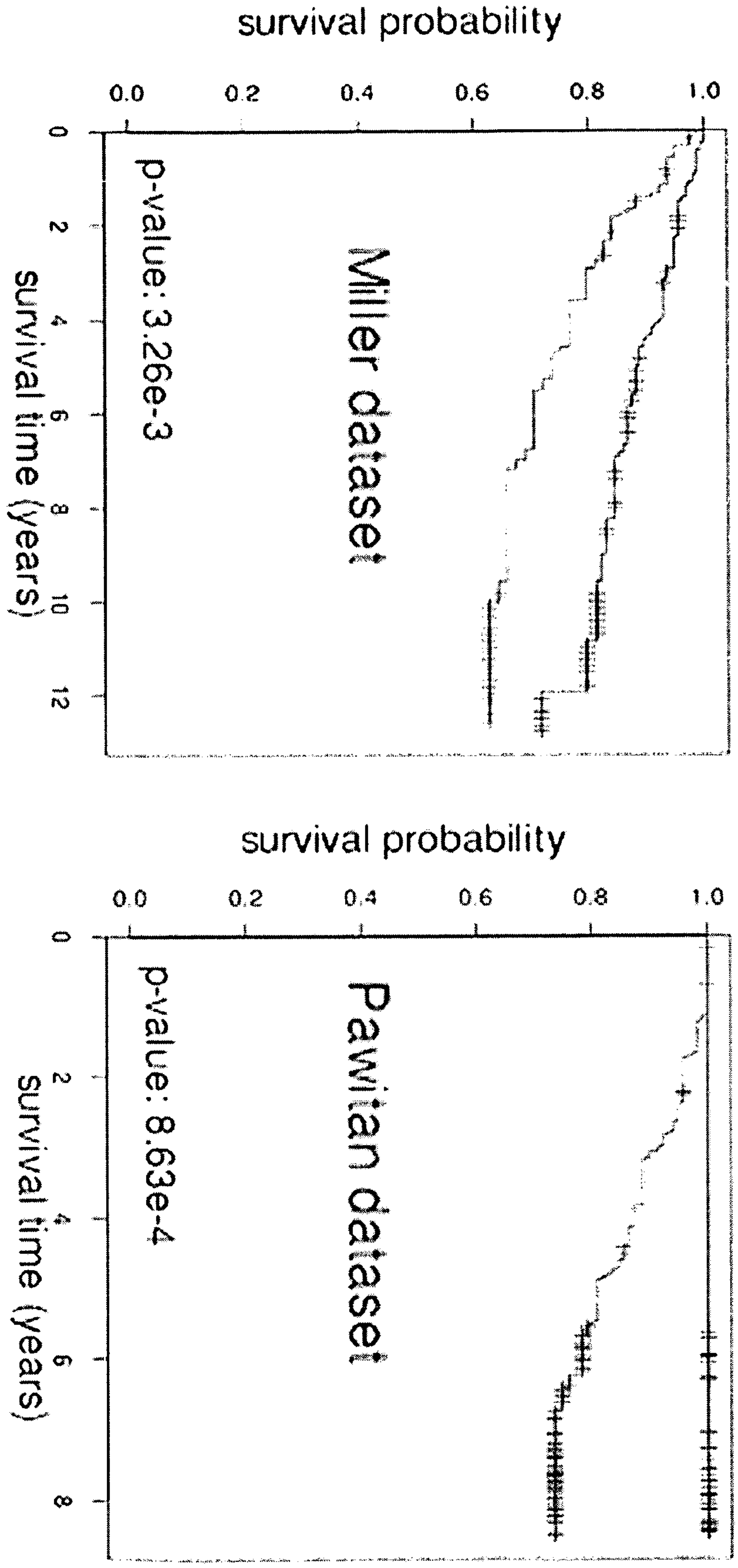
survival probability
Miller dataset
p-value: 3.26e-3
survival time (years)
survival probability
Pawitan dataset
p-value: 8.63e-4
survival time (years)

Fig. 1

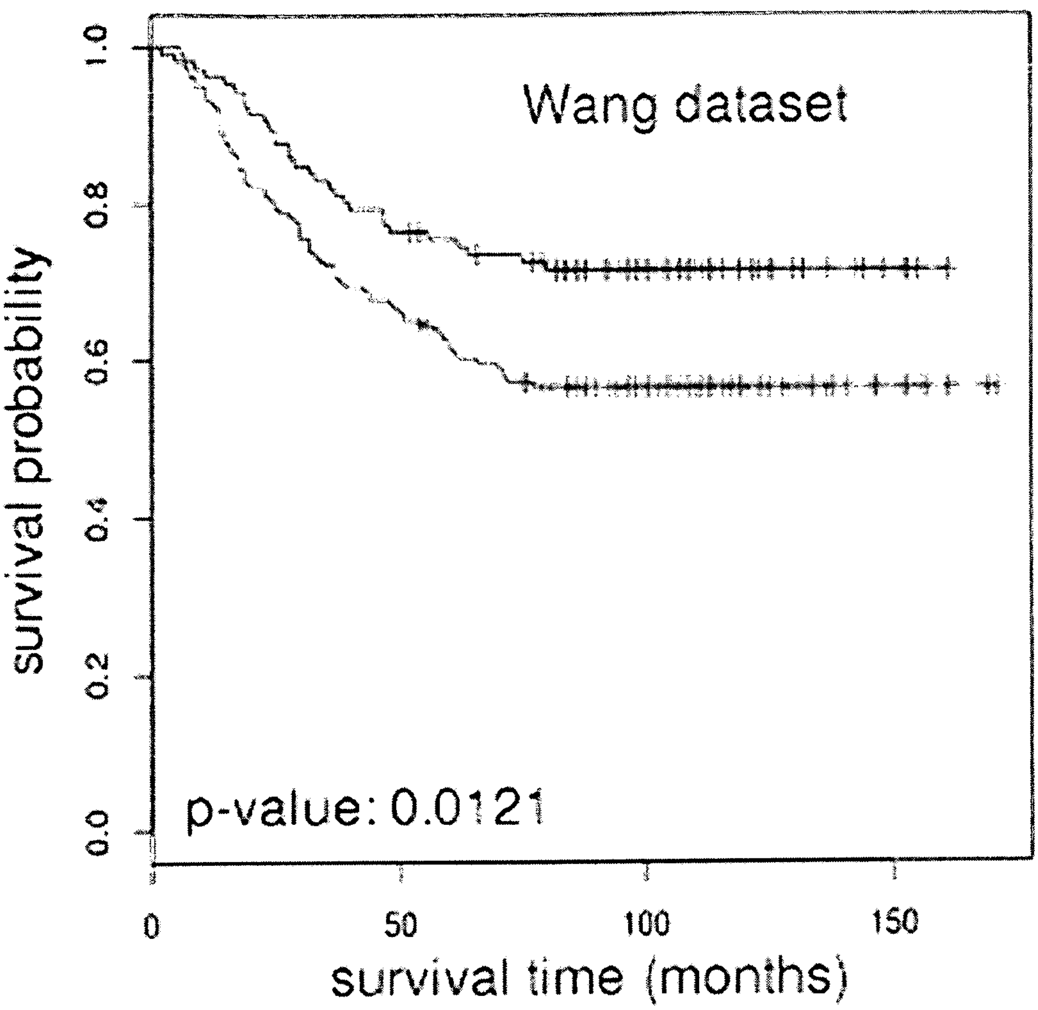
Wang dataset
p-value: 0.0121
survival probability
0.0
0.2
0.4
0.6
0.8
1.0
0
50
100
150
survival time (months)

Fig. 1

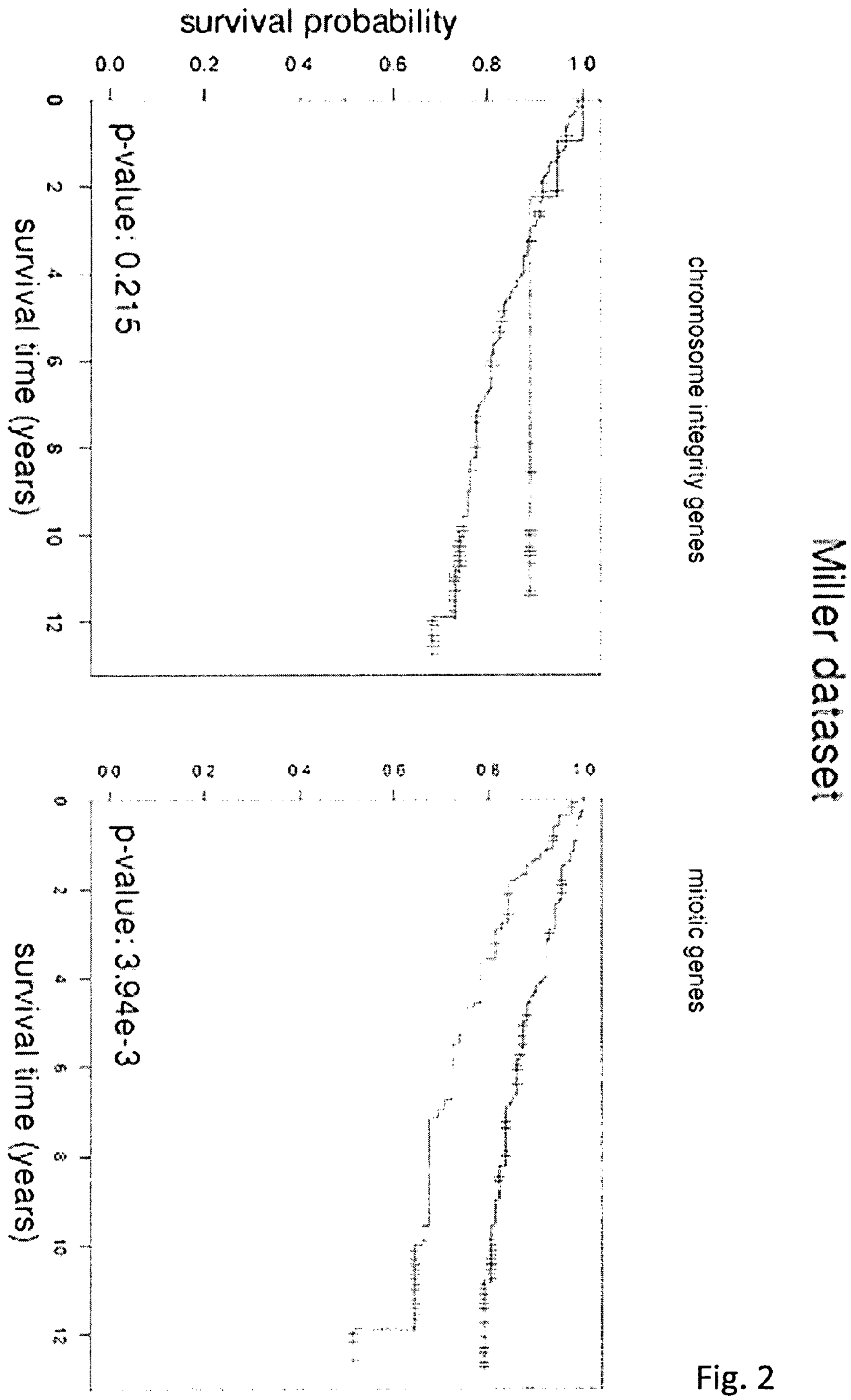
Miller dataset
chromosome integrity genes
mitotic genes
survival probability
0.0
0.2
0.4
0.6
0.8
1.0
0
2
4
6
8
10
12
p-value: 0.215
p-value: 3.94e-3
survival time (years)
survival time (years)

Fig. 2

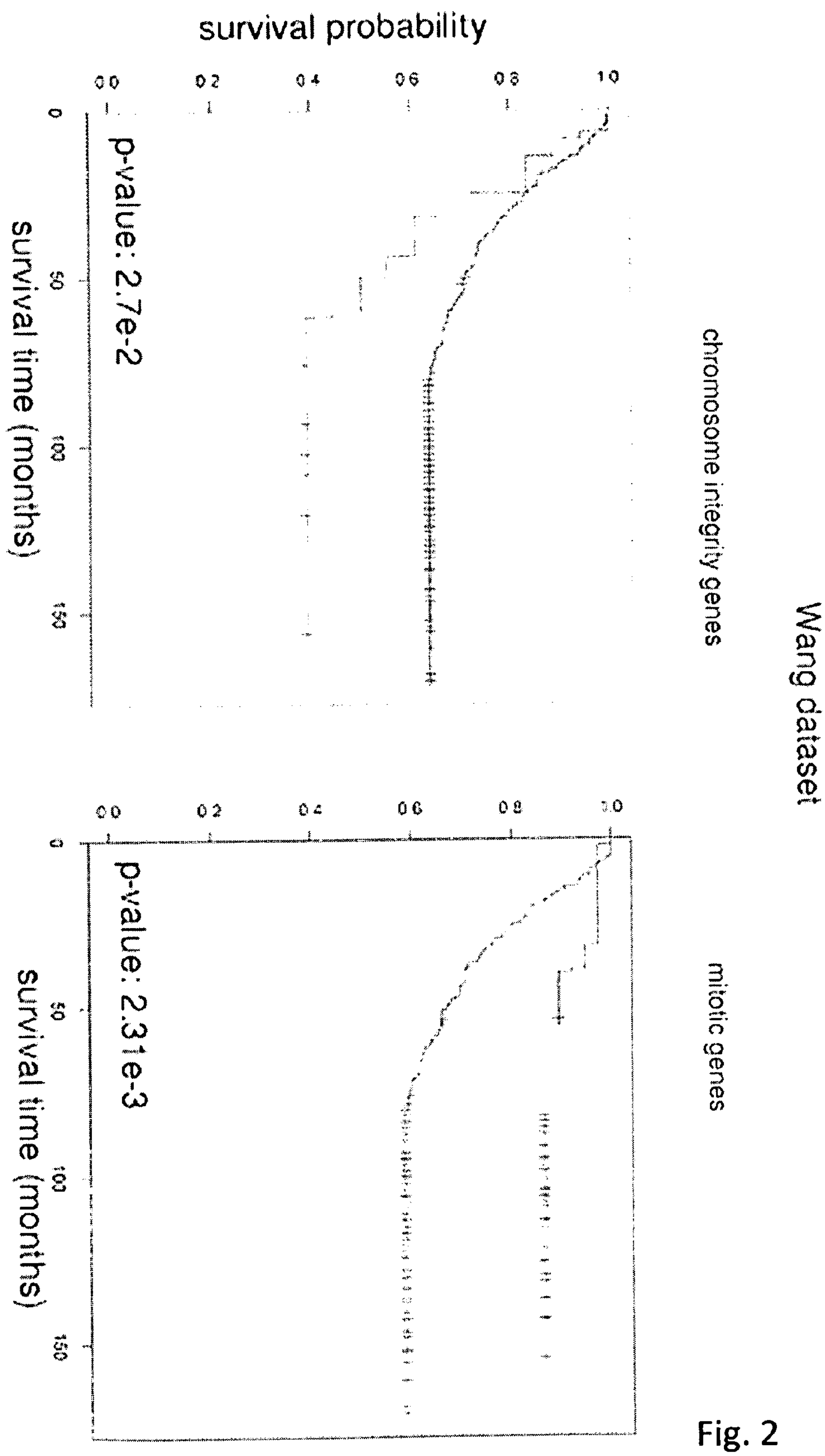
Wang dataset
chromosome integrity genes
mitotic genes
survival probability
0.0
0.2
0.4
0.6
0.8
1.0
0
50
100
150
p-value: 2.7e-2
p-value: 2.31e-3
survival time (months)
survival time (months)

Fig. 2

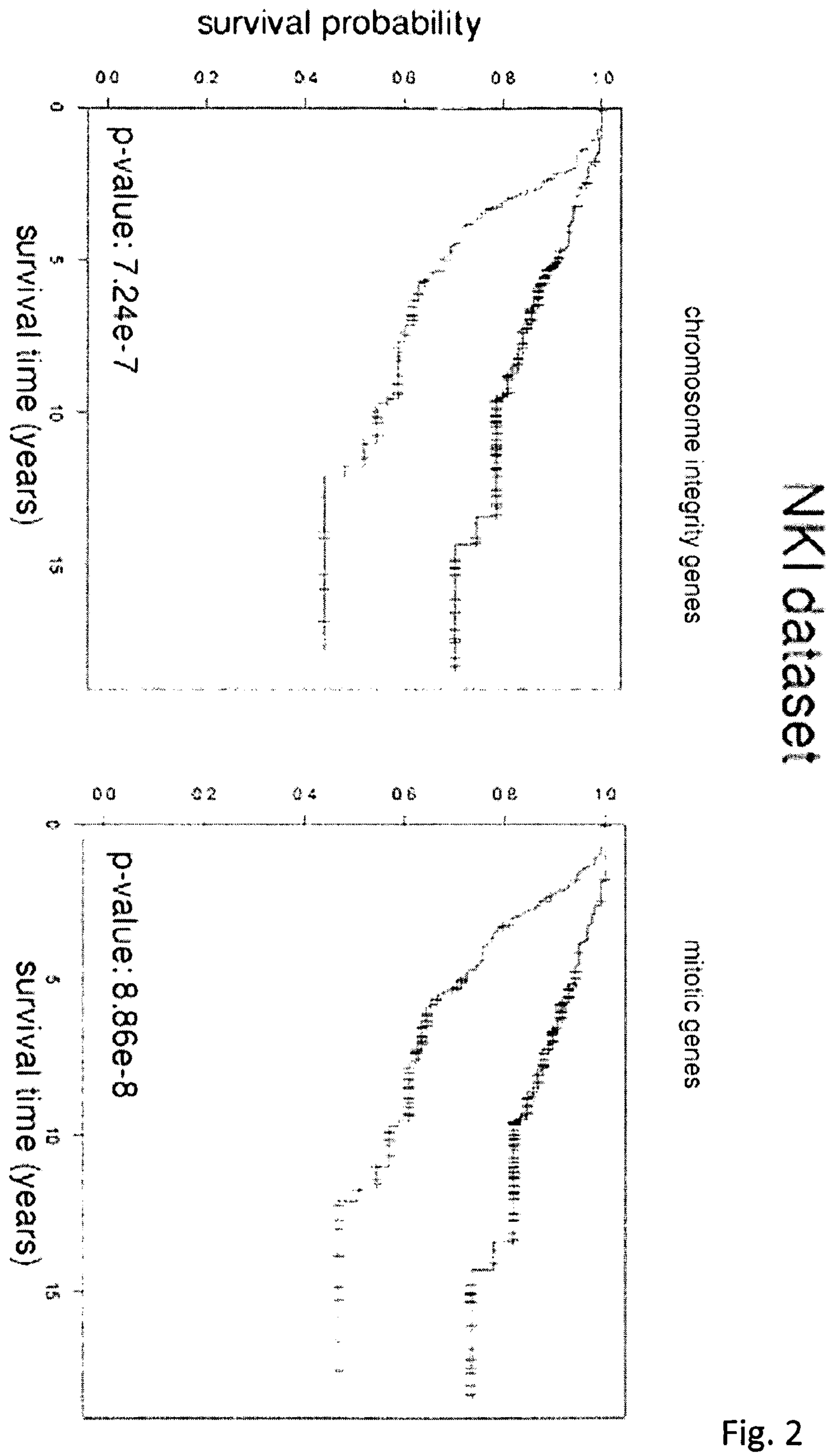
NKI dataset
chromosome integrity genes
mitotic genes
survival probability
0.0
0.2
0.4
0.6
0.8
1.0
p-value: 7.24e-7
p-value: 8.86e-8
survival time (years)
0
5
10
15

Fig. 2

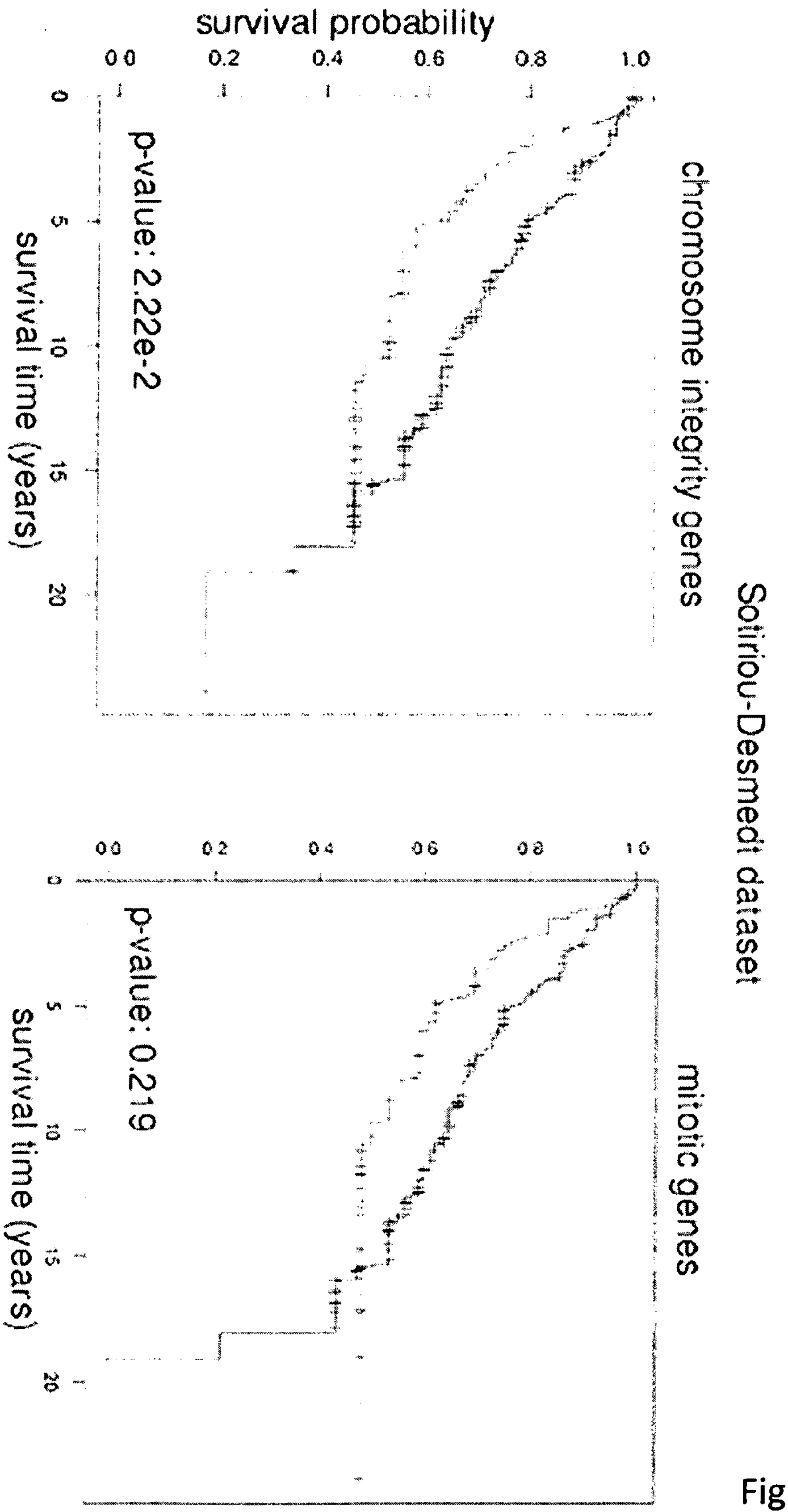
Sotiriou-Desmedt dataset
chromosome integrity genes
mitotic genes
survival probability
0.0
0.2
0.4
0.6
0.8
1.0
p-value: 2.22e-2
p-value: 0.219
survival time (years)
0
5
10
15
20

Fig. 2

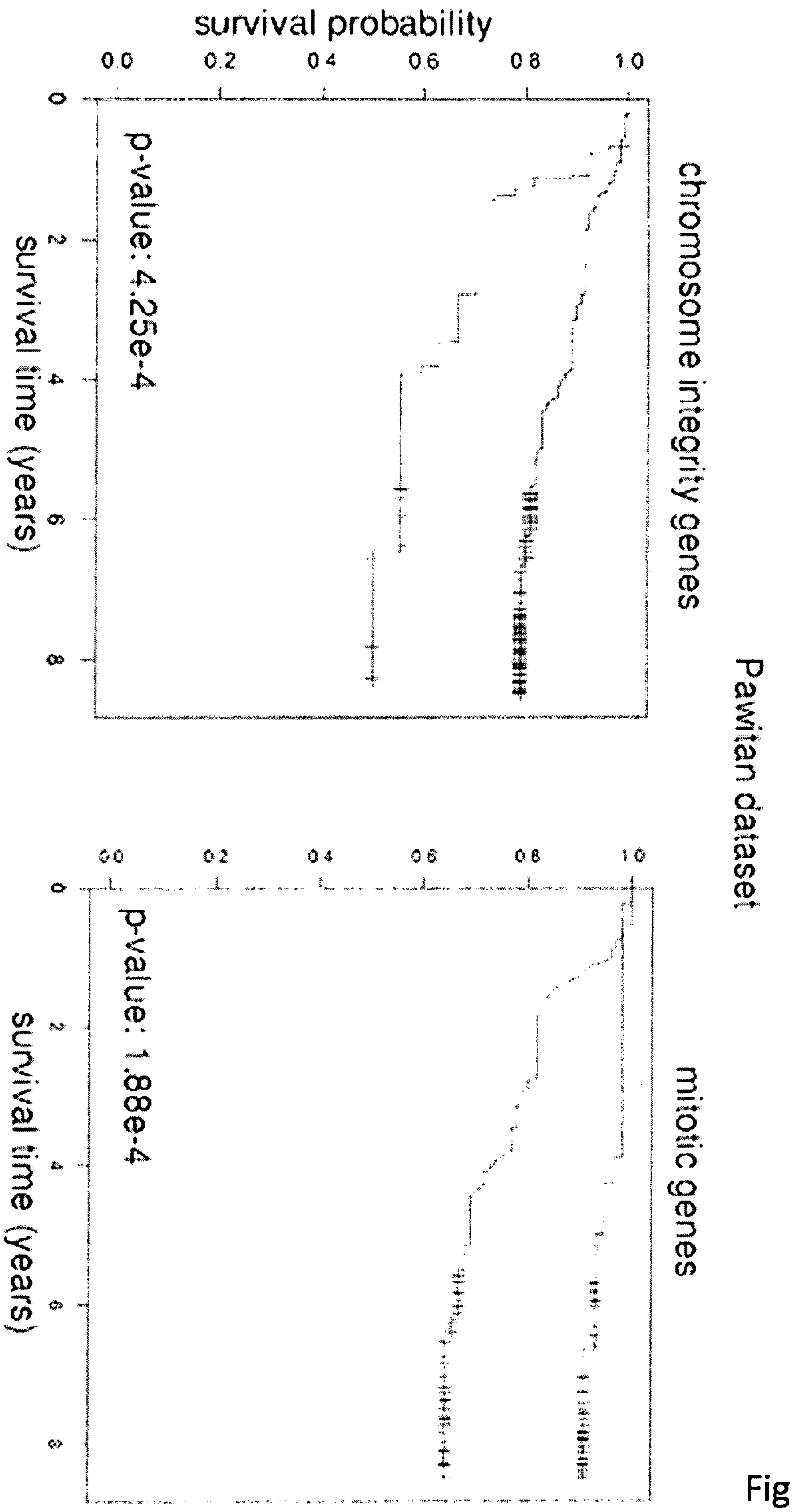
survival probability
0.0
0.2
0.4
0.6
0.8
1.0
chromosome integrity genes
p-value: 4.25e-4
0
2
4
6
8
survival time (years)
Pawitan dataset
mitotic genes
p-value: 1.88e-4
survival time (years)

Fig. 2

| signature \ dataset | Miller | Pawitan | Sotiriou Desmedt | NKI | Wang | -(log)p-value complessivo |
|---|---|---|---|---|---|---|
| DM | 2.18 | 6.19 | 2.25 | 9.73 | 2.87 | 23.23 |
| Proliferation | 3.29 | 3.46 | 3.96 | 8.14 | 1.17 | 20.03 |
| Module | 4.35 | 3.02 | 2.55 | 6.56 | 1.33 | 17.81 |
| CIN | 2.48 | 1.64 | 0.19 | 9.80 | 3.39 | 17.50 |
| Hypoxia (Sung) | 1.84 | 0.28 | 1.75 | 10.20 | 0.35 | 14.42 |
| ES | 0.07 | 5.00 | 0.01 | 8.07 | 0.24 | 13.39 |
| 70-gene | 2.14 | 1.30 | 0.44 | 6.28 | 1.00 | 11.16 |
| IGS | 0.52 | 0.82 | 0.49 | 6.18 | 0.34 | 8.35 |
| Hypoxia (Winter) | 0.13 | 1.02 | 0.03 | 5.91 | 0.77 | 7.86 |
| Wound | 0.12 | 0.60 | 0.98 | 3.21 | 0.40 | 5.31 |

| |
|---|
| P > 0.05 |
| 0.05 > P > 0.01 |
| P < 0.01 |

Fig. 3

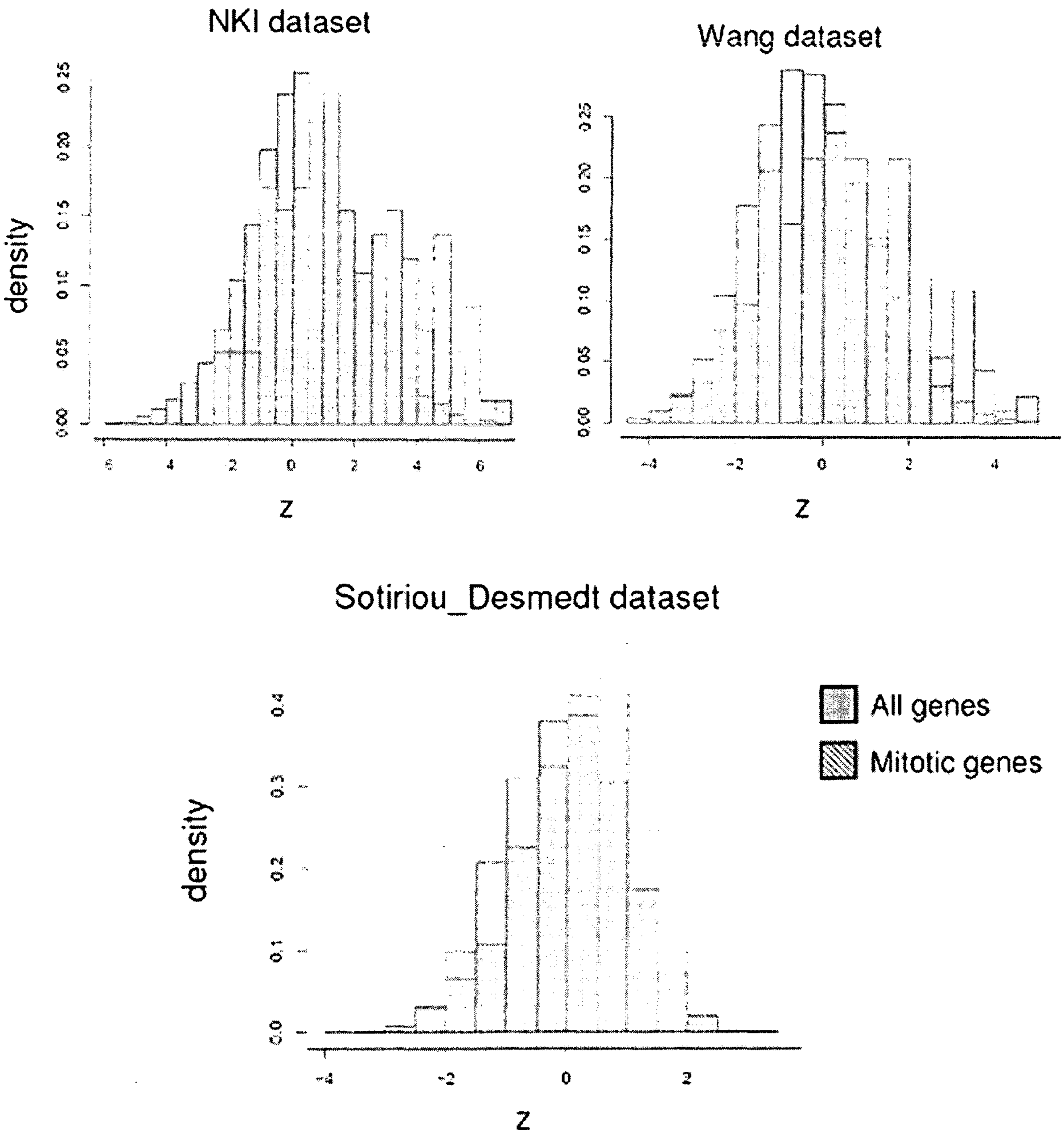
NKI dataset
Wang dataset
Sotiriou_Desmedt dataset
density
Z
All genes
Mitotic genes

Fig. 4

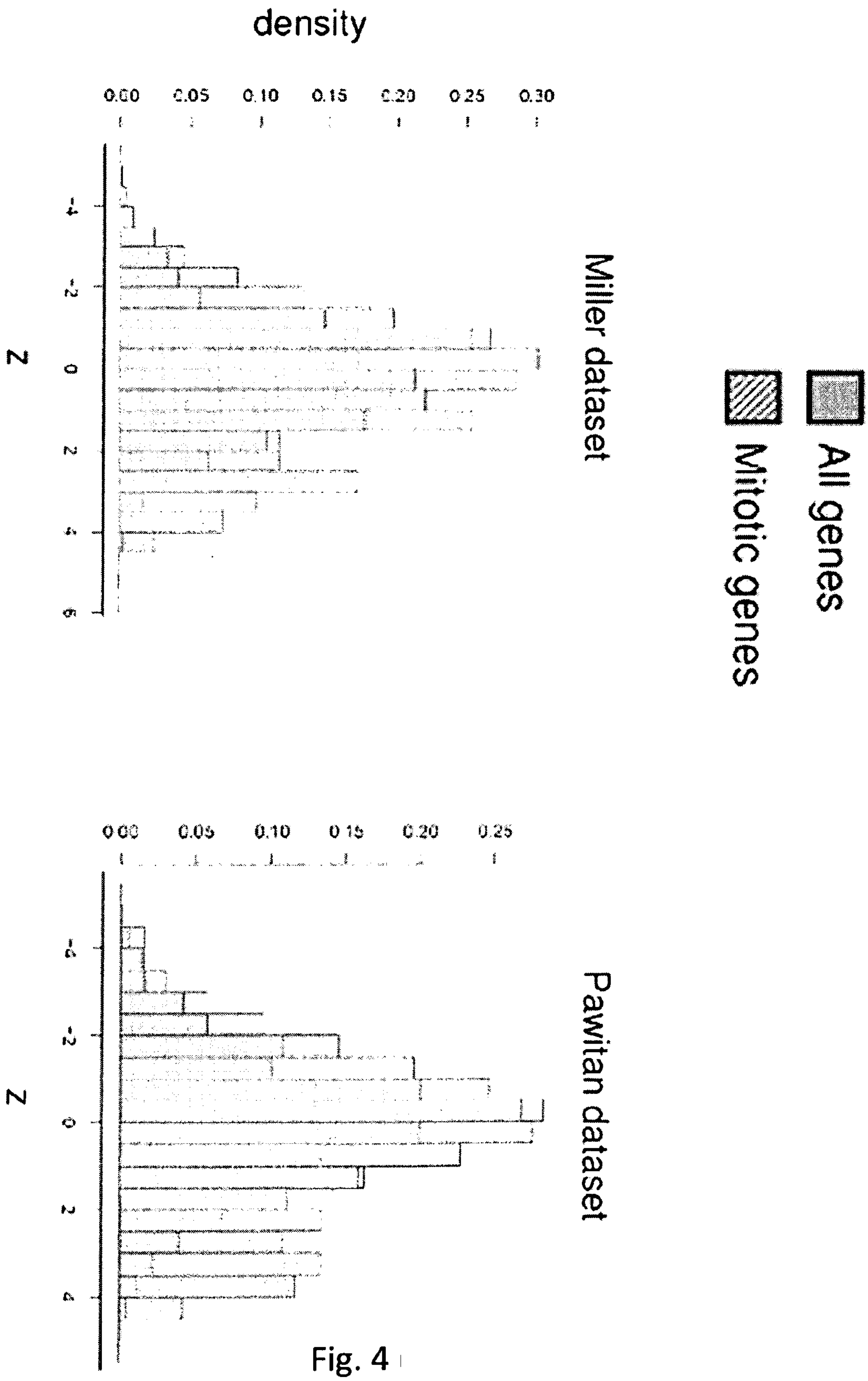
density
0.00
0.05
0.10
0.15
0.20
0.25
0.30
Z
Miller dataset
Pawitan dataset
All genes
Mitotic genes

Fig. 4

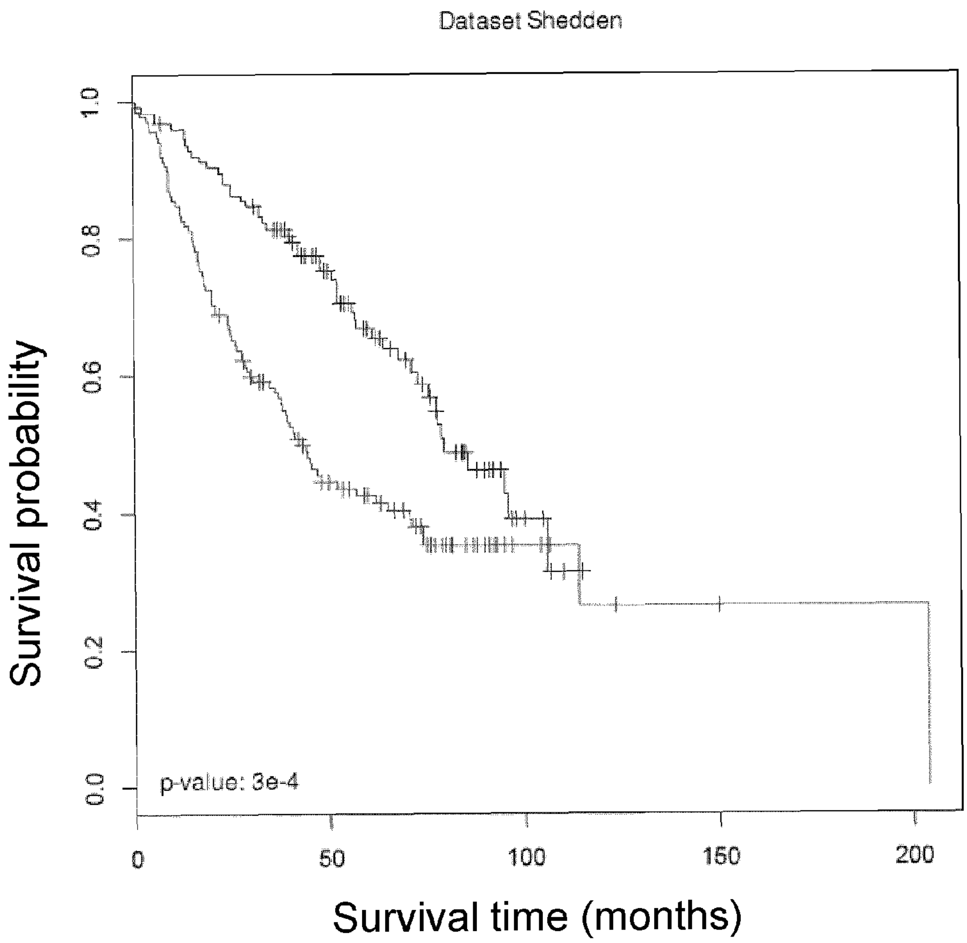
Dataset Shedden
Survival probability
1.0
0.8
0.6
0.4
0.2
0.0
p-value: 3e-4
0
50
100
150
200
Survival time (months)

Fig. 6

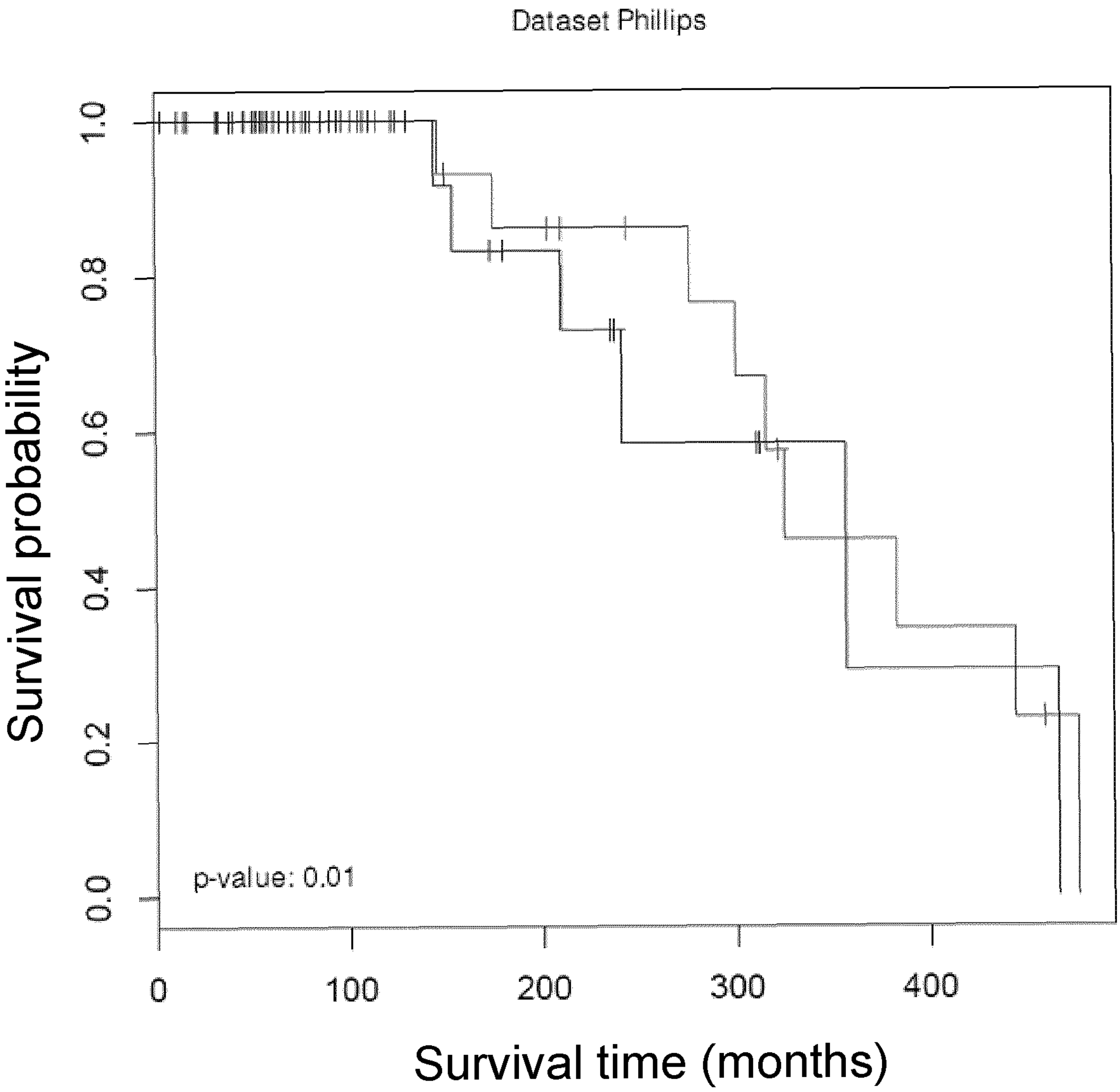
Dataset Phillips
Survival probability
1.0
0.8
0.6
0.4
0.2
0.0
p-value: 0.01
0
100
200
300
400
Survival time (months)

Fig. 7

Europäisches Patentamt
European Patent Office
Office européen des brevets

# EUROPEAN SEARCH REPORT

Application Number
EP 12 15 3511

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CARTER SCOTT L ET AL: "A signature of chromosomal instability inferred from gene expression profiles predicts clinical outcome in multiple human cancers", NATURE GENETICS, NATURE PUBLISHING GROUP, NEW YORK, US, vol. 38, no. 9, 1 September 2006 (2006-09-01), pages 1043-1048, XP002445768, ISSN: 1061-4036, DOI: 10.1038/NG1861 * the whole document * | 1-13 | INV. C12Q1/68 |
| A | WO 2009/067611 A1 (UNIV SOUTH FLORIDA [US]; H LEE MOFFITT CANCER CT AND RE [US]; YEATMAN) 28 May 2009 (2009-05-28) * the whole document * | 1-13 | |
| A | WO 2010/129965 A1 (UNIV CALIFORNIA [US]; HU ZHI [US]; MAO JIAN-HUA [US]; KUO WEN-LIN [US]) 11 November 2010 (2010-11-11) * the whole document * | 1-13 | TECHNICAL FIELDS SEARCHED (IPC) |
| X | "GeneChip Human Genome U133 Set", INTERNET CITATION , 26 February 2003 (2003-02-26), XP002232760, Retrieved from the Internet: URL:http://www.affymetrix.com/support/technical/datasheets/hgu133_datashe et.pdf * the whole document * | 8-10 | C12Q |
| | -/-- | | |

The present search report has been drawn up for all claims

1

| Place of search | Date of completion of the search | Examiner |
|---|---|---|
| The Hague | 14 May 2012 | Santagati, Fabio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 15 3511

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | CHRISTIAN DAMASCO ET AL: "A Signature Inferred from Drosophila Mitotic Genes Predicts Survival of Breast Cancer Patients", PLOS ONE, vol. 6, no. 2, 28 February 2011 (2011-02-28), page E14737, XP055005383, ISSN: 1932-6203, DOI: 10.1371/journal.pone.0014737 * the whole document * ----- | 1-13 | |
| | | | TECHNICAL FIELDS SEARCHED (IPC) |

The present search report has been drawn up for all claims

1

| Place of search | Date of completion of the search | Examiner |
|---|---|---|
| The Hague | 14 May 2012 | Santagati, Fabio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 15 3511

This annex lists the patent family members relating to the patent documents cited in the above-mentioned European search report.
The members are as contained in the European Patent Office EDP file on
The European Patent Office is in no way liable for these particulars which are merely given for the purpose of information.

14-05-2012

| Patent document cited in search report | Publication date | Patent family member(s) | Publication date |
|---|---|---|---|
| WO 2009067611 A1 | 28-05-2009 | US 2011046002 A1<br>WO 2009067611 A1 | 24-02-2011<br>28-05-2009 |
| WO 2010129965 A1 | 11-11-2010 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SAMBROOK et al.** Molecular Cloning - A laboratory manual. Cold Spring Harbor Laboratory, 1989, vol. 1-3
- **AUSUBEL et al.** Current Protocols in Molecular Biology. Current Protocol Publishing, 1994, vol. 2
- **DUPUY A ; SIMON RM.** *J Natl Cancer Inst,* 2007, vol. 99, 147-157
- **WIRAPATI P et al.** *Breast Cancer Res,* 2008, vol. 10, R65
- **VAN'T VEER LJ et al.** *Nature,* 2002, vol. 415, 530-536
- **VAN DE VIJVER MJ et al.** *N Engl J Med,* 2002, vol. 347, 1999-2009
- **CHANG HY et al.** *PLoS Biol,* 2004, vol. 2, E7
- **CHANG HY et al.** *Proc Natl Acad Sci USA,* 2005, vol. 102, 3738-3743
- **CHI JT et al.** *PLoS Med,* 2006, vol. 3, e47
- **SUNG FL et al.** *Cancer Lett,* 2007, vol. 253, 74-88
- **WINTER SC et al.** *Cancer Res,* 2007, vol. 67, 3441-3449
- **WHITFIELD ML et al.** *Mol Biol Cell,* 2002, vol. 13, 1977-2000
- **STARMANS MH et al.** *Br J Cancer,* 2008, vol. 99, 1884-1890
- **BEN-PORATH I et al.** *Nat Genet,* 2008, vol. 40, 499-507
- **REYAL F et al.** *Breast Cancer Res,* 2008, vol. 10, R93
- **LIU R et al.** *N Engl J Med,* 2007, vol. 356, 217-226
- **CARTER SL et al.** *Nat Genet,* 2006, vol. 38, 1043-1048
- **SOMMA MP et al.** *PLoS Genet,* 2008, vol. 4, e1000126
- **SAYERS EW et al.** *Nucleic Acids Res,* 2010, vol. 38, D5-16
- **SHEDDEN K et al.** *Nat Med,* 2008, vol. 14, 822-827
- **PHILLIPS HS et al.** *Cancer Cell,* 2006, vol. 9, 157-173
- **PAWITAN Y et al.** *Breast Cancer Res,* 2005, vol. 7, R953-964
- **MILLER LD et al.** *Proc Natl Acad Sci USA,* 2005, vol. 102, 13550-13555
- **WANG Y et al.** *Lancet,* 2005, vol. 365, 671-679
- **DESMEDT C et al.** *Clin Cancer Res,* 2007, vol. 13, 3207-3214
- **SOTIRIOU C et al.** *J Natl Cancer Inst,* 2006, vol. 98, 262-272
- **WILSON CL ; MILLER CJ.** *Bioinformatics,* 2005, vol. 21, 3683-3685
- **GENTLEMAN RC et al.** *Genome Biol,* 2004, vol. 5, R80
- **BILD AH et al.** *Nature,* 2006, vol. 439, 353-357
- **FREIJE WA et al.** *CancerRes,* 2004, vol. 64, 6503-6510
- **ZHAO H et al.** *PLoS Med,* 2006, vol. 3, e13
- **EGGERT US ; MITCHISON TJ ; FIELD CM.** *Annu Rev Biochem,* 2006, vol. 75, 543-566
- **GALANTY Y et al.** *Nature,* 2009, vol. 462, 935-939
- **COPPOLA D et al.** *J Cancer Res Clin Oncol,* 2009, vol. 135, 1287-1291
- **WHITFIELD ML ; GEORGE LK ; GRANT GD ; PEROU CM.** *Nat Rev Cancer,* 2006, vol. 6, 99-106
- **PAIK S et al.** *N Engl J Med,* 2004, vol. 351, 2817-2826
- **SUZUKI C et al.** *Cancer Res,* 2005, vol. 65, 11314-11325
- **TAMURA K et al.** *Cancer Res,* 2007, vol. 67, 5117-5125
- **SKRZYPSKI M et al.** *Clin Cancer Res,* 2008, vol. 14, 4794-4799
- **FIELDS AP ; JUSTILIEN V.** *Adv Enzyme Regul.,* 2009
- **HORVATH S et al.** *Proc Natl Acad Sci USA,* 2006, vol. 103, 17402-17407
- **LIN SY et al.** *Clin Cancer Res,* 2008, vol. 14, 4814-4820
- **SHIMO A et al.** *Cancer Sci,* 2007, vol. 98, 174-181
- **PELLEGRINO R et al.** *Hepatology,* 2009
- **SCHMIT TL et al.** *J Invest Dermatol,* 2009, vol. 129, 2843-2853
- **SHIMIZU S et al.** *Oncol Rep,* 2007, vol. 18, 1489-1497

SEQUENCE LISTING

<110> Consiglio Nazionale delle Ricerche
Somma, Maria Patrizia
Gatti, Maurizio
Provero, Paolo
Di Cunto, Ferdinando
Damasco, Christian
Lembo, Antonio

<120> Markers to predict survival of breast cancer patients and uses thereof

<130> BE 115428

<150> RM2011A000044
<151> 2011-02-01

<160> 217

<170> PatentIn version 3.5

<210> 1
<211> 457
<212> DNA
<213> Homo sapiens

<220>
<221> misc_feature
<222> (87)..(87)
<223> n is a, c, g, or t

<400> 1
catggtttac atttactcag ctactatata tgcagtgtgg tgcacatttt cacagaattc 60

tggcttcatt aagatcatta tttttgnctg cgtagcttac agacttagca tattagtttt 120

ttctactcct acaagtgtaa attgaaaaat ctttatatta aaaaagtaaa ctgttatgaa 180

gctgctatgt actaataata ctttgcttgc caaagtgttt gggttttgtt gttgtttgtt 240

tgtttgtttg tttttggttc atgaacaaca gtgtctagaa acccattttg aaagtggaaa 300

attattaagt cacctatcac ctttaaacgc cttttttttaa aattataaaa tattgtaaag 360

cagggtctca acttttaaat acactttgaa cttcttctct gaattattaa agttctttat 420

gacctcattt ataaacacta aattctgtca cctcctg 457

<210> 2
<211> 115
<212> DNA
<213> Homo sapiens

<400> 2
gccgcctact actactatac ggctgcgaga agacgacaga agggactggc ctctgcccac 60

accttgactt cagtatttct gacctcctaa actctaataa agtcatgctt acagc 115

<210> 3
<211> 375
<212> DNA

<213> Homo sapiens

<400> 3
ttttaccttg gatgctgact tctaaatgaa ctgaagatgt gcccttactt ggctgatttt 60

ttttttccat ctcataagaa aaatcagctg aagtgttacc aactagccac accatgaatt 120

gtccgtaatg ttcattaaca gcatctttaa aactgtgtag ctacctcaca accagtcctg 180

tctgtttata gtgctggtag tatcaccttt tgccagaagg cctggctggc tgtgacttac 240

catagcagtg acaatggcag tcttggcttt aaagtgaggg gtgacccttt agtgagctta 300

gcacagcggg attaaacagt cctttaacca gcacagccag ttaaaagatg cagcctcact 360

gcttcaacgc agatt 375

<210> 4
<211> 387
<212> DNA
<213> Homo sapiens

<220>
<221> misc_feature
<222> (166)..(166)
<223> n is a, c, g, or t

<400> 4
ccaccttcat tggcaacagc acggccatcc aggagctgtt caagcgcatc tccgagcagt 60

tcacggccat gttccggcgc aaggccttcc tgcactggta cacgggcgag ggcatggacg 120

agatggagtt caccgaggcc gagagcaaca tgaatgacct ggtgtncgag taccagcagt 180

accaggatgc cacagccgag gaggagggcg agttcgagga ggaggctgag gaggaggtgg 240

cctagagcct tcagtcactg gggaaagcag ggaagcagtg tgaactcttt attcactccc 300

agcctgtcct gtggcctgtc ccactgtgtg cacttgctgt tttccctgtc cacatccatg 360

ctgtacagac accaccatta aagcatt 387

<210> 5
<211> 523
<212> DNA
<213> Homo sapiens

<400> 5
ttcaggggcc gcatgtccat gaaggaggtg gatgagcaaa tgcttaatgt ccaaaacaaa 60

aacagcagct attttgttga gtggatcccc aacaatgtga aaacggctgt ctgtgacatc 120

ccacctcggg ggctaaaaat gtccgccacc ttcattggca acagcacggc catccaggag 180

ctgttcaagc gcatctccga gcagttcacg gccatgttcc ggcgcaaggc cttcctgcac 240

tggtacacgg gcgagggcat ggacgagatg gagttcaccg aggccgagag caacatgaat 300

gacctggtgt ccgagtacca gcagtaccag gatgccacag ccgaggagga gggcgagttc 360

gaggaggagg ctgaggagga ggtggcctag agccttcagt cactggggaa agcagggaag 420

cagtgtgaac tctttattca ctcccagcct gtcctgtggc ctgtcccact gtgtgcactt 480

gctgttttcc ctgtccacat ccatgctgta cagacaccac cat 523

<210> 6
<211> 516
<212> DNA
<213> Homo sapiens

<400> 6
gttgtaatcg cagtattcct tgtatggaag tcatcagata tgctgtgcaa gtcttgctta 60

atgtatctaa gtatgagaaa actacttcag cagtttatga tgtagaaaat tgtatagata 120

tactattgga gcttttgcag atataccgag aaaagcctgg taataaagtt gcagacaaag 180

gcggaagcat ttttacaaaa acttgttgtt tgttggctat tttactgaag acaacaaata 240

gagcctctga tgtacgaagt aggtccaaag ttgttgaccg tatttacagt ctctacaaac 300

ttacagctca taaacataaa atgaatactg aaagaatact ttacaagcaa aagaagaatt 360

cttctataag cattcctttt atcccagaaa cacctgtaag gaccagaata gtttcaagac 420

ttaagccaga ttgggttttg agaagagata acatggaaga aatcacaaat cccctgcaag 480

ctattcaaat ggtgatggat acgcttggca ttcctt 516

<210> 7
<211> 463
<212> DNA
<213> Homo sapiens

<220>
<221> misc_feature
<222> (61)..(61)
<223> n is a, c, g, or t

<400> 7
tttgcacttc cttcggagag catctaagat tggagaggtt gatgtcgagc aacatacttt 60

ngccaaatac ctgatggaac taactatgtt ggactatgac atggtgcact ttcctccttc 120

tcaaattgca gcaggagctt tttgcttagc actgaaaatt ctggataatg gtgaatggac 180

accaactcta caacattacc tgtcatatac tgaagaatct cttcttccag ttatgcagca 240

cctggctaag aatgtagtca tggtaaatca aggacttaca aagcacatga ctgtcaagaa 300

caagtatgcc acatcgaagc atgctaagat cagcactcta ccacagctga attctgcact 360

agttcaagat ttagccaagg ctgtggcaaa ggtgtaactt gtaaacttga gttggagtac 420

tatatttaca aataaaattg gcaccatgtg ccatctgtac ata 463

<210> 8
<211> 487
<212> DNA
<213> Homo sapiens

<400> 8

gatgatttct cgaaagccat gccagaagca gtcttccagg tcatcttgta gaactccagc 60

tttgttgaaa atcacggacc tcagctacat catacactga cccagagcaa agctttccct 120

atggttcaaa gacaactagt attcaacaaa ccttgtatag tgtatgtttt gccatattta 180

atattaatag cagaggaaga ctcctttttt catcactgta tgaatttttt ataatgtttt 240

tttaaaatat atttcatgta tacttataaa ctaattcaca caagtgtttg tcttagatga 300

ttaaggaaga ctatatctag atcatgtctg atttttattt gtgacttctc cagccctggt 360

ctgaatttct taaggtttta taaacaaatg ctgctattta ttagctgcaa gaatgcactt 420

tagaactatt tgacaattca gactttcaaa ataaagatgt aaatgactgg ccaataataa 480

ccatttt 487

<210> 9
<211> 378
<212> DNA
<213> Homo sapiens

<400> 9
gctactttga attaatctgc ctttatgttt gggagaagaa agctgagaca ttgcatgaaa 60

gatgatgaga gataaatgtt gatcttttgg ccccatttgt taattgtatt cagtatttga 120

acgtcgtcct gtttattgtt agttttcttc atcatttatt gtatagacaa tttttaaatc 180

tctgtaatat gatacatttt cctatctttt aagttattgt tacctaaagt taatccagat 240

tatatggtcc ttatatgtgt acaacattaa aatgaaaggc tttgtcttgc attgtgaggt 300

acaggcggaa gttggaatca ggttttagga ttctgtctct cattagctga ataatgtgag 360

gattaacttc tgccagct 378

<210> 10
<211> 538
<212> DNA
<213> Homo sapiens

<400> 10
tgctacggta acttcatcag cccgccaaga tggcgatgca agcggccaag agggcgaaca 60

ttcgacttcc acctgaagta aatcggatat tgtatataag aaatttgcca tacaaaatca 120

cagctgaaga aatgtatgat atatttggga aatatggacc tattcgtcaa atcagagtgg 180

ggaacacacc tgaaactaga ggaacagctt atgtggtcta tgaggacatc tttgatgcca 240

agaatgcatg tgatcaccta tcgggattca atgtttgtaa cagatacctt gtggttttgt 300

actataatgc caacagggca tttcagaaga tggacacaaa gaagaaggag gaacagttga 360

agcttctcaa ggagaaatat ggcatcaaca cagatccacc aaaataaatg ttttctacat 420

tttcatttgg actaaatccc acgaatgaca actaccacct ttttttcctt tttaattaat 480

actaaatatt gtgatttctt atttgaggtt caaaatgacc tgcttgaaac tttgatac 538

<210> 11
<211> 357
<212> DNA
<213> Homo sapiens

<220>
<221> misc_feature
<222> (142)..(142)
<223> n is a, c, g, or t

<220>
<221> misc_feature
<222> (192)..(192)
<223> n is a, c, g, or t

<400> 11
gaaagagcta aaacgtcatc ctctcttcag tgatgtggac tgggaaaatc tgcagcatca 60

gactatgcct ttcatccccc agccagatga tgaaacagat acctcctatt ttgaagccag 120

gaatactgct cagcacctga cngtatctgg atttagtctg tagcacaaaa attttccttt 180

tagtctagcc tngtgttata gaatgaactt gcataattat atactcctta atactagatt 240

gatctaaggg ggaaagatca ttatttaacc tagttcaatg tgcttttaat gtacgttaca 300

gctttcacag agttaaaagg ctgaaaggaa tatagtcagt aatttatctt aacctca 357

<210> 12
<211> 420
<212> DNA
<213> Homo sapiens

<220>
<221> misc_feature
<222> (81)..(81)
<223> n is a, c, g, or t

<220>
<221> misc_feature
<222> (87)..(87)
<223> n is a, c, g, or t

<220>
<221> misc_feature
<222> (92)..(92)
<223> n is a, c, g, or t

<220>
<221> misc_feature
<222> (95)..(95)
<223> n is a, c, g, or t

<220>
<221> misc_feature
<222> (119)..(119)
<223> n is a, c, g, or t

<220>
<221> misc_feature
<222> (130)..(130)
<223> n is a, c, g, or t

<220>
<221> misc_feature
<222> (185)..(185)
<223> n is a, c, g, or t

<220>
<221> misc_feature
<222> (188)..(188)
<223> n is a, c, g, or t

<220>
<221> misc_feature
<222> (192)..(192)
<223> n is a, c, g, or t

<400> 12
gtacaactcg tatttatctc tgatgtgccg ctgctgaact ttgggttcat ttggggtcaa 60

agccagtttt tcttttaaaa nttgaanttc antcntgatg cttggccccc atacccccna 120

accttgtccn agtggagccc aacttctaaa ggtcaatata tcatcctttg gcatcccaac 180

taacnatnaa gnagtaggct ataagggaag attgtcaata ttttgtggta agaaaagcta 240

cagtcatttt ttctttgcac tttggatgct gaaatttttc ccatggaaca tagccacatc 300

tagatagatg tgagcttttt cttctgttaa aattattctt aatgtctgta aaaacgattt 360

tcttctgtag aatgtttgac ttcgtattga cccttatctg taaaacacct atttgggata 420

<210> 13
<211> 501
<212> DNA
<213> Homo sapiens

<400> 13
gtgtccgaag ttgagatggc ctgccctact ggcaaagagg tgacaggaag gctgggagca 60

gctttgttaa attgtgttca gttctgttac acagtgcatt gccctttgtt gggggtatgc 120

atgtatgaac acacatgctt gtcggaacgc tttctcggcg tttgtccctt ggctctcatc 180

tcccccattc ctgtgcctac tttgcctgag ttcttctacc cccgcagttg ccagccagat 240

tgggagtctg tttgttccaa tgggttgagc tgtctttgtc gtggagatct ggaactttgc 300

acatgtcact actggggagg tgttcctgct ctagcttcca cgatgaggcg ccctctttac 360

ctatcctctc aatcactact cttcttgaag cactattatt tattcttccg ctgtctgcct 420

gcagcagtac tactgtcaac atagtgtaaa tggttctcaa aagcttacca gtgtggactt 480

ggtgttagcc acgctgttta c 501

<210> 14
<211> 444
<212> DNA
<213> Homo sapiens

<400> 14
tttgaatgtg gttacttcct actgtagggt agcggaaaag ttgtcttaaa aggtatggtg 60

gggatatttt taaaaactcc ttttggttta cctggggatc caattgatgt atatgtttat 120

atactgggtt cttgttttat atacctggct tttactttat taatatgagt tactgaaggt 180

gatggaggta tttgaaaatt ttacttccat aggacatact gcatgtaagc caagtcatgg 240

agaatctgct gcatagctct attttaaagt aaaagtctac caccgaatcc ctagtccccc 300

tgttttctgt ttcttcttgt gattgctgcc ataattctaa gttatttact tttaccacta 360

tttaagttat caactttagc tagtatcttc aaactttcac tttgaaaaat gagaatttta 420

tattctaagc cagttttcat tttg 444

<210> 15
<211> 372
<212> DNA
<213> Homo sapiens

<400> 15
gtaacactct ggtacagatc tccagaagta ttgctggggt cagctcgtta ctcaactcca 60

gttgacattt ggagtatagg caccatattt gctgaactag caactaagaa accacttttc 120

catggggatt cagaaattga tcaactcttc aggattttca gagctttggg cactcccaat 180

aatgaagtgt ggccagaagt ggaatcttta caggactata agaatacatt tcccaaatgg 240

aaaccaggaa gcctagcatc ccatgtcaaa aacttggatg aaaatggctt ggatttgctc 300

tcgaaaatgt taatctatga tccagccaaa cgaatttctg gcaaaatggc actgaatcat 360

ccatatttta at 372

<210> 16
<211> 468
<212> DNA
<213> Homo sapiens

<220>
<221> misc_feature
<222> (399)..(399)
<223> n is a, c, g, or t

<400> 16
tgctaagttc aagtttcgta atgctttgaa gtatttttat gctctgaatg tttaaatgtt 60

ctcatcagtt tcttgccatg ttgttaacta tacaacctgg ctaaagatga atatttttct 120

actggtattt taatttttga cctaaatgtt taagcattcg gaatgagaaa actatacaga 180

tttgagaaat gatgctaaat ttataggagt tttcagtaac ttaaaaagct aacatgagag 240

catgccaaaa tttgctaagt cttacaaaga tcaagggctg tccgcaacag ggaagaacag 300

ttttgaaaat ttatgaacta tcttattttt aggtaggttt tgaaagcttt ttgtctaagt 360

gaattcttat gccttggtca gagtaataac tgaaggagnt gcttatcttg gctttcgagt 420

ctgagtttaa aactacacat tttgacatag tgtttattag cagccatc 468

<210> 17
<211> 462
<212> DNA
<213> Homo sapiens

<400> 17
ctgtcaaggc cgtagcatgg tgtccctggc agtccaatgt cctggcaaca ggagggggca 60

ccagtgatcg acacattcgc atctggaatg tgtgctctgg ggcctgtctg agtgccgtgg 120

atgcccattc ccaggtgtgc tccatcctct ggtctcccca ttacaaggag ctcatctcag 180

gccatggctt tgcacagaac cagctagtta tttggaagta cccaaccatg gccaaggtgg 240

ctgaactcaa aggtcacaca tcccgggtcc tgagtctgac catgagccca gatggggcca 300

cagtggcatc cgcagcagca gatgagaccc tgaggctatg gcgctgtttt gagttggacc 360

ctgcgcggcg gcgggagcgg gagaaggcca gtgcagccaa aagcagcctc atccaccaag 420

gcatccgctg aagaccaacc catcacctca gttgtttttt at 462

<210> 18
<211> 518
<212> DNA
<213> Homo sapiens

<400> 18
gtgaagacat caagagctcg aagtgtaaat tacccgaaca agaatcacta ccaaatgata 60

acaaagacat tttacaacgg cttgatcctt cttcattctc aactaagcat tctatgcctg 120

taccaagcat ggtgccatcc tacatggcaa tgactactgc tgccaaaagg aaacggaaat 180

taacaagttc tacatcaaac agttcgttaa ctgcagacgt aaattctgga tttgccaaac 240

gtgttcgaca agataattca agtgagaagc acttacaaga aaacaaacca acaatggaac 300

ataaaagaaa catctgtaaa ataaatccaa gcatggttag aaaatttgga agaaatattt 360

caaaaggaaa tctaagataa atcacttcaa aaccaagcaa aatgaagttg atcaaatctg 420

cttttcaaag tttatcaata ccctttcaaa aatatattta aaatctttga aagaagaccc 480

atcttaaagc taagtttacc caagtacttt cagcaagc 518

<210> 19
<211> 547
<212> DNA
<213> Homo sapiens

<400> 19
cagatttctg gggagcactc cagccgaggc ctgcagtgtg gggccccccct cagtcttgtg 60

gacctggccg ggagtgagcg acttgacccc ggcttagccc tcggccccgg ggagcgggaa 120

cgccttcggg aaacacaggc cattaacagc agcctgtcca cgctggggct ggttatcatg 180

gccctgagca acaaggagtc ccacgtgcct taccggaaca gcaaactgac ctacctgctg 240

cagaactctc tgggtggtag tgctaagatg ctcatgtttg tgaacatttc tccactggaa 300

gagaacgtct ccgagtccct caactctcta cgctttgcct ccaaggtgaa ccagtgtgtt 360

attggtactg ctcaggccaa caggaagtga agacggatcc agatctgtgt gtgtgtgtgt 420

gtgtgtgtgt gtgtgtgtgt gtgtgtgtgt gtgtgtgtcc ctatgtctat gtatcgggtg 480

aggggtggga gggttgctgg agggtgcttt attgggtgga gggcaccatg tcccagggct 540

atcaaat 547

<210> 20
<211> 407
<212> DNA
<213> Homo sapiens

<400> 20
tacagatact ctactacact cagcctctta tgtgccaagt ttttctttaa gcaatgagaa 60

attgctcatg ttcttcatct tctcaaatca tcagaggccg aagaaaaaca ctttggctgt 120

gtctataact tgacacagtc aatagaatga agaaaattag agtagttatg tgattatttc 180

agctcttgac ctgtcccctc tggctgcctc tgagtctgaa tctcccaaag agagaaacca 240

atttctaaga ggactggatt gcagaagact cggggacaac atttgatcca agatcttaaa 300

tgttatattg ataaccatgc tcagcaatga gctattagat tcattttggg aaatctccat 360

aatttcaatt tgtaaacttt gttaagacct gtctacattg ttatatg 407

<210> 21
<211> 479
<212> DNA
<213> Homo sapiens

<220>
<221> misc_feature
<222> (352)..(356)
<223> n is a, c, g, or t

<400> 21
cctcctccac agctaacata gacgacttga aaaaaagact ggagagaata aagagcagtc 60

gcaaatgaag ctgccccact cccccggcac cctgcagctt tagtttacta aactagaagt 120

cctcatagtt taaaatggcc tcagcaggcc tagtgtatac aaactggttg tatgtatcat 180

gccgtggagc taggggggagg agtcattgtg gcacaagtat ttgtacatac tctgcttctc 240

tctgtcagcg tcctgctgct ctagaagact gtccgtggat gagtttagtg tacagacttg 300

taaacagctg ccccctctct gctcagtcta gttcccagat ccttttcttt tnnnnntaat 360

tgctcatttg taaaattgtc ctaatctttc ctagcttttt aatagttaat attagaaact 420

ctttaatagt tttcctttca gtttgtgagc tcttctctgt cgccctgaag ggtcactgt 479

<210> 22
<211> 220
<212> DNA
<213> Homo sapiens

<400> 22
ggacgaacag gaggtgtcag actgctgaag ccgactctga aagtgatcat gaagttccag 60

aaccagaatc agaaatgaag atgagactac caagacgagc caaaaccgca gcactagaaa 120

aaagtaaact taaccttgcc caatttctca atgaagatct aagttaggaa agacgatgga 180

ggtggaatcc tttaagatta tgtccagtta tttgctttaa 220

<210> 23
<211> 408
<212> DNA
<213> Homo sapiens

<400> 23
agctgacggc ggagctgatc gagcaggcgg cgcagtacac caacgcggtg cgcgaccggg 60

agctggacct ccgggggtga tctgaaccct ctggcatctc tcaaatcgct gacttaccta 120

agtatcctaa gaaatccggt aaccaataag aagcattaca gattgtatgt gatttataag 180

gttccgcaag tcagagtact ggatttccag aaagtgaaac taaaatttta atccaggtgc 240

tggtttgcca actgacaaaa agaaaggtgg gccatctcca ggggatgtag aagcaatcaa 300

gaatgccata gcaaatgctt caactctggc tgaagtggag aggctgaagg ggttgctgca 360

gtctggtcag atccctggca gagaacgcag atcagggccc actgatga 408

<210> 24
<211> 353
<212> DNA
<213> Homo sapiens

<400> 24
aattcgagtc tatacaaaag ccttgagttc tttagaactc agtagccatc ttgcaaaaga 60

tcttctggtt ctattgaatg agattctgga gcaagtaaaa gataggacat gtctgagagc 120

tttggagaaa atcaagattc agttagaaaa aggaaataaa gaatttggtg accaagctga 180

agcagcacag gatgccacct tgactacaac tactttccaa aatgaagatg aaaagaataa 240

agaagtatat atgactccac tcaggggtgt aaaagcaacc caagcatcaa agtctactca 300

gctaaagact aacagaggac agagaaaagt gacagtttca gctaggacga aca 353

<210> 25
<211> 481
<212> DNA
<213> Homo sapiens

<400> 25
tggatccagc acttatcaga ccaggccgca ttgacaggaa gattgagttc cccctgcctg 60

atgaaaagac gaagaagcgc atctttcaga ttcacacaag caggatgacg ctggctgatg 120

atgtaaccct ggacgacctg atcatggcta aagatgacct ctctggtgct gacatcaagg 180

caatctgtac agaagctggt ctgatggcct taagagaacg tagaatgaaa gtaacaaatg 240

aagacttcaa aaaatctaaa gaaaatgttc tttataagaa acaggaaggc acccctgagg 300

ggctgtatct ctaatgaacc atggctgtca tcaggaaaat ggttgggaga tttctcaatc 360

cctgaaaggg atgaggttgg gggagttgcc cagaggaatc cctgttccca ctgattttta 420

ttagcaaaac atcctgtgtc ttttggagta cgatgtgtaa gtgcccattg ggtggcctgt 480

t 481

<210> 26
<211> 563
<212> DNA
<213> Homo sapiens

<400> 26
atggacgagg tcctgtacag catcgccgag aaggttaaaa attttgcagt tatttatctt 60

gtggatatta cagaagtgcc tgacttcaac aaaatgtatg agttatacga tccatgtact 120

gtcatgtttt tcttcaggaa caagcacatc atgattgact tggggactgg caacaacaac 180

aagattaact gggccatgga ggacaagcag gagatggtgg acatcatcga gacggtgtac 240

cgcggggccc gcaaaggccg cggcctggtg gtgtcccccca aggactactc caccaagtac 300

cgctactgag gcgccctcag tctgcgcgga taaatgtcgt ggagcccttt ttgtatggaa 360

acgttttaag ctatttaaag cctttggaaa atacaggaag ctccagggct ggagcacctc 420

tgagatggaa ttgataacat ggtcttaact caccgaaata aacaagcacg tggtgagagg 480

agcaggccta cttgtttgtt ctcaggaaac ttaatgaata gattactgat tttcctagtc 540

aaagttaatt cttacccttg gag 563

<210> 27
<211> 524
<212> DNA
<213> Homo sapiens

<400> 27
acgccgcgcg aaggtgatga gctcgcccgg ctgccctacc tacggacctg gttccgcacc 60

cgcagcgcca tcatcctgca cctcagcaac ggcagcgtgc agatcaactt cttccaggat 120

cacaccaagc tcatcttgtg cccactgatg gcagccgtga cctacatcga cgagaagcgg 180

gacttccgca cataccgcct gagtctcctg gaggagtacg gctgctgcaa ggagctggcc 240

agccggctcc gctacgcccg cactatggtg gacaagctgc tgagctcacg ctcggccagc 300

aaccgtctca aggcctccta atagctgccc tcccctccgg actggtgccc tcctcactcc 360

cacctgcatc tggggcccat actggttggc tcccgcggtg ccatgtctgc agtgtgcccc 420

ccagccccgg tggctgggca gagctgcatc atccttgcag gtgggggttg ctgtataagt 480

tatttttgta catgttcggg tgtgggttct acagacttgt cccc 524

<210> 28
<211> 282
<212> DNA

<213> Homo sapiens

<400> 28
gaatgaagcc atcaggctaa tggagatgtc aaaggactct cttctaggag acaaggggca 60

gacagctagg actcagagac cagcagatgt gatatttgcc accgtccgtg aactggtctc 120

agggggccga agtgtccggt tctctgaggc agagcagcgc tgtgtatctc gtggcttcac 180

acccgcccag ttccaggcgg ctctggatga atatgaggag ctcaatgtct ggcaggtcaa 240

tgcttcccgg acacggatca cttttgtctg attccagcct gc 282

<210> 29
<211> 406
<212> DNA
<213> Homo sapiens

<400> 29
aagcccactt tagagtatac attgctatta tgggagacca cccagacatc tgactaatgg 60

ctctgtgcca cactccaaga cctgtgcctt ttagagaagc tcacaatgat ttaaggactg 120

tttgaaactt ccaattatgt ctataattta tattcttttg tttacatgat gaaacttttt 180

gttgttgctt gtttgtatat aatacaatgt gtacatgtat ctttttctcg attcaaatct 240

taacccttag gactctggta tttttgatct ggcaaccata tttctggaag ttgagatgtt 300

tcagcttgaa gaaccaaaac agaaggaata tgtacaaaga ataaattttc tgctcacgat 360

gagtttagtg tgtaaagttt agagacatct gactttgata gctaaa 406

<210> 30
<211> 451
<212> DNA
<213> Homo sapiens

<400> 30
tgaacgtctg tgacaacctg ggagaccacc tggtggggaa cgtgtacgtc aagtttcgcc 60

gtgaggaaga tgcggaaaag gctgtgattg acttgaataa ccgttggttt aatggacagc 120

cgatccacgc cgagctgtca cccgtgacgg acttcagaga agcctgctgc cgtcagtatg 180

agatgggaga atgcacacga ggcggcttct gcaacttcat gcatttgaag cccatttcca 240

gagagctgcg gcgggagctg tatggccgcc gtcgcaagaa gcatagatca agatcccgat 300

cccgggagcg tcgttctcgg tctagagacc gtggtcgtgg cggtggcggt ggcggtggtg 360

gaggtggcgg cggacgggag cgtgacagga ggcggtcgag agatcgtgaa agatctgggc 420

gattctgagc catgccattt ttaccttatg t 451

<210> 31
<211> 458
<212> DNA
<213> Homo sapiens

<400> 31
ggggattgtg ttttgtcact ctagaagagt tcttcacaga gacttaaaac ctcaaaatct 60

```
cttgattgat gacaaaggaa caattaaact ggctgatttt ggccttgcca gagcttttgg      120

aatacctatc agagtatata cacatgaggt agtaacactc tggtacagat ctccagaagt      180

attgctgggg tcagctcgtt actcaactcc agttgacatt tggagtatag gcaccatatt      240

tgctgaacta gcaactaaga aaccactttt ccatggggat tcagaaattg atcaactctt      300

caggattttc agagctttgg gcactcccaa taatgaagtg tggccagaag tggaatcttt      360

acaggactat aagaatacat ttcccaaatg gaaaccagga agcctagcat cccatgtcaa      420

aaacttggat gaaaatggct tggatttgct ctcgaaaa                              458


<210>  32
<211>  532
<212>  DNA
<213>  Homo sapiens

<400>  32
aaagctcagg attcttcgaa aagttgagaa aattgatgac ttcaaagctg aagactttca       60

gattgaaggg tacaatccgc atccaactat taaaatggaa atggctgttt agggtgcttt      120

caaaggagct tgaaggatat tgtcagtctt taggggttgg gctggatgcc gaggtaaaag      180

ttctttttgc tctaaaagaa aaaggaacta ggtcaaaaat ctgtccgtga cctatcagtt      240

attaattttt aaggatgttg ccactggcaa atgtaactgt gccagttctt tccataataa      300

aaggctttga gttaactcac tgagggtatc tgacaatgct gaggttatga acaaagtgag      360

gagaatgaaa tgtatgtgct cttagcaaaa acatgtatgt gcatttcaat cccacgtact      420

tataaagaag gttggtgaat ttcacaagct atttttggaa tatttttaga atattttaag      480

aatttcacaa gctattccct caaatctgag ggagctgagt aacaccatcg at              532


<210>  33
<211>  401
<212>  DNA
<213>  Homo sapiens

<400>  33
ggacgccgaa ctcgagcttg tagacagctt ggatcctttc tctgaaggaa tcatgttcag       60

tgttcgacca cctaagaaaa gttggaaaaa gatcttcaac ctttcgggag gagagaaaac      120

acttagttca ttggctttag tatttgctct tcaccactac aagcccactc ccctttactt      180

catggatgag attgatgcag cccttgattt taaaaatgtg tccattgttg cattttatat      240

atatgaacaa acaaaaaatg cacagttcat aataatttct cttcgaaata atatgtttga      300

gatttcggat agacttattg gaatttacaa gacatacaac ataacaaaaa gtgttgctgt      360

aaatccaaaa gaaattgcat ctaagggact ttgttgaact t                          401


<210>  34
<211>  460
<212>  DNA
```

<213> Homo sapiens

<400> 34
tcatctgagg aggcctcgtc tctgaacttg ggttgtgccg agagagtttg ttctgtgttt 60

cccaccctct ccctgaccca agtctttgcc tctactccct taacagtgtt gaattcaact 120

gaaggcgagg aatgttggtg atgaagctga gttcaggact cggtggaccc tttgggaatg 180

ggtcatgaaa gctgccatgg ggtgaggaaa gaggagacag tgggagagga caatgactat 240

tgcatcttca ttgcaaaagc actggctcat ccgccctact tcccatccca cacaaaccca 300

attgtaaata acatatgact tctgagtact tttgggggca caactgtttt ctgtttgctg 360

tttttttgtt ttgttttttt tctccagagc actttggtct agactaggct ttgggtggtt 420

ccaattggtg gagagaagct ctgaggcacg tcatgcaggt 460

<210> 35
<211> 511
<212> DNA
<213> Homo sapiens

<400> 35
gagtcgccag gcagtatatc cagttagaac ttccggcttt tgcattagct tgtctgatgc 60

tcatgcccca ctcagagaaa agacaccagc aaattaagaa ttttctgggt tcctgtgacc 120

ctcaggttat tttaaagcaa ttggaagagc atatgaacac gggccagcta gcaggatttt 180

cacatcaaat tagaagtctg attttgaata atatcatcaa taagaaggag tttgggattt 240

tggcaaagac caaatacttt caaatgttga agatgcatgc gatgaatacc aacaatatca 300

ctgagctagt gaactatttg gcaaatgact taagtttaga tgaagcttca gtcttgataa 360

ctgaatattc aaagcactgc gggaaacctg tgcctccaga cactgctccc tgtgaaattc 420

tgaagatgtt tcttagtgga ttatcgtaaa tcactgaacc ttttttttcaa gaaggacaag 480

aattttggag tctgctatta atggaccata t 511

<210> 36
<211> 420
<212> DNA
<213> Homo sapiens

<400> 36
gctggcacag atgacgcggt gcccggagca ggagcagcgg ctgcagcgct tagaacggct 60

gcctgagctg gcccgcgtgc tgcggagcgt ctttgtgtcc gaacgcaagc ctgcgctcag 120

catggaggtg gcctgtgcca ggatggtggg cagctgttgt actatcatga gccctgggga 180

aatggagaag cacctgctgc tcctctccga gctgctgccg gactggctca gcctccaccg 240

catccgcacc gacacctacg tcaagctgga caaggccgcg gacctggccc acatcactgc 300

acgcctggcc caccagacac gtgctgagga ggggctgtga gcctgggggc cactgtggac 360

agacgtgggc ttcagaagct cgctggcctg ggcccaccag cattttcttt tatgaacatg 420

<210> 37
<211> 523
<212> DNA
<213> Homo sapiens

<400> 37
ttatgctgca tttggctgga gcccggtgtt cagtggtttc cctgccccgag gtcgctgcag 60

ccccatctac cacatcttca tgtggacatt gagattcaca tgctggctcc tgaagggtgc 120

tcagtctcct tggtgattaa ggtcctgctt gaactgctgc caactccatg tcagggaagt 180

cgcttttggt gcctggctgg tttgcccaga gccaagctgg ggcaaggggc agccagccct 240

ggcttccaag gctcccgtac tgtctgtgtc cttgtataag gagctttgct cttggaatta 300

ctgaaagtct gtggccctaa gagagagaca caagtggcct taagtctttt tgaagtgtta 360

tttcatccag ggaaatgcct cgagccatag agcctgaaat catctttgtt ggctcagaaa 420

ataccttagc ttcactcagc tggactgcat tgaaggcgag gctgcccctt ggatcaagca 480

gaaaacaaga gaaagaaaga acgttccctt tggggatagt ctg 523

<210> 38
<211> 446
<212> DNA
<213> Homo sapiens

<400> 38
agccacagtt cccctatgtg gaagtggggc gggcttcata gagacttggg aatgagctga 60

aggtgaaaca ttttctccct ggatttttac cagtctcaca tgattccagc catcacctta 120

gaccaccaag ccttgattgg tgttgccagt tgtcctcttc cgggaagatt tgcagttctt 180

tggctgaagg aagctgtgcg tgtgtgtgtg tgtatgttgt gggtatgtgt atctcacact 240

catgcattgt cctcttttta tttagattgg cagtgtaggg agttgtgggt agtggggaag 300

agggttagga gggtttcatt gtctgtgaag tgagaccttc cttttacttt tcttctattg 360

cctctgagag catagctaga ggcctgactg ccaagccatg ggtagcctgg gtgtaaaacc 420

tggagatggt ggatgatccc cacgcc 446

<210> 39
<211> 565
<212> DNA
<213> Homo sapiens

<400> 39
cattgcagat cgtagcgcgt tgcctgtcgc tttcccttgg atacctagac cgttataaag 60

tgtgccacat ggacttaccg agcatggaga gaggatttta gctaggattt gaacacttgg 120

tgctgggaac ctcagggtat tgcttgccac taagccatga aaccagagac aaaatctcta 180

tactgccctg agttgggggg aattctcagt gccaactgtg gctggtcctc attcaaaggg 240

acggtcagtt tggtgtcaac atgaaacacc aagatgtctg tctctgaagc gtgattttaa 300

aatccccatg cctgtgcgtg cgcttcctat ttctagggct gggaaacact ccttgcatca 360

aggggtcact tacagaacaa agaatctttt gggggaaact tcctctaaaa ccctctcata 420

tatagacagc tttgactgga gggtccattt ttcttccagg atggtgttac tgcagttgaa 480

gggcaatatg aagttacttt cttaatgtga cctagcaata ggcatagcta cgtggcacta 540

tattctggcc agactcgatg tgtac 565

<210> 40
<211> 562
<212> DNA
<213> Homo sapiens

<220>
<221> misc_feature
<222> (112)..(112)
<223> n is a, c, g, or t

<220>
<221> misc_feature
<222> (184)..(184)
<223> n is a, c, g, or t

<220>
<221> misc_feature
<222> (190)..(190)
<223> n is a, c, g, or t

<220>
<221> misc_feature
<222> (312)..(312)
<223> n is a, c, g, or t

<220>
<221> misc_feature
<222> (317)..(317)
<223> n is a, c, g, or t

<220>
<221> misc_feature
<222> (327)..(327)
<223> n is a, c, g, or t

<220>
<221> misc_feature
<222> (338)..(338)
<223> n is a, c, g, or t

<220>
<221> misc_feature
<222> (342)..(342)
<223> n is a, c, g, or t

<220>
<221> misc_feature
<222> (348)..(348)
<223> n is a, c, g, or t

<220>
<221> misc_feature
<222> (351)..(351)

<223> n is a, c, g, or t

<220>
<221> misc_feature
<222> (356)..(356)
<223> n is a, c, g, or t

<220>
<221> misc_feature
<222> (359)..(359)
<223> n is a, c, g, or t

<220>
<221> misc_feature
<222> (361)..(361)
<223> n is a, c, g, or t

<220>
<221> misc_feature
<222> (366)..(366)
<223> n is a, c, g, or t

<220>
<221> misc_feature
<222> (375)..(375)
<223> n is a, c, g, or t

<220>
<221> misc_feature
<222> (380)..(380)
<223> n is a, c, g, or t

<220>
<221> misc_feature
<222> (386)..(387)
<223> n is a, c, g, or t

<220>
<221> misc_feature
<222> (392)..(393)
<223> n is a, c, g, or t

<220>
<221> misc_feature
<222> (395)..(395)
<223> n is a, c, g, or t

<220>
<221> misc_feature
<222> (402)..(402)
<223> n is a, c, g, or t

<220>
<221> misc_feature
<222> (404)..(405)
<223> n is a, c, g, or t

<220>
<221> misc_feature
<222> (411)..(413)
<223> n is a, c, g, or t

<220>
<221> misc_feature
<222> (416)..(416)

<223> n is a, c, g, or t

<220>
<221> misc_feature
<222> (418)..(418)
<223> n is a, c, g, or t

<220>
<221> misc_feature
<222> (421)..(421)
<223> n is a, c, g, or t

<220>
<221> misc_feature
<222> (423)..(423)
<223> n is a, c, g, or t

<220>
<221> misc_feature
<222> (426)..(426)
<223> n is a, c, g, or t

<220>
<221> misc_feature
<222> (435)..(435)
<223> n is a, c, g, or t

<220>
<221> misc_feature
<222> (446)..(447)
<223> n is a, c, g, or t

<220>
<221> misc_feature
<222> (450)..(450)
<223> n is a, c, g, or t

<220>
<221> misc_feature
<222> (452)..(452)
<223> n is a, c, g, or t

<220>
<221> misc_feature
<222> (459)..(459)
<223> n is a, c, g, or t

<400> 40
acttcttatc cgaccagtac agaggttacc cagtgttgca ttacttttaa atgatcttaa 60

gaagcataca gctgatgaaa atccagacaa aagcacttta gaaaaagcta tnggatcact 120

gaaggaagta atgacgcata ttaatgagga taagagaaaa acagaagctc aaaagcaaat 180

tttngatgtn gtttatgaag tagatggatg cccagctaat cttttatctt ctcaccgaag 240

cttagtacag cgggttgaaa caatttctct aggtgagcac ccctgtgaca gaggagaaca 300

agtaactctc tncctcnttc aatgatngcc tagagatnag cnaagaanaa ncggcncang 360

nttatnggca ctttnaggan gtcctnnatg gnncnaaccc gnannccccca nnnttntntt 420

nanagncata ttcancctaa tgcctnnttn tnagattana gaaggtatcg gcaccataca 480

gagagacaga aagatgccat aatggctttg ccttgctttg tgaggccacc aaacagagca 540

ggcaatgtgc tactcagttt cc 562

<210> 41
<211> 512
<212> DNA
<213> Homo sapiens

<220>
<221> misc_feature
<222> (152)..(153)
<223> n is a, c, g, or t

<220>
<221> misc_feature
<222> (157)..(157)
<223> n is a, c, g, or t

<220>
<221> misc_feature
<222> (302)..(304)
<223> n is a, c, g, or t

<400> 41
tggagtgtca ttttatgcca cctgaaaaag tacataaaga actctttaaa gactggaata 60

ttccatttaa gcagccatca tatccatcag tgaaacggta taatcggaat cggacactaa 120

cacaaaagct acggatggag gagcgattta annaaangaa agaaagatta ctcaggaaga 180

aattagctaa aaaaggaatt gactatgatt ttccttcttt gattttacag aaaacggaaa 240

gtatttcaaa aactaatcgt cagacgtcta caaaaggcca ggttttacgt aagaagaaga 300

annnaaaagt ttcaggtact cttgacactc ctgagaagac tgtggatagc cagggcccca 360

caccagtttg tacaccaaca tttttggaga ggcgaaaatc tcaagtggct gaactgaatg 420

atgatgataa agatgatgaa atagttttca aacagcccat atcctgtgta aaagaagaaa 480

tacaagagac tcaaacacct acacattcac gg 512

<210> 42
<211> 233
<212> DNA
<213> Homo sapiens

<400> 42
gagtgtgtaa aactttccaa atggcagaat acctgaaact ggagtttatc aaggaaattg 60

gatacactca catgaaaata gcggaaggag tgaactctct tttgcagatg gcaggcctcc 120

tggcaaggct gtgtcagaag acaatggccc cggtggccag ttagagcaga gacttcactg 180

actgacttac aggtgcccta ttctgaggta caggagccgc ggctttctga tgg 233

<210> 43
<211> 557
<212> DNA
<213> Homo sapiens

<400> 43
gacacaatta cagctgattg cagccttgac cttcccagct caagtgatcc tcctacctca 60

gcctcccaag tagttaggac acacaggtgt gcacctcata tccagataat ttttttcaat 120

tttttttgt agaggtgggg ggtctcccta tgttgcccag gcagatctca gactcctggg 180

ctcaagcgat cctcacacct cagcgtccca gagtgctggg attacagttg tgagccactg 240

tgcctggcct tttttttttt ttaacctttt cgtttaactt ctctcttcac tgcatcccaa 300

tccatctaca ggcatgcaca cttattagga aaggaggttt gaggtaacaa cagagacttt 360

cactatattt tgctttgaca gaaggaaaga ggaggagttt ctattaaaat ctgtcacttg 420

agtgatgtca tttaagtcct attttaggag ataaaaacag ctttggggac tggttaaagt 480

cccccagaaa ctacaataaa gaacaacttt tgttttaact cttaatcact ttgtaatttt 540

gactcaatcc ttttctg 557

<210> 44
<211> 406
<212> DNA
<213> Homo sapiens

<400> 44
acaaaagtcc atttgtcctg ctactccttt ggtgacacac aggtagtgtc aactggaaca 60

tcagtaggta tttgatataa ccttttttgtt agcagaaaat tagtcactgc ttaggtttta 120

agtctgccaa actgggacta agtatgcttt aaataggtat agattcttcg gtttcttaga 180

attctagatc catgatgtac tttaattttt taatgactgt atatttagtt ttaaccacta 240

cttaaagtaa tctaagcaaa gaattcctat tgctctccat cagttcaaga taaaaaaaca 300

aaaacaaaac aaaacgaaac ctatgttaaa atttgacccg tcaaaattac tcagtattga 360

gctgcctaaa gccgttttcc agaaagagac ttgcctgact atagaa 406

<210> 45
<211> 530
<212> DNA
<213> Homo sapiens

<400> 45
tggatctttc tcatacccaa aacattggac agatgctgcg tactcatttc acacattctc 60

agttcattgt ggtgtcacta aaagaaggta tgttcaacaa tgcaaacgtt cttttcaaaa 120

ccaagtttgt ggatggtgtt tctacagtag ccagatttac tcaatgtcaa aatggaaaga 180

tttcaaagga agcaaaatcc aaggcaaaac cacccaaagg agcacatgtg gaagtttaaa 240

ctacaaagtt atttcttcat cttgacctgt ttttttaaat gtaaactttt aaggacttga 300

gataactaat ttgtttatat acaaaaatta atgttactgt gttacttaac ccatgttttc 360

tctttatata atcacttatc gcttacaaat gagcatatat tcctcatctc ttaactagtc 420

taattatggt ccaattattg tggttgtgat tttatgcata tccatcaaaa tgtttttttt 480

cttatgcggg tcttttatat attagggatc ctgagatacc cgattctata 530

<210> 46
<211> 524
<212> DNA
<213> Homo sapiens

<400> 46
gtgaaactgc cagcatactc atgcatgcaa cagcacattc tctggtgctt gtggatgaat 60

taggaagagg tactgcaaca tttgatggga cggcaatagc aaatgcagtt gttaaagaac 120

ttgctgagac tataaaatgt cgtacattat tttcaactca ctaccattca ttagtagaag 180

attattctca aaatgttgct gtgcgcctag gacatatggc atgcatggta gaaaatgaat 240

gtgaagaccc cagccaggag actattacgt tcctctataa attcattaag ggagcttgtc 300

ctaaaagcta tggctttaat gcagcaaggc ttgctaatct cccagaggaa gttattcaaa 360

agggacatag aaaagcaaga gaatttgaga agatgaatca gtcactacga ttatttcggg 420

aagtttgcct ggctagtgaa aggtcaactg tagatgctga agctgtccat aaattgctga 480

ctttgattaa ggaattatag actgactaca ttggaagctt tgag 524

<210> 47
<211> 395
<212> DNA
<213> Homo sapiens

<220>
<221> misc_feature
<222> (65)..(66)
<223> n is a, c, g, or t

<220>
<221> misc_feature
<222> (234)..(234)
<223> n is a, c, g, or t

<400> 47
gccctagaca accctaacga ggaggtggca gaagtgaaga tctcccactt cccggccgcg 60

gaccnnggct tcagtgtgtc gcagcgctgt ttcgtgttgc agcctaaaga gaaaattgtt 120

atttctgtta actggacacc actcaaagaa ggccgagtaa gagagattat gacatttctt 180

gtaaatgatg ttctgaaaca ccaagctata ttactaggaa atgcagaaga gcanaaaaag 240

aaaaagagga gtctttggga taccattaaa aagaagaaaa tttcagcctc tacaagtcac 300

aacagaaggg tttcaaatat tcagaatgtt aataaaacat ttagtgtttc ccaaaaagtt 360

gacagagtta ggagcccact acaagcttgt gaaaa 395

<210> 48
<211> 331
<212> DNA
<213> Homo sapiens

<400> 48
tcaccagaag atatcattgg caacatcttt cgagtgtgta aaactttcca aatggcagaa 60

tacctgaaac tggagtttat caaggaaatt ggatacactc acatgaaaat agcggaagga 120

gtgaactctc ttttgcagat ggcaggcctc ctggcaaggc tgtgtcagaa gacaatggcc 180

ccggtggcca gttagagcag agacttcact gactgactta caggtgccct attctgaggt 240

acaggagccg cggctttctg atgggggaaa atgcgcctta ggctgagcca acatgactgt 300

cccccaaact ccagtggctg gccaggcgcg g 331

<210> 49
<211> 334
<212> DNA
<213> Homo sapiens

<220>
<221> misc_feature
<222> (40)..(42)
<223> n is a, c, g, or t

<220>
<221> misc_feature
<222> (44)..(55)
<223> n is a, c, g, or t

<220>
<221> misc_feature
<222> (57)..(59)
<223> n is a, c, g, or t

<220>
<221> misc_feature
<222> (184)..(184)
<223> n is a, c, g, or t

<400> 49
tgatcgagca ggcggcgcag tacaccaacg cggtgcgcgn nngnnnnnnn nnnnncnnng 60

tatcctaaga aatccggtaa ccaataagaa gcattacaga ttgtatgtga tttataaagt 120

tccgcaagtc agagtactgg atttccagaa agtgaaacta aaattttaat ccaggtgctg 180

gttngccaac tgacaaaaag aaaggtgggc catctccagg ggatgtagaa gcaatcaaga 240

atgccatagc aaatgcttca actctggctg aagtggagag gctgaagggg ttgctgcagt 300

ctggtcagat ccctggcaga gaacgcagat cagg 334

<210> 50
<211> 496
<212> DNA
<213> Homo sapiens

<220>
<221> misc_feature
<222> (458)..(458)
<223> n is a, c, g, or t

<400> 50
ttgaacctcc cattagccaa gtgggaaatg tagacactgc ttcagaactt gagaaggggg 60

tgtctgaggc tgcagtccta aagccttctg aagagttacc tgctgaggct acctcatccg 120

ttgaacccga aaaggattct ggctcagcag cagaggctcc tcgttagaga ctggaatttg 180

tgaaaatgtg acagtgacac ttcctggagt gtagagcttg aggtgtacag atgctgtatt 240

atatccgctc ccgctgtact gcagccccgc gccagctggt ggggaactgt aagcaatttg 300

attgcttccc ttctatttaa aaatagccac aaaataacaa aaaatactga aaatatgaat 360

aaatattacc ctttttgctg taacttttta aaagttttga ctttaaaaag tttacaaatc 420

gtaattagaa gtgctctcta tttttttttt tttttttnaa tttaagacaa ggtaacggtg 480

aaagctcctc aaaaca 496

<210> 51
<211> 356
<212> DNA
<213> Homo sapiens

<400> 51
ctctcttcaa cggtgacact cagtatgtct gcagatgtac cccttgttgt agagtataaa 60

attgcggata tgggacactt aaaatactac ttggctccca agatcgagga tgaagaagga 120

tcttaggcat tcttaaaatt caagaaaata aaactaagct ctttgagaac tgcttctaag 180

atgccagcat atactgaagt cttttctgtc accaaatttg tacctctaag tacatatgta 240

gatattgttt tctgtaaata acctattttt tttctctatt ctctccaatt tgtttaaaga 300

ataaagtcca aagtctgatc tggtctagtt aacctagaag tatttttgtc tcttag 356

<210> 52
<211> 360
<212> DNA
<213> Homo sapiens

<400> 52
gcagaacata cagctacctg ctgcactgct ctcccggttt gacctcctct ggctgattca 60

ggaccggccc gaccgagaca atgacctacg gttggcccag cacattacct atgtgcacca 120

gcacagccgg cagccccccct cccagtttga acctctggac atgaagctca tgaggcgtta 180

catagccatg tgccgcgaga agcagcccat ggtgccagag tctctggctg actacatcac 240

agcagcatac gtggagatga ggcgagaggc ttgggctagt aaggatgcca cctatacttc 300

tgcccggacc ctgctggcta ttctgcgcct tttcactgct ctggcacgtc tgagaatggt 360

<210> 53
<211> 541
<212> DNA
<213> Homo sapiens

<220>

<221> misc_feature
<222> (136)..(136)
<223> n is a, c, g, or t

<220>
<221> misc_feature
<222> (141)..(141)
<223> n is a, c, g, or t

<220>
<221> misc_feature
<222> (147)..(147)
<223> n is a, c, g, or t

<220>
<221> misc_feature
<222> (159)..(159)
<223> n is a, c, g, or t

<220>
<221> misc_feature
<222> (161)..(161)
<223> n is a, c, g, or t

<220>
<221> misc_feature
<222> (167)..(167)
<223> n is a, c, g, or t

<220>
<221> misc_feature
<222> (172)..(172)
<223> n is a, c, g, or t

<220>
<221> misc_feature
<222> (181)..(181)
<223> n is a, c, g, or t

<220>
<221> misc_feature
<222> (188)..(188)
<223> n is a, c, g, or t

<220>
<221> misc_feature
<222> (191)..(191)
<223> n is a, c, g, or t

<220>
<221> misc_feature
<222> (195)..(195)
<223> n is a, c, g, or t

<220>
<221> misc_feature
<222> (220)..(220)
<223> n is a, c, g, or t

<220>
<221> misc_feature
<222> (260)..(260)
<223> n is a, c, g, or t

<220>

<221> misc_feature
<222> (509)..(509)
<223> n is a, c, g, or t

<400> 53
gagttaatgc agcactcgtc attcagaaat attggcgaag agtcttagca cagagaaaat 60

tattaatgtt aaaaaaggaa aagctggaaa aagttcaaaa taaagcagca tcacttattc 120

agggatattg gagaanatat nccactngac aaagatttnc ngaaatngaa anattattca 180

ntcatccngc naatntagga taagaatgat aattgctgtn acatcttata aacgatatct 240

ttgggctaca gttacaattn cagaggcatt ggcgtgctta tttaagaaga aaacaagatc 300

aacaaagata tgaaatgcta aaatcatcaa ctcttataat ccaatctatg ttcagaaaat 360

ggaagcaacg taaaatgcaa tcacaagtaa aagctacagt aatattgcaa agagctttta 420

gagaatggca tttaagaaaa caagctaaag aagaaaattc tgctattatc atacaatcat 480

ggtatagaat gcataaagaa ttacggaant atatttatat tagatcttgt gttgttatca 540

t 541

<210> 54
<211> 483
<212> DNA
<213> Homo sapiens

<400> 54
ggacagtgtt tcaacaagcc taggctatct cgtaagttga aaaatatccc actatagttg 60

cttcatgagt atgaagtaag atggcctctg atttacactg gttcaattta caaattttca 120

actttatgat aggtttatcc gggtactaaa tgcatttcaa cttgatagtt tcaacttatg 180

ataggtttac caggatgtag tcccactgtt gaggagcatc tatttagggg ttaattactt 240

tagtaataag tggaaagtaa gataccttga gtaatgtttg cctataaaat tgtcagcgta 300

tttttacact attggctcaa gaatgttata atgctaaggg acataagttg gcaaccactt 360

ggtttttgga aggactttcg gtattgtatt agaagtctgc cctagctgtt aaatttctgg 420

gtatttatcc taaggaatta attaaagagt taattgttcc tttcttcagt gggccattgt 480

ttt 483

<210> 55
<211> 531
<212> DNA
<213> Homo sapiens

<400> 55
gaagtcacag gccctgtcat tgcgcctctc ttcccgcaga aacgtgaaga gggctggtgg 60

gtggtgattg gagatgccaa gtccaatagc ctcatctcca tcaagaggct gaccttgcag 120

cagaaggcca aggtgaagtt ggactttgtg gccccagcca ctggtgccca caactacact 180

ctgtacttca tgagtgacgc ttacatggga tgtgaccagg agtacaaatt cagcgtggat 240

gtgaaagaag ctgagacaga cagtgattca gattgagtcc tgaggcattt acttttgggt 300

aaaggagagt tgagcctgaa ttaggaatgt gtacattgta ggaatcctgg ttgtggggac 360

caggtctgtg ggcctcaggt ctggccagcc agggctggtg ctgtccccgc ctacctccac 420

ttcctttccc ttgctcactc tggatccagt gacagcaggt gtcatgggtc aagcataaat 480

catatatagc attttcaggc atgttcctgg tagttctttt gagtctgaca t 531

<210> 56
<211> 141
<212> DNA
<213> Homo sapiens

<400> 56
ataagatccc ttttgctgat gctctggatt tgtttcgagg aaggaaagtc tatttggaag 60

atggctttgc ttacgtacca cttaaggaca ttgtggcaat catcctgaat gaatttagag 120

ccaaactgtc caaggctttg g 141

<210> 57
<211> 432
<212> DNA
<213> Homo sapiens

<400> 57
ttggacccaa gccctgaggt acagaagaag aagtatgcct tcaaatgtca cagactaaaa 60

gaaaataata ttgagcagat ttacccagtc aatgccattt cttttcacaa tatccacaat 120

acatttgcca caggtggttc tgatggcttt gtaaatattt gggatccatt taacaaaaag 180

cgactgtgcc aattccatcg gtaccccacg agcatcgcat cacttgcctt cagtaatgat 240

gggactacgc ttgcaatagc gtcatcatat atgtatgaaa tggatgacac agaacatcct 300

gaagatggta tcttcattcg ccaagtgaca gatgcagaaa caaaacccaa gtcaccatgt 360

acttgacaag atttcattta cttaagtgcc atgttgatga taataaaaca attcgtactc 420

cccaatggtg ga 432

<210> 58
<211> 381
<212> DNA
<213> Homo sapiens

<400> 58
gacagccaga atgggacctc tactcacggt tttctgaatg gaaatcagga acaaactgct 60

catcactgtc tggtacctga gctgcagaga gaagtcagag ccccaccagc gacaccctca 120

ggccccacca aaacgcactg aagaatccaa ccaggaaacc tcctgaacac accacttccc 180

catcagaccc agccacggac ccagccacgg accccacccc agccagctca cagctaactc 240

cccagcacac aggaaggggg gaatttgcct ggaggcagag gtattcaagc gggatggaag 300

agggaggaac acttgctatt agaattacaa tcaaggctat ttggggaggg agccctgggc 360

tctctgtgcc aaaggactgc c 381

<210> 59
<211> 491
<212> DNA
<213> Homo sapiens

<400> 59
tagctgtttc agagagagta cggtatattt atggtaattt tatccactag caaatcttga 60

tttagtttga tagtgtgtgg aattttattt tgaaggataa gaccatggga aaattgtggt 120

aaagactgtt tgtacccttc atgaaataat tctgaagttg ccatcagttt tactaatctt 180

ctgtgaaatg catagatatg cgcatgttca actttttatt gtggtcttat aattaaatgt 240

aaaattgaaa attcatttgc tgtttcaaag tgtgatatct ttcacaatag ccttttttata 300

gtcagtaatt cagaataatc aagttcatat ggataaatgc atttttattt cctatttctt 360

tagggagtgc tacaaatgtt tgtcacttaa atttcaagtt tctgttttaa tagttaactg 420

actatagatt gttttctatg ccatgtatgt gccacttctg agagtagtaa atgactcttt 480

gctacatttt a 491

<210> 60
<211> 349
<212> DNA
<213> Homo sapiens

<220>
<221> misc_feature
<222> (31)..(32)
<223> n is a, c, g, or t

<220>
<221> misc_feature
<222> (44)..(44)
<223> n is a, c, g, or t

<220>
<221> misc_feature
<222> (73)..(73)
<223> n is a, c, g, or t

<220>
<221> misc_feature
<222> (83)..(83)
<223> n is a, c, g, or t

<400> 60
ccaagcaccg catctacgag tatgtggagt nnccccggatg tccnttcatc gcacccaacc 60

tgtccatcat tantcggggc atnccacggc cgccaagatc atgggtgtgg ccggcggcct 120

gaccaacctc tccaagatgc ccgcctgcaa catcatgctg ctcggggccc agcgcaagac 180

gctgtcgggc ttctcgtcta cctcagtgct gccccacacc ggctacatct accacagtga 240

catcgtgcag tccctgccac cggatctgcg gcggaaagcg gcccggctgg tggccgccaa 300

gtgcacactg gcagcccgtg tggacagttt ccacgagagc acagaaggg 349

<210> 61
<211> 396
<212> DNA
<213> Homo sapiens

<400> 61
gactggactt attactccca aagcaggctt tgactctgat tatgaccaag ctcttgctga 60

cataagagaa aatgaacaga gcctcctgga atacctagag aaacagcgca acagaattgg 120

ctgtaggacc atagtctatt gggggattgg taggaaccgt taccagctgg aaattcctga 180

gaatttcacc actcgcaatt tgccagaaga atacgagttg aaatctacca agaagggctg 240

taaacgatac tggaccaaaa ctattgaaaa gaagttggct aatctcataa atgctgaaga 300

acggagggat gtatcattga aggactgcat gcggcgactg ttctataact ttgataaaaa 360

ttacaaggac tggcagtctg ctgtagagtg tatcgc 396

<210> 62
<211> 524
<212> DNA
<213> Homo sapiens

<400> 62
gagacttttt tgaactcaga cttaaatatt atggattaag aaaagaatgg ctcctaggaa 60

tgcttggtgc tgaatctgct aaactgaata atcaggctcg ctttatctta gagaaaatag 120

atggcaaaat aatcattgaa aataagccta agaaagaatt aattaaagtt ctgattcaga 180

ggggatatga ttcggatcct gtgaaggcct ggaaagaagc ccagcaaaag gttccagatg 240

aagaagaaaa tgaagagagt gacaacgaaa aggaaactga aaagagtgac tccgtaacag 300

attctggacc aaccttcaac tatcttcttg atatgcccct ttggtattta accaaggaaa 360

agaaagatga actctgcagg ctaagaaatg aaaaagaaca agagctggac acattaaaaa 420

gaaagagtcc atcagatttg tggaaagaag acttggctac atttattgaa gaattggagg 480

ctgttgaagc caaggaaaaa caagatgaac aagtcggact tcct 524

<210> 63
<211> 445
<212> DNA
<213> Homo sapiens

<400> 63
cactctcagt ttacttggct ggagtttatc tgttgtactt tttacccaca acttcaatac 60

tgtgatcaag ggcgatgtga cttagatctt ttacatcttt aagcaggctt actcttgcct 120

gcttaagtta tgaataaata catcacatgc ttgttacagt agaccatctt ttaaattaac 180

aaattgttgt atttgaagtg gtattgccaa atataattaa cacttgaatc ccctaactgg 240

aagttaatat cttgagagta ttacagtgtt ctagtcctgt gagcccatag tatttttcaa 300

aggtatatgc atatgcctgt ttgctttcat aatctgagtt gttttaggaa agaacctatt 360

gattacctct gagatgtagg actgtgagac attggtttgt aatggaggac ttttactttt 420

cacctatgct attcagtact gttgg 445

<210> 64
<211> 487
<212> DNA
<213> Homo sapiens

<400> 64
gtgaagcccg tggactacag agagtatggc cgaaggctgg tcatgagcat caggagaagt 60

gcattgatgt gagaggagca gtgccaatcg ggcagaagtt tgcaaatagg cagaatggaa 120

tcgatttcct cccacctccg tgtgacgatc ccatgttagc tacacagtgc agaggctctt 180

gatggtggac taagcaattc ctccctcgtg cgcatctcag aacccatcgg taggcaaagg 240

aaaatacgct caggtggttg tggtgtagac tgtgtcaggc ctacggagtc agccagtggc 300

tagcgcaaga ccagtcactc cctctgcctt caggcttctg tcaatttcat tatcatcaag 360

caggaattat gtcgtaagtc actgacccta actgcagacc atgaagtaaa ttatgtaact 420

aggtttttgc ttctccagtg gtgaccaccc ccccccatcc ccgctcacaa cttgggttct 480

tctcagc 487

<210> 65
<211> 361
<212> DNA
<213> Homo sapiens

<400> 65
acccaacttg attcagcgac ccaaggcgga tgatacaagt gcagccacca ttgagaagaa 60

agccacagca accatcagtg ccaagccaca gatcactaat cccaaggcag agattactcg 120

atttgtgccc actgcactga gagtacgtcg ggagaataaa ggggctactg ctgctcccca 180

aagaaagtca gaggatgatt ctgctgtgcc tcttgccaaa gcagcaccca aatctggtcc 240

ttctgttcct gtctcagtac aaactaagga tgatgtctat gaggctttca tgaaagagat 300

ggaagggcta ctgtgacagc ttttgatgcc agaaaaggct tctgttcaca acagtggccc 360

a 361

<210> 66
<211> 172
<212> DNA
<213> Homo sapiens

<400> 66
tgagctcctt gaagtccatg gcggaacggg cagccatcag ctctggcatt gaggaccctg 60

tgccaacgct gcacctgacc gagcgagaca tcatcctgag cagtacatca gcacctccgg 120

cctcagccca gccgcccctg cagctgtcag aggtgaacat accgctgtcg ct 172

<210> 67
<211> 412
<212> DNA
<213> Homo sapiens

<400> 67
tagccttgtt cagaatttac tgcacataaa aaagggtatt tcatccagaa tagatcagtt 60

attgaagcag tgctgctaac atccattccc tttcatacca ccattttcac cctgtttctt 120

cccctcctcc agttctttgg aaatttgtga tcgggggatc ttagttgctt atttgttttg 180

actcttgtgt gctgtgggca ctggagtaga gatttctgga gaaaaaaaaa cagtttattt 240

catcttgcct tttgtgtttg agttattttt aatattttcc tgtaaatatt ttgtaatatt 300

ttacttgtaa tgaaatggat cacaatgtca tttcctaata caaggcagga tatgtgggaa 360

gaatatgtac aattatttga ttaaaattat ttcccactga cctaaacttt ca 412

<210> 68
<211> 550
<212> DNA
<213> Homo sapiens

<400> 68
gtttattgta atgtcatcgt cctccagcgc cccatccacc cagcaggtcc tgtccctcag 60

cacctcggcc cccggctcag gttccaccac cacttcgccc gtcaccacca ccgtccccag 120

cgtgcagccc atcgtcaagt tggtctccac cgccaccacc gcacccccca gcactgctcc 180

ctctggtcct gggagtgtcc agaagtacat cgtggtctca cttcccccaa caggggaggg 240

caaaggaggc cccacctccc atccttctcc agttcctccc ccggcatcgt ccccgtcccc 300

actcagcggc agtccccttt gtggggggaa gcaggaggct ggggacagtc cccctccagc 360

tccagggact ccaaaagcca atggctccca gcccaactcc ggctcccctc agcctgctcc 420

gtgatgctcc acctgccagc cccggattc ccacacatgc agacatgtac acacgtgcac 480

gtacacacat gcatgctcgc taagcggaag gaagttgtag attgcttcct tcatgtcact 540

ttctttttag 550

<210> 69
<211> 520
<212> DNA
<213> Homo sapiens

<400> 69
aagccaggat tctccggtct ggaatttctg agtgagtcct ttttttatgg tgtcctccct 60

ctgtgaatgt acaggcggaa ctgtacgaac agctcccttc catccatttt taactctttc 120

ggaaataaca cctcacagca gcttcgtgct tttgtacaga cctttgtaac aagtgtacag 180

aaaactcatt ttgtttgaga aacaggagtt gatgaaccca tcatgctggt tttctctga 240

gcacaaagtt ttaggctgta cacagccagc cttgggaatc tcgttgagcg ttcggcgtgg 300

atccacgggg ccaggccacc ctgcgggagg ccacacgcat ccacttcgga ttcagtgggt 360

gaagacagaa ctctgagagt ctgcaggcgg ctcctgtgct ttttatttct ggctcttcgg 420

atgtcttcta gacatttact atcactgcac ctgaagaaaa aatcactttt accttcctaa 480

tttaaaaaga caaaacagaa atgtacgttc cttcgctagc 520

<210> 70
<211> 278
<212> DNA
<213> Homo sapiens

<400> 70
agctgatttc tatgctgcct acattaacat tcttcttgga gttttctaca ctgtttgtcg 60

agatttgaaa gagctcagac atctggcagt acttaatttt cctaaatatt gtgaacccgt 120

ggttaaagga gaagcaagtg aacgtgatac tcgcaaactg tggagaaata ttgaacctca 180

tttgaagaaa gctatgcaga ctgtttatct cagggaaata tcaagttccc agtgggaaaa 240

gctacagaaa gatgacacag atccggggca actgaaag 278

<210> 71
<211> 446
<212> DNA
<213> Homo sapiens

<400> 71
gcacagcttg caaaggatat tgccaggaga agcaaaactt ttaatccagg tgctggtttg 60

ccaactgaca aaaagagagg tgggccatct ccaggggatg tagaagcaat caagaatgcc 120

atagcaaatg cttcaactct ggctgaagtg gagaggctga aggggttgct gcagtctggt 180

cagatccctg gcagagaacg cagatcaggg cccactgatg atggtgaaga agagatggaa 240

gaagacacag tcacaaacgg gtcctgagca gtgaggcaga tgtataataa taggccctct 300

tggaacaagt cttgcttttc gaacatggta taatagcctt gtttgtgtta gcaaagtgga 360

atctatcagc attgttgaaa tgcttaagac tgctgctgat aattttgtaa tataagtttt 420

gaaatctaaa tgtcaatttt ctacaa 446

<210> 72
<211> 282
<212> DNA
<213> Homo sapiens

<400> 72
ccgtggccag ggtcagaaag tgcagaaggt tatggtgcag cccatcaacc tcatcttcag 60

atacttacaa aatagatcgc ggattcaggt gtggctctat gagcaagtga atatgcggat 120

agaaggctgt atcattggtt ttgatgagta tatgaacctt gtattagatg atgcagaaga 180

gattcattct aaaacaaagt caagaaaaca actgggtcgg atcatgctaa aaggagataa 240

tattactctg ctacaaagtg tctccaacta gaaatgatca at 282

<210> 73
<211> 514
<212> DNA
<213> Homo sapiens

<400> 73
caatgccacg gactactgga cgttccggaa gatgttcacc atccagctgg ctctgatagg 60

cttcgcggaa ttcgtcctgc atttaaatag actcaacccc gagatgttac agatcgctca 120

ggacactggc aaactgaatg ttgcctactt tcgatttgac ataaacgacg cgactggaga 180

cctggatgcc aaccgtcctg tcccatttcg actcacgccc aacatttctg agtttctgac 240

caccatcggg gtctccggcc cgttgacagc gtccatgatt gcggtcgccc ggtgcttcgc 300

ccagccaaac tttaaggtgg atggcattct gaaaacggtt ctccgggacg agatcattgc 360

ttggcacaaa aaaacacaag aggacacgtc ctctcctctc tcggccgccg ggcagccaga 420

gaacatggac agccagcaac tggtgtccct ggttcagaaa gccgtcaccg ccatcatgac 480

ccgcctgcac aacctcgccc agttcgaagg cggg 514

<210> 74
<211> 414
<212> DNA
<213> Homo sapiens

<400> 74
atagcttctg tataggccta acctttattc atatggcatc tcagaagtat gtgttacgga 60

gacatgctct tattgtacag ggcttttcct ttcttaatcg atacctcagt ttacgtgggc 120

cctgccagga atcattctac aatttgggcc gtggccttca tcagttgggg ctgattcatc 180

ttgcaatcca ctattatcag aaggccctgg agctccctcc acttgtggta gagggtatag 240

aacttgacca gttagactta cgaagagata ttgcctacaa cttgtctctc atctatcaga 300

gcagtgggaa taccggaatg gctcaaacgc ttttgtatac ctattgttct atataaagca 360

ccgcaactga gaacagagca atggcagctg ctgtgtgagg accagtgtct tctg 414

<210> 75
<211> 512
<212> DNA
<213> Homo sapiens

<400> 75
agcaggatgt agagcgccac ttctctctgg gcgagttgaa ggagctgttt atcctggatg 60

aagctagcct cagtgacaca catgacaggt tgcactgccg acgttgtgtc aacagccgtc 120

agatccggcc accccctgat ggttctgact gcacttcaga cctggcaggg tggaaccact 180

gcactgataa gtgggggctc cgggatgagg tactccaggc tgcctgggat gctgcctcca 240

ctgctatcac cttcgtcttc caccagcatt ctcatgagga acagcggggc ctccgctgat 300

aaccagctgg tctgggtgta gctcttagag gaaggagata gggaaaaggg gctccttgct 360

ccacagggcc ctgttgaatt ttgttctctg ggagaaaatc atcaagaagg gctgcatgat 420

gtttgcccaa aatttatttt ataagaaaaa cttttttggt taaaaaaaag aataaaggta 480

tgaaagggct ggtgacagtc agggatgccc cc 512

<210> 76
<211> 443
<212> DNA
<213> Homo sapiens

<400> 76
gtgttggagt ctcagttctc ccagctgctt catcagatca attctacccg agactttgaa 60

agcatccgat tggctcatga ccacttcctg agcaatttgc tggctcaatc ctttatccta 120

ttgaaacctg tgtttcactg cctgaatgaa atcctagatc tctgtcacag tttttgttcg 180

ctggtcagtc agaacctagg cccactggat gagcgtggag ccgcccagct gagcattctc 240

gtgaagggct ttagccgcca gtcttcactc ctgttcaaga ttctctccag tgttcggaat 300

catcagatca actcagattt ggctcaacta ctgttacgac tagattataa caaatactat 360

acccaggctg gtggaactct gggcagtttc gggatgtgaa aatttctggc tcataaattg 420

aaataacagc cacgttccca agg 443

<210> 77
<211> 511
<212> DNA
<213> Homo sapiens

<400> 77
tggagacccc tgaactcatt gagctgagga agaagaagat tgaggaggcg atggacggaa 60

gtgagacacc tcagctcttc actgtgttgc cagagaagag aacagccact gttggagggg 120

ccatgatggg atcaacccac atttatgaca tgtccacggt tatgagccgg aagggcccgg 180

ctcctgagct gcaaggtgtg gaagtggcgc tggcgcctga agagttggag ctggatccta 240

tggccatgac ccagaagtat gaggagcatg tgcgggagca gcaggctcaa gtagagaagg 300

aggacttcag tgacatggtg gctgagcacg ctgccaaaca gaagcaaaaa aaacggaaag 360

ctcagcccca ggacagccgt gggggcagca agaaatataa ggagttcaag ttttaggtcc 420

cctcacacta gcccttttttt tggccctacg tctggatgcc tgggcttcac acaagaacca 480

cctctcccgc agttcccaag gacttgtcat t 511

<210> 78
<211> 494
<212> DNA
<213> Homo sapiens

<400> 78
caaggcatct gttgctttgg gtcctccacg actcttaggc ccgcctcaac aacccaggca 60

cctcctaggt aggctcaaag gtagacccgt ttccaccgca gcaggtgaac atgaccgtgt 120

tttcaactgt gtccacagtt cagatccctt tccagattgc aacctggcct gcatcccagc 180

tccttcctgc tcgtgtctta acctaagtgc tttcttgttt gaaacgccta caaacctcca 240

tgtggtagct cctttggcaa atgtcctgct gtggcgtttt atgtgttgct tggagtctgt 300

ggggtcgtac tccctcccct cccgtcccca gggcagattt gattgaatgt ttgctgaagt 360

tttgtctctt ggtccacagt atttggaaag gtcactgaaa atgggtcttt cagtcttggc 420

atttcattta ggatctccat gagaaatggg cttcttgagc cctgaaaatg tatattgtgt 480

gtctcatctg tgaa 494

<210> 79
<211> 536
<212> DNA
<213> Homo sapiens

<400> 79
gcacagtgtc tctagacttc atcagagcta ttgcaaggac ggtgaacttt gacataataa 60

aatacttgta tgatttcttg tgaaaacaag cttcaaagcc atatggacac tgtgacaatg 120

actaagccaa gctgtgttca tccagctact tagctggcca aggagaggag ttctttggct 180

ctattggatt tgtccaaaca ggtgctggcc cagcatggaa tctgatgaaa atattctgat 240

tggtctgggt ggatgtgagc agaagactat ttaccaggga ccctggagta tttggaagca 300

acgtgttaat tataaacagc agggtttgag cacaatctgt tctactctta atgatgttat 360

cttaacactg aaattgcctg aaacccattt acttaggact acattttgct ctgtgaacta 420

tcccctgcgc tttgaacgtg ccagcagccc ttgtttatat gcccattctt ttcacttcct 480

ctccacagga gcctctgcag tcgcttgcca aagcagattt tcctaaggcc actgtt 536

<210> 80
<211> 173
<212> DNA
<213> Homo sapiens

<220>
<221> misc_feature
<222> (34)..(39)
<223> n is a, c, g, or t

<400> 80
gtggctcagc tgtttcagac aactcaaggc caannnnnnc agcagatcct tcagactttt 60

caacagcctc caaaaccaca gtctcctgcc cttgacaatg ctgtgatggc tcaggttcag 120

gctatcacag ctcagttaaa gacaactcct acacaaccat ctgaacaaaa agc 173

<210> 81
<211> 508
<212> DNA
<213> Homo sapiens

<220>
<221> misc_feature
<222> (64)..(64)
<223> n is a, c, g, or t

<220>
<221> misc_feature
<222> (79)..(80)
<223> n is a, c, g, or t

<220>
<221> misc_feature
<222> (87)..(87)
<223> n is a, c, g, or t

<220>
<221> misc_feature
<222> (94)..(94)
<223> n is a, c, g, or t

<220>
<221> misc_feature
<222> (112)..(112)
<223> n is a, c, g, or t

<400> 81
agtggtaatt ttctacctcc atcatggcgg aagctcatga gtgatcctga ttctagtata 60

attngacttc tatcctgann agatttngct attngatttg aatgggaaga anatatgcat 120

ggcaaaggtg ttgctctctt gccattcgtg gatggagcga agggctacga gctgccctag 180

aacgagggta tacccagacc tcactctccc aaggaaagga ggacccaagg aagaaaacaa 240

ggcctttggg agggtgatgt ctatattatg tggggaaaca tcacccactc catgacttca 300

ttttagagct gtaccagaca ggttccacag agccagtgga ggtacccccct gaactatgtc 360

atgggattca aggaaagtct tctttggatg aagaagccat tcttccagat caaatagtat 420

gtgctcctgt tcctatgtta aagggatctg acacagaaca ctgtagtcag tattaatttt 480

aacagaccca cagtttgctg aagattac 508

<210> 82
<211> 497
<212> DNA
<213> Homo sapiens

<220>
<221> misc_feature
<222> (109)..(109)
<223> n is a, c, g, or t

<220>
<221> misc_feature
<222> (173)..(173)
<223> n is a, c, g, or t

<220>
<221> misc_feature
<222> (220)..(222)
<223> n is a, c, g, or t

<220>
<221> misc_feature
<222> (267)..(267)
<223> n is a, c, g, or t

<400> 82
tgaggcaggt gatcgacttc cagtggaagg tctgagacca ccactccttg tttttatcat 60

tagagaaact ttaaaaatca gtttttgatg tttgtgtgtt ggctgtgcnt gatatttgct 120

gccctgccat cttccctacc ccctctgata gacatgggtc cagagggggt ggncgtgcca 180

gccttgggtt agcctcctcc aggcaccagt ggacacggan nnggctttct gctctttgct 240

gtctccagac agtagaggtg agccacnttc caagttagcg tgattgtcac caggcccatc 300

atgctgctcg agcttctctg gctgcgtcct cactttcaca tccctagctg aggaaacagc 360

gagtcgcatc acgtcatgga aacatgaaaa attaagctga aaggtagaaa atgggccgta 420

aattgctctt agcgggtctg tttggttaat tgaggagtgc ctgttatctt gggcttagat 480

ccgctctgag ttcagtt 497

<210> 83
<211> 343
<212> DNA
<213> Homo sapiens

<400> 83
tggtggtcca ataactctac aatcagcctt gtagaggtca ttaaagacag aatcctgaaa 60

gtccgtgact acaaatacat tttcaaattc cggagaatcc aaaccttcaa attcttccac 120

tgactccatc tttacaaagc ccactttaat gtcctttaag gcttttataa gttcttcttg 180

ttttccagct tcttgaacca atatcactct tgtttcaatc tgaggcatct cttcttctac 240

atatgaagta gatccaataa gtaagttttc cttggaaatc tcagtaactt tagaatcaaa 300

aatggaagag tctgccaagc tagtcctccc agtagtggat gtt 343

<210> 84
<211> 497
<212> DNA
<213> Homo sapiens

<400> 84
tggctttaac cgtgaccgga ggcctgtgca cctggatcag gcagccttca ggactttggg 60

ccatgtgatg ccaagaggct caggaactgg catttacagc aatgctgcac caccacctgt 120

gacttaccag ggaaacttat acaggccgct tttgagagga caagcccaga ttccaaaact 180

tatgtcaaat atgaggcccc aggattcctg gcgaggtcct cctccccttt tccagcagca 240

aaggtttgac agaggcgttg ggctgaacc tctgctccca tggaaccgga tgctgcaaac 300

ccagaatgca gccttccagc caaaccagta ccagatgcta gctgggcctg gtgggtatcc 360

acccagacga gatgatcgtg gagggagaca gggatatccc agagaaggaa ggaaataccc 420

tttgccacca ccctcaggaa gatacaattg gaattaagct tttgtaaagc tttcccaaat 480

cctttcatca ttctaca 497

<210> 85
<211> 209
<212> DNA
<213> Homo sapiens

<400> 85
ccgagactta gtctcgggcc gccatggcca gcgtccacga gagcctctac ttcaatccca 60

tgatgaccaa tggggttgtg cacgccaatg tgttcggcat caaggactgg gtgacgccgt 120

acaagatcgc ggtgctggtg ctgctgaacg agatgagccg cacaggcgag ggcgccgtca 180

gcctcatgga gcggcggagg ctcaaccag 209

<210> 86
<211> 539
<212> DNA
<213> Homo sapiens

<400> 86
tctaccattc agctgccatt cataatcatc aatacaagca gaaaaacagt catagattgc 60

agcatctcca gtgacaagtt tgagtatctt ttcaattttg acaacacctt tgagatccat 120

gatgacatag aagtactaaa gcggatggga atgtcgtttg gcctggagtc aggcaaatgc 180

tctctggagg atctgaaact tgcgaaatcc ctggtgccaa aggctttaga aggttatatc 240

acagatatct ccacaggacc ttcttggtta aatcagggac tacttctgaa ctctacccaa 300

tcagtttcaa atttagacct gaccactggt gccaccttac cccagtcaag tgtaaaccaa 360

gggttatgct tggatgcaga agtggcctta gcaactgggc agttcctggc cccaaacagt 420

caccagtcca gcagtgcggc ctctcactgc tccgagtccc gaggcgagac cccctgttcg 480

ttcaatgatg aagatgagga agatgatgag gaggattcct cctccccaga ataaagaca 539

<210> 87
<211> 373
<212> DNA
<213> Homo sapiens

<400> 87
aacataaagt gtctgagccc tcgagatcct catccctaag tctgagtaaa atggactttg 60

atgatgaaag aacttggact gaccttgaag agaatttgtg taaccatgat gttgttcttg 120

ggaatgaatc cacttatggg acgccgcaga catgctaccc taataatgaa ataggtatcc 180

tggacaaaac aataaaaagg aagattgcac cagtcaagag gggagaagac ttgagcaagt 240

ccaggaggag cagaagtcct cctacatcgg agctgatgat gaaattcttt ccttctttga 300

aaccaaaacc aaagtcagat tcacacttgg gaaatgaact caagttaaac ataagtcaag 360

accaaccacc tgg 373

<210> 88
<211> 419
<212> DNA
<213> Homo sapiens

<400> 88
aaggctttgg caatatggag tcttgccatg ttgcccaggc tggtcttgaa ctcctagcct 60

caagtgatcc acccacctca gcctccgaaa gtgctgggat tataggcact aggcggcatg 120

cctgggccct ctactctgtt ctgaataaaa agtctctggg gatggaccct aggtgattca 180

taggcacact gaaatctgag aaccaggtac aggaaataaa attatttgaa atgcaaacca 240

ttgaatttta tgactttttc attgcttttg gtaagaggct ggactgtgga ggagatgaaa 300

acttgaccaa aaatggattt ctgtgagcac cacagaattt ggtgaggttt agtaggactc 360

cggaggaata acattcggga aggacaggat gtctgaactg tatgaccagt cctgtcctt 419

<210> 89
<211> 346
<212> DNA
<213> Homo sapiens

<400> 89
taattttgga ttgcctgccc ttggctgaaa tacaggggtg cataccatct tgcagtggct 60

tggctgacat tgcctctttg tcctggcctc tagttttctt ttgatatttc atagctctcc 120

ttagtttact ctgcctggat agaaagttga ccactaactg caggtttaag tactaaactg 180

cagccttttc tgtcgccagc aattaaagac caccaatctt gtttgtccat ctacatggtt 240

tgtcggggac atttaactca tggaggtgct ttagatttca acatcagatg gttgaagctg 300

gaagtttaat tatatgtaga gtgagaaggc agttccagtt ttagca 346

<210> 90
<211> 436
<212> DNA
<213> Homo sapiens

<400> 90
tagatgaaca gcgcacagcc acgataatag aggagttggc agatgccctg acctactgcc 60

atgacaagaa agtgattcac agagatatta agccagagaa cctgctgctg gggttcaggg 120

gtgaggtgaa gattgcagat tttggctggt ctgtgcacac cccctccctg aggaggaaga 180

caatgtgtgg gacactggac tacttgccgc cagaaatgat tgaggggaga acatatgatg 240

aaaaggtgga tttgtggtgc attggagtgc tctgctatga gctgctggtg ggatatccac 300

cctttgagag cgcctcccac agtgagactt acagacgcat cctcaaggta gatgtgaggt 360

ttccactatc aatgcctctg gggggcccggg acttgatttc caggcttctc agataccagc 420

ccttggagag actgcc 436

<210> 91

<211> 438
<212> DNA
<213> Homo sapiens

<400> 91
gagcgtcgta tatggcggga agtccaccat ccgcgaccgc tcctcgggca cggcctccag 60

cgtggccttc accccactcc agggcctgga gattgtgaac ccacaggcgg cagagaagaa 120

ggtggctgag gccaaccaga agtatttctc cagcatggct gagttcctca aggtcaaggg 180

cgagaagagt ggccttatgt ccacctgaat gactgcgtgt gtccaaggtg gcttcccact 240

gaagggacac agaggtccag tccttctgaa gggctaggat cgggttctgg cagggagaac 300

ctgccctgcc actggcccca ttgctgggac tgcccaggga ggaggccttg gaagagtccg 360

gcctggcttc cccaggacc gagatcaccg cccagtatgg gctagagcag gtcttcatca 420

tgccttgtct tttttaac 438

<210> 92
<211> 453
<212> DNA
<213> Homo sapiens

<400> 92
gacgtgaagc aggtcatgcc agaccaaaga gtgatttact actatgcagc tgcccagacc 60

actcacacga catacccgga gggactggaa gttttacatt tttcaagtgg acaaatagaa 120

aaacattacc cagatggaag gaaagaaatc acgtttcctg accagactgt taaaaactta 180

tttcctgatg gacaagaaga aagcattttc ccagatggta caattgtcag agtacaacgt 240

gatggcaaca aactcataga gtttaataat ggccaaagag aactacatac tgcccagttc 300

aagagacggg aatacccaga tggcactgtt aaaaccgtat atgcaaacgg tcatcaagaa 360

acgaagtaca gatccggtcg gataagagtt aaggacaagg agggtaatgt gtcaatggac 420

acggagctgt gacgatcctc atgtgatcat gaa 453

<210> 93
<211> 516
<212> DNA
<213> Homo sapiens

<400> 93
cccaacttcg atggcctgga gagtaatcca tacagaagcc ggaagcagcg ccaggagtgg 60

gaggtgaagg ccctgctaga gaaggtacct gcagagctta tttgtctgga cccacgagcc 120

ctggccgagg tggatgtcat ctccctggag cagggaaaga aggagcagat agagaggctg 180

ggctatgacc cgcaggctaa ggctcccttc cagccaaagc caaagcagaa gggccgcagc 240

tccacggcaa gcctggtgaa gaggaagagg aaggtcatgg atgaggaaca cagggacaag 300

gtccggcaga gccttcagca gcagcatcat aaggaggcga aggccaagcc cacgggggcc 360

cggccatctg ccctggacag atttgtgcgc tgagccagac tccagggttg cctgggaaca 420

gtctctcccc aagatcacct gtagggaaat gagtgttccc tggaacaagg aggtgggggc 480

agtgtggccc cttccccaac tgggggtgga cagctg 516

<210> 94
<211> 467
<212> DNA
<213> Homo sapiens

<400> 94
gcaagtgccg ggcttgatga gctcccaggt gcagatgggc ggccacccga ctgaggtcct 60

gtgcctcatg aacatggtgc tgcctgagga gctgctggac gacgaggagt atgaggagat 120

cgtggaggat gtgcgggacg agtgcagcaa gtacgggctt gtcaagtcca tcgagatccc 180

ccggcctgtg gacggcgtcg aggtgcccgg ctgcggaaag atctttgtgg agttcacctc 240

tgtgtttgac tgccagaaag ccatgcaggg cctgacgggc cgcaagttcg ccaacagagt 300

ggttgtcaca aaatactgtg accccgactc ttatcaccgc cgggacttct ggtagaggcg 360

gctgggggag ggtgggggca gggctggctg gggcttctc cccactcccg ccccccccct 420

tatccccctc tgaagacgat gggcagagga gtgacagccg cagacac 467

<210> 95
<211> 438
<212> DNA
<213> Homo sapiens

<220>
<221> misc_feature
<222> (204)..(204)
<223> n is a, c, g, or t

<220>
<221> misc_feature
<222> (235)..(235)
<223> n is a, c, g, or t

<220>
<221> misc_feature
<222> (257)..(257)
<223> n is a, c, g, or t

<220>
<221> misc_feature
<222> (274)..(274)
<223> n is a, c, g, or t

<220>
<221> misc_feature
<222> (280)..(280)
<223> n is a, c, g, or t

<220>
<221> misc_feature
<222> (334)..(334)
<223> n is a, c, g, or t

<220>

<221> misc_feature
<222> (376)..(376)
<223> n is a, c, g, or t

<400> 95
cgagcccatc ttggctgacg gggctatcct ggacaaaggt cgtgccatgt tcttagtggc 60

caagtgccag gtggcttcag cagcttccta cgatcagccg aagaaagcag aagctctgga 120

ggctgccatc gagaacctca atgaagccaa gaactatttt gcaaaggttg actgcaaaga 180

gcgcatcagg gacgtcgttt actnccaggc cagactctac cataccctgg ggaanaccca 240

ggagaggaac cggtgtncga tgctcttccg gcanctgcan caggagctgc cctctcatgg 300

ggtacccttg ataaaccatc tctagagagg acanccctgc tgggctgctg tgcagagtat 360

aagattttgg acttgntcat gtcccctctc tccctataaa tgatgtattt gtgacaccct 420

atcttgtcaa taaacagc 438

<210> 96
<211> 528
<212> DNA
<213> Homo sapiens

<220>
<221> misc_feature
<222> (300)..(301)
<223> n is a, c, g, or t

<220>
<221> misc_feature
<222> (350)..(350)
<223> n is a, c, g, or t

<400> 96
agagatgatg tttccttgac tgagcatctt gagaaatcaa gatttagttg acaattagac 60

atgaggagaa tagagagcta gaagaccttg cataaactga ttgaccaaga gaatagatac 120

actaatcatg tctacaggaa cagaaaataa aagagacaga gaagagataa taaatctgat 180

ggtaaaaaaa aaaaaaggca ggaagattac gaatggcttc tactctctgg gtgtggtggc 240

gcatgcctgt aatctcagca cttgagctgg ggaggtcaag gctgcagtga gcctaggtan 300

ngccactgca ctccagcctg gacacaagag tgagagagac cctgtctccn aaaaaaaatg 360

atttgatcat agcctcatat atgatttgac tgcccccttg tggtaattta catttgtcaa 420

tggtttaggg agacttgcct gtataccggg atatacaaat ttatgcaagc acgaagacag 480

tttaatttcc agttttaaac ttttgacttg tgtaaaacct tatcccct 528

<210> 97
<211> 108
<212> DNA
<213> Homo sapiens

<400> 97
cctctccaag gagtaccggt tacagtactt ggcctctgaa acagtgctga acttggcttt 60

tgcgcagctc attcttggaa tcccagaaca ggccttaagt cttctcca 108

<210> 98
<211> 310
<212> DNA
<213> Homo sapiens

<400> 98
atgagcccac ctccaaaaaa ctgaagacag aggacagcct catgccagag gaggagttcc 60

tgcgcagaaa caagggtcca gtgtccatca aagtccaggt gcccaacatg caggataaga 120

cggaatggaa actgaatggg caggtgctgg tcttcaccct cccactcacg gaccaggtct 180

ctgtcattaa ggtgaagatt catgaagcca caggcatgcc tgcagggaaa cagaagctac 240

agtatgaggg tatcttcatc aaagattcca actcactggc ttactacaac atggccaatg 300

gcgcagtcat 310

<210> 99
<211> 247
<212> DNA
<213> Homo sapiens

<400> 99
tggagttaga tattttaccc ttacaagaag caaatgctga gctgagtgag aaaagcggta 60

tgttgcaggc agagaagaag ctcttagaag aggatgtcaa acgttggaaa gcacgtaacc 120

agcatctagt aagtcaacag aaagatccag atacagaaga atatcggaag ctcctttctg 180

aaaaggaagt tcatactaag cgtattcaac aattgacaga agaaattggt agacttaaag 240

ctgaaat 247

<210> 100
<211> 532
<212> DNA
<213> Homo sapiens

<400> 100
gtatgcactc aatgcctgca gtgactttgt ctccaaatgg aaaatggcta gcatgccaat 60

caatggacaa ccaaatctta atttttggag cacagaacag atttagatta aataagaaaa 120

aaatttttaa gggccatatg gtagcaggct atgcttgtca ggtggacttt tcaccagaca 180

tgagttatgt gatttcagga gatggaaatg gaaaattaaa catttgggac tggaagacca 240

caaaactcta cagtcgattt aaagctcatg ataaagtgtg tataggtgca gtgtggcatc 300

ctcatgaaac ttctaaggtc ataacatgtg gttgggatgg tctcattaaa ttgtgggatt 360

aatgagatta atccttaaac tagctgggat catttttgat ccattgtcat atttatattt 420

aattattaaa tgtatctgat gataacttga tttacagata atgttgatga cattgaccct 480

ttgtttaaaa aaagaaactg taaatttgac ataatttcat ttgcaacttc at 532

<210> 101
<211> 531
<212> DNA
<213> Homo sapiens

<400> 101
ggcactgtaa tggcgttgac tggcgtcaga agctggactc tcagcgaggg gctgtcattg 60

ccacggagct gaagaacaac agctacaagt tggcccggtg gacctgctgt gctttgctgg 120

ctggatctga gtacctcaag cttggttatg tgtctcggta ccacgtgaaa gactcctcac 180

gccacgtcat cctaggcacc cagcagttca agcctaatga gtttgccagc cagatcaacc 240

tgagcgtgga gaatgcctgg ggcattttac gctgcgtcat tgacatctgc atgaagctgg 300

aggagggcaa atacctcatc ctcaaggacc ccaacaagca ggtcatccgt gtctacagcc 360

tccctgatgg caccttcagc tctgatgaag atgaggagga agaggaggag gaagaagagg 420

aagaagaaga ggaagaaact taaaccagtg atgtggagct ggagtttgtc cttccaccga 480

gactacgagg gcctttgatg cttagtggaa tgtgtgtcta acttgctctc t 531

<210> 102
<211> 315
<212> DNA
<213> Homo sapiens

<220>
<221> misc_feature
<222> (39)..(39)
<223> n is a, c, g, or t

<220>
<221> misc_feature
<222> (45)..(45)
<223> n is a, c, g, or t

<220>
<221> misc_feature
<222> (57)..(57)
<223> n is a, c, g, or t

<220>
<221> misc_feature
<222> (71)..(71)
<223> n is a, c, g, or t

<220>
<221> misc_feature
<222> (81)..(81)
<223> n is a, c, g, or t

<220>
<221> misc_feature
<222> (94)..(94)
<223> n is a, c, g, or t

<400> 102
gcgttttgta tttcagacca ttgccctctt gaacatttnc cgtanccctc aaaactnttc 60

ccagtctgct nacggtttgc nctgtaagtt catncaagtt ccttccccgg ttccctgggc 120

ttgcgtgtca gagctcagtg tccactccat ctggtctgcc gtgctagtgt caaggaccgc 180

gtttcactag aggtggcaag gagctttcgt cccactgacc ccatggaaac ctcttttgga 240

gatttgaact cccaccgtgt gttaaaaggg aaaaagtaac tggaaagggt gccctttaaa 300

acagtctaga gctgg 315

<210> 103
<211> 315
<212> DNA
<213> Homo sapiens

<220>
<221> misc_feature
<222> (204)..(204)
<223> n is a, c, g, or t

<220>
<221> misc_feature
<222> (249)..(249)
<223> n is a, c, g, or t

<220>
<221> misc_feature
<222> (267)..(268)
<223> n is a, c, g, or t

<220>
<221> misc_feature
<222> (271)..(271)
<223> n is a, c, g, or t

<400> 103
ctagaattcg aaggctctct ctttctagag gtgctacata gttggtaatg cttggaatgg 60

caatagggta gaatgattaa tcaaaggcat atcttctata tctgaagagt atccttcctt 120

cagggtttaa tagactgagt cagatgggtc tgatattaat caaaattgtc tcttctgagg 180

accgctgata agcattgact tgcngtcccc taaggaaatc cgagcggcta caaagcgttt 240

ctttacttnt cacttcaatt aatgctnncg nttcgcttgg tgagtgcgta ctttttctac 300

ctgtacacat tcctg 315

<210> 104
<211> 492
<212> DNA
<213> Homo sapiens

<400> 104
ttacctctct ggagacttct tgctggaatg aacagtgtgc tcagggacta tttggaactg 60

gatgtttttg aattatttta tacttagaga tattctgaat tttttgaggg ccttttaaca 120

ctccccgagc tgattgtttg caagtgtgtt tgttccagag tgtggaagta taaagacatg 180

ggcatcacgt aaattggttt tgtttgctat tctgtgtgtc agaaccaacg agtgtaatgg 240

agagggcagg tcatctctta ttgtttctaa aacaacttaa aaggtgtaga ttgggaagag 300

gtgagtgatc cagctttctc cttttggatt gaggctatgt acttggtggg ggcaggggag 360

ggaatatatt ataatactat tcagttggga taatgggaaa aacagagtat atagggtatc 420

tacccagcct agaaagcaca ggaacaatac gtcatatatt tggaacagtt attgtctgtg 480

ccatgacctt ca 492

<210> 105
<211> 499
<212> DNA
<213> Homo sapiens

<220>
<221> misc_feature
<222> (52)..(52)
<223> n is a, c, g, or t

<220>
<221> misc_feature
<222> (58)..(58)
<223> n is a, c, g, or t

<220>
<221> misc_feature
<222> (63)..(63)
<223> n is a, c, g, or t

<220>
<221> misc_feature
<222> (143)..(143)
<223> n is a, c, g, or t

<400> 105
gtgctggagt ccgaagctgg gcaaccccag gctctgggaa gcagtgggac tntgcagntc 60

ccntgaagaa gcagaagctg agggcagaga gcgactttgt gaagtttgac accccttct 120

taccaaagcc cctgttcttc agnaagagcc aagagcagca ctgccaccca ccctccaggc 180

cctgccgtcc agctaaacaa gacaccatcc agctccaaga aagtcacctt tgggctgaac 240

agaaacatga ctgccgaatt caagaagaca gacaagagta tcttggtcag tcccacgggc 300

ccttctcgag tggccttcga ccctgaacag aagcccctcc acggggtgct gaagaccccc 360

accagctcac ctgccagctc acccctggtg gccaagaagc ccctgaccac cacaccaagg 420

agaaggccca gggctatgga tttcttctga ggagcagcag agtcccttgt aaaagactgc 480

ttttgtacag aatgcgcta 499

<210> 106
<211> 566
<212> DNA
<213> Homo sapiens

<220>
<221> misc_feature
<222> (87)..(87)

<223> n is a, c, g, or t

<400> 106
ggcctccgta agatgcttga caattttgac tgttttggag acaaactgtc agatgagtcc 60

atcttcagtg ctttttgtc agttgtnggc aagctgcgac gtggggccaa gcctgagggc 120

aaggctataa tagatgaatt tgagcagaag cttcgggcct gtcataccag aggtttggat 180

ggaatcaagg agcttgagat tggccaagca ggtagccaga gagcgccatc agccaagaaa 240

ccatccactg gttctaggta ccagcctctg gcttctacag cctcagacaa tgactttgtc 300

acaccagagc cccgccgtac tacccgtcgg catccaaaca cccagcagcg agcttccaaa 360

aagaaaccca aagttgtctt ctcaagtgat gagtccagtg aggaagatct ttcagcagag 420

atgacagaag acgagacacc caagaaaaca actcccattc tcagagcatc ggctcgcagg 480

cacagatcct aggaagtctg ttcctgtcct ccctgtgcag ggtatcctgt agggtgacct 540

ggaattcgaa ttctgtttcc cttgta 566

<210> 107
<211> 411
<212> DNA
<213> Homo sapiens

<400> 107
gaaattgcag acttcgaaca acagaaagca aaagaattag ctcgaataga agagtttaaa 60

aaggaggaga tgaggaagct acaaaaggaa cgtaaagttt ttgaaaagta tactacagct 120

gcaagaactt ttccagataa aaaggaacgt gaagaaatac agactttaaa acagcaaata 180

gcagatttac gggaagattt gaaaagaaag gagaccaaat ggtcaagtac acacagccgt 240

ctcagaagcc agatacaaat gttagtcaga gagaacacag acctccggga agaaataaaa 300

gtgatggaaa gattccgact ggatgtctgg aagagagcag aagccataga gagcagcctc 360

gaggtggagg aggagggcaa gcttgcgaac acatctgttc gatttcaaaa c 411

<210> 108
<211> 566
<212> DNA
<213> Homo sapiens

<400> 108
tccactacaa gtagcagccc cagtgactgt atttactgag agcaccacct ctgatgcttc 60

ggaacatgcc tctcaatctg ttccaatggt gactacatcc actggcactt tatctacaac 120

aaatgaaaca gcaacaggtg atgatggaga tgaagtattt gtggaggcag aatctgaagg 180

tattagttca gaagcaggcc tagaaattga tagccagcag gaagaagagc cggttcaagc 240

atctgatgag tcagatctcc cctccaccag ccaggatcct ccttctagct catctgtaga 300

tactagtagt agtcaaccaa agcctttcag acgagtaaga cttcagacaa cattgagaca 360

aggtgtccgt ggtcgtcagt ttaacagaca gagaggtgtg agccatgcaa tgggagggag 420

aggaggaata aacagaggaa atattaatta aatggtctgt aaacaataac aactgtgaat 480

aagattatca aatctgtttt agtgtaatga ttgtcaagtt taaaaacatt tttatatata 540

aactggtata ctcatgtcaa tattct 566

<210> 109
<211> 280
<212> DNA
<213> Homo sapiens

<400> 109
ggggcaactg aaaggtatca gaggaagtat tgagacagtt accggagata cttcttcagc 60

taattatcaa gatactaaag ttaatactaa agagcattca gtcaggaaat agtgttataa 120

aagagaattt acttttcaag ctataatgct attttgatta atgatttttc tttaactgaa 180

gctctaattt aatgggagga aataaaagtt tgaataccag ctttctattt tctaaatatt 240

ggattatatc atgaaaatca agcagtttct atttctttaa 280

<210> 110
<211> 558
<212> DNA
<213> Homo sapiens

<400> 110
cacttagcat catgttctca aggctcatcc atgttgtggc atgtatcagt actgcattcc 60

tttttatggc taaatgatgt ttcattgtat gagtgtgtac cacattttat ttatccattc 120

agcaattaat ggacaggaac aatggctttt aagtattaaa ttgtaagttc aacattaaat 180

gtatccacag ttattgataa tatcaagatt atacatggtg tgaacagaat gctgtgtcga 240

aatggtatgt aaattatttg tcagcatttc atgtaagtga ttattttcta aggacccttc 300

tagccctggt tttaagaaat atgtgaatgt agtattttca tcaataaagt ttaatgcatt 360

aagcattagc ttaaaatttg aatgaaggca gatgtgaaga tatttgccac atgttgtaat 420

aatcatgttt tgaaattatt tcaatatgaa gtatttgaaa aatgtcaata cataaaggaa 480

aggaaatgag tataattaag tcaatatatt tttaaagcaa tttttataat ttagcagaca 540

ctgcatctta atataagt 558

<210> 111
<211> 445
<212> DNA
<213> Homo sapiens

<400> 111
gatgattgat tctggagaca agctgaaact tgaccagact catttagaga cagtaattcc 60

agcaccagga aaaagaattc tagttttaaa tggaggctac agaggaaatg aaggtaccct 120

agaatccatc aatgagaaga ctttttcagc tactatcgtc attgaaactg gcccttttaaa 180

aggacgcaga gttgaaggaa ttcaatatga agacatttct aaacttgcct gagtttgaaa 240

atttgttaac aatacattaa aatcttaaag catcaaattg gtgttcgcca aggcattatg 300

agactctact gtgttagggt atattctttt gtataaaaca aacaggtttt tgaaaatatt 360

actgtatagt tgttcagcta aactttgaga agaatttaat tatgtctcat gaggtatcaa 420

actatgtaat tttgtccttg ttatt 445

<210> 112
<211> 512
<212> DNA
<213> Homo sapiens

<400> 112
gtaccggtta cagtacttgg cctctgaaac agtgctgaac ttggcttttg cgcagctcat 60

tcttggaatc ccagaacagg ccttaagtct tctccacatg gccatcgagc ccatcttggc 120

tgacggggct atcctggaca aaggtcgtgc catgttctta gtggccaagt gccaggtggc 180

ttcagcagct tcctacgatc agccgaagaa agcagaagct ctggaggctg ccatcgagaa 240

cctcaatgaa gccaagaact attttgcaaa ggttgactgc aaagagcgca tcagggacgt 300

cgtttacttc caggccagac tctaccatac cctggggaag acccaggaga ggaaccggtg 360

tgcgatgctc ttccggcagc tgcatcagga gctgccctct catggggtac ccttgataaa 420

ccatctctag agaggacatc cctgctgggc tgctgtgcag agtataagat tttggacttg 480

ttcatgtccc ctctctccct ataaatgatg ta 512

<210> 113
<211> 548
<212> DNA
<213> Homo sapiens

<400> 113
taaaaaggaa ctccaggtct ctctttttca aacactggtg ctgctaatgt ttaatgaggg 60

agaggagttc agtttagaag agatcaagca ggcaactgga atagaggatg gagagttaag 120

gagaacactg cagtcattag cctgtggcaa agctagagtt ctggcgaaaa atccaaaggg 180

caaagacatt gaagatggtg acaagttcat ttgtaatgat gatttcaaac ataaactttt 240

caggataaag atcaatcaaa tccagatgaa agaaacggtt gaagaacaag caagcactac 300

agaaagagta tttcaagaca gacagtatca aattgatgct gcaattgttc gaattatgaa 360

gatgagaaag acacttagcc acaatctcct tgtttcagaa gtgtacaacc agttgaaatt 420

tccagtaaag cctgctgatc ttaagaagag aatagaatct ttaattgacc gggactacat 480

ggaaagagat aaagaaaatc caaaccagta caactatatt gcatagaatg ttggccttgc 540

agcatttg 548

<210> 114
<211> 253
<212> DNA
<213> Homo sapiens

<400> 114
cccccaacta tgaccatgtg gtcctgggcg gtggtcagga agccatggat gtaaccacaa 60

cctccaccag gattggcaag tttgaggcca ggttcttcca tttggccttt gaagaagagt 120

ttggaagagt caagggtcac tttggaccta tcaacagtgt tgccttccat cctgatggca 180

agagctacag cagcggcggc gaagatggtt acgtccgtat ccattacttc gacccacagt 240

acttcgaatt tga 253

<210> 115
<211> 515
<212> DNA
<213> Homo sapiens

<220>
<221> misc_feature
<222> (27)..(28)
<223> n is a, c, g, or t

<220>
<221> misc_feature
<222> (79)..(79)
<223> n is a, c, g, or t

<400> 115
gcttgtacgt acatatgtga actcatnngg aaatattgtg tgtttaatgc aaatgatata 60

ttgaattgtt tagcaattng ttttctttgc ttaacgatgt ttttgagatc tgtgcatgtt 120

acttaatgta gctcaatcca tcttctgtaa ttgctgtata gattgtcatc atatgattac 180

cacattttac ttacgcattt cttttgtgat ggacattaag actgttttta ggttttgcta 240

ttacaaaata ctacacagga gcatcactat gcctgtgtga aagtatatgt atgaaagttt 300

acctagggtt gattcctaga agtggaattg caaagtcata ggatatttat atattggttt 360

ttaataatac ttccaaattg ccctcctgta ctatttactc agtatttttc ttgaggttga 420

tctgaggtct aacattgtta tcctatatca ttttcatccc aagtagtgat atctgtgaaa 480

tcacaggttt gatgtgtgct aattatgtat tcttc 515

<210> 116
<211> 322
<212> DNA
<213> Homo sapiens

<400> 116
tttggcttct cagtagctaa ggggaaggct gatgtcagga gagggagagg gggctgagga 60

ggtagtgctg taggcccagg gggtcaggga aagggagggg ggcatgtgag ggatggaaat 120

gacctcctgg caccaggctc acccacccaa ggccccctgc cccagcactg aatcccagcg 180

ctgccctgag gcccccagcc actccctcca gcagcctggt tcaccacaca aactctgcct 240

ggaccccatt gtctgtctgc ttcccacctg ccctccccac cccctgcccc tcgggcacca 300

gcctgcatat gtgttcactt tt 322

<210> 117
<211> 399
<212> DNA
<213> Homo sapiens

<400> 117
ttgggttacc ggtaaggctc atctttctgt gagattgttt ccataaatgg tcgagccttt 60

gtctcttttt ctgcccacct ctgtcattaa ccagcgatag atttagaatg ggtttcaaag 120

tgtggtttta ttcttggcag tttcatataa ctttaattat taatcagacc actaaattct 180

gtgtggtagg tttctctctg ttccctgtat cctaataaca tctgtggaaa ttccctttac 240

caatttgata agagatcatt gtttagttta ttgagcaaca ttaatttagc acatagtcac 300

ttagaaaaat tttagcaaag gcttattttt aaaccaggga ttggtgaact ttcccgccct 360

tgggccaaat ctggctttct gcctacttag gggcttcag 399

<210> 118
<211> 460
<212> DNA
<213> Homo sapiens

<400> 118
gggatactga gggcagtcct gtggctgagg ggcacagatt gaactgctga actagttgga 60

ggtctagatg aggtgcttta cgcatcagct gccttagaca gcttctagaa aggagcgagc 120

gctacttctt aagtacttaa gtgacattta gataatttat agtaaaactg aaattattat 180

tagccaatgc attggtgcat agaatttact agggctactt ctggaagccc ccaatagaat 240

agcatttcca tgtgcattaa atactttgcc agcactgcct ttgccagcat cctaaatctg 300

gagttttacc aagaaggaaa ctgtatcttt aggttaatcc aagctatgca tttcatatag 360

ctttttcatt taaaacaagg caaagaaaca aattcctatg accaaattgc ttgcctacag 420

ttccctgcag taattgtatg atctcaccca gtgtgcaatt 460

<210> 119
<211> 520
<212> DNA
<213> Homo sapiens

<400> 119
attcttcttg caatctcctg aggattatct gccccatttt taaaacgagg tggaataccc 60

aaggtcatgt agccagtgag tgctctggaa agccaaagca gctcatccct tcctggggac 120

cacactgctc tgctccacca gaccacacta tgaaatagga ataagtgctc ctgttgcagg 180

actgctggga aaacaggtgg ggtgggactt aagtcaccat aattttgaag acttgcatgc 240

agagggctcc aggaattgta gacattaagg aatttcactt tcagttctac ccactactta 300

agtacttgtc atgtactctt agaggaggcc agtaatgatc agaaccattt tactttaaaa 360

ttaataatat tgtattagag aatatattaa atggttatat tgggttatgt taggatatat 420

acttgaatgg aaatacatgt actattagca atcatatttc atttatccct gtaattagac 480

aagaaagcat aatatagctc tactcatggg tacacatacc 520

<210> 120
<211> 565
<212> DNA
<213> Homo sapiens

<400> 120
gtttctcttc cttgaaccag tatttcccaa gattgagtga cagcccagga gaggatgtgg 60

gtctcatgca gagagctggg atcttcttgg ccattaaagt atattgttca cccccacccc 120

gcgagaacca agctcaggag ttgcctgcct ctgcacacat gctgagtgtg ctgtttgtgt 180

atatggcagg accgtcccta tggggcatgt gtgagctccg cagctgggtc aggctcctcg 240

gtccaggttt acttcatccc gcctggacgt gatttggctt tgactgtctc tcctgcagcc 300

caaagcactt cctccctgtg gttgctgtag ctgtctcatt ggagcagtgc ctctccaagt 360

ttgaggattt gaacaaagaa ctgggactgg tgacttgtta atgaacagtt cagagggcag 420

agggccatca tctcagcttg tggagacctt tctttccctg gatgctgctt ctcagctaac 480

tccctctctc ttcgtgtgtg tactcggcct tcagggtttc caccgatttt tacaccttct 540

tcccaccacg atagcttggc tttaa 565

<210> 121
<211> 516
<212> DNA
<213> Homo sapiens

<400> 121
ggtctcagac ccaactaagc taagcaaaca gaagaagcaa cagcgtcttg aacccttgta 60

caaccgctat gaggaaccca atgcctggag aatatctcga gctttccgac ggcgctgaaa 120

ggcaagattg ttcctttgcc tctccagcag cagtagccag ggcttggact tatcgatgac 180

aggctggtcc tgaggataca gctgtcccgt gactgactgt cttaactgag cattttctca 240

actcgactct catttcttcc ctgctggtaa aatagaaaca gggatttaaa cctggctttg 300

gcaagagcct gcagcctcca tcaccccaag tccttgggcc cagttgggag ctcatatcta 360

acacagagac acattgcatc aacttcaaga aagggacaat ttgtgcagct ccaggatggg 420

aaggtggagt gggtgagcat cttgtgcagg gacatggtga gtgccctgat gccccagcta 480

gcaggagcta ctgtgctcat ctaaagtgtt tgcccc 516

<210> 122
<211> 213
<212> DNA
<213> Homo sapiens

<400> 122

gtgagtatga aagcgggccc tggaggttgt ctttgtaaag ggggctgact tggtaactgg 60

tgttaacgac gagaatccca ttggccattt tgattcccct caggctctct ctctgagggc 120

tgatggctct agactgtgtc atggccagga gtctggtggt gatgaaggca caagcaccaa 180

ggtagcttgt gtggcatcat gatccattta gaa 213

<210> 123
<211> 535
<212> DNA
<213> Homo sapiens

<220>
<221> misc_feature
<222> (46)..(46)
<223> n is a, c, g, or t

<220>
<221> misc_feature
<222> (79)..(79)
<223> n is a, c, g, or t

<400> 123
taagtcaact aggtttacag tcccttattt ttaatgccta agtttngaca gcaggaagaa 60

aacaattttt taaaaattnc tcattacata gacgcacaag aatatgtcac ataaagaaaa 120

tgtgtttaga atactggttt tctatttacg catgatattt tcctaagtaa aattgccaag 180

tggacttgga agtccagaaa ggaaaataat ttaaattaat gctggtgatc ttaacaatat 240

tttgtaaaat gatgcttccc ccttctccat ggtctagtca attttgtaca attaggtatc 300

tgactttaca agtttgttat cctttctaat ttttactgaa ctgaaagcac aaagaagact 360

acacagaaaa tctggaaaca gttgcaggtg ttgggaggaa gatgaaatcg agctgtcttt 420

taacttttgt atgtgtttta tcagaatttg ctggactatg ctggcaagga ctttgtttac 480

gatcaaattg tactagtgtc tgcagggttt gtcagtactc gtcaaagcca agtcc 535

<210> 124
<211> 538
<212> DNA
<213> Homo sapiens

<400> 124
accaccatcc tggacgagga accgatcgtg aataggggc tggcagctgc cctgctcctg 60

tgtcagaaca aagggctgct ggagaccaca gtgcagaagg tggcccgggt gaaggccccc 120

aacaagtcgc tgccctcagc cgtgtactgc atcgaggata agatggccat cgatgacaag 180

tacagccgga gggaggaata ccgaggcttc acacaggact tcaaggagaa ggacggctac 240

aaacccgacg ttaagatcga atacgtggat gagacgggcc ggaaactcac acccaaggag 300

gctttccggc agctgtcgca ccgcttccat ggcaagggct caggcaagat gaagacagag 360

cggcggatga agaagctgga cgaggaggcg ctcctgaaga agatgagctc cagcgacacg 420

cccctgggca ccgtggccct gctccaggag aagcagaagg ctcagaagac cccctacatc 480

gtgctcagcg gcagcggcaa gagcatgaac gcgaacacca tcaccaagtg acagcgcc 538

<210> 125
<211> 295
<212> DNA
<213> Homo sapiens

<400> 125
actgtgcctg gccatgtaat agagactttt aatataggag ggtgtaccag aagcaccagt 60

ttcctgtggc aaacagaatt attcctgctg tatttgtaat ctggtgccac gaggtagccc 120

agatcccttc agctctgatg gaagagcatt gcttcagccg taaatggaca cctgcagaaa 180

ccttgcaccg atggatagtc tccctcagct ccgtgccatc gctgcagagg ctgttatgga 240

catcactgca gcccagtggc tctctctcct ggtctccacc atatgagttg gcttc 295

<210> 126
<211> 167
<212> DNA
<213> Homo sapiens

<400> 126
aggaaagaag cacgttccct cagctcttaa ggaggctgac agtttaatga gaagagacac 60

atgaggcttt cgagccatga gcccaggaag taagttggtt tttctagtag ggatgggagt 120

aataagagct ggtgaataat ggagccatag atctgagttc aaatccc 167

<210> 127
<211> 507
<212> DNA
<213> Homo sapiens

<220>
<221> misc_feature
<222> (170)..(170)
<223> n is a, c, g, or t

<220>
<221> misc_feature
<222> (174)..(175)
<223> n is a, c, g, or t

<400> 127
ggaattagtt ctgtccactg tggaggggag aggaaataat gctgtaaatg ttgagttaca 60

gaaagtccaa tgtcaaatat agttttttg tttcctttca aatgtattac agactgtgcc 120

aaaacagtta ccaattcaca ctgtcaatat taaagtatac catagtatan aaannagtca 180

gtacttgctg ttaattttaa tatttctgat ttaacagtta gttattaagt ggtacttcat 240

tgctgtttta gccaacgttt taaaaataat ttgggagttt gactattttg gcttacgtac 300

tcatttcctt ttctctgcta aaaatgtttt gcttgtgtgc gttcctgatt tttgtcttgt 360

ataatcttga tctttgaaaa ccctcaaaca tgtattaaat tgttgtaact ttttttcatt 420

agagggaaga cattaagggg attggggaca tttgtttcac acatctgcag taatatgagt 480

taactaatat ttaacaagct ctttctt 507

<210> 128
<211> 387
<212> DNA
<213> Homo sapiens

<400> 128
gaaaggatta caaccctctg ctggcgcggc agggccagga cgtagcgcca cctcctaacc 60

cagttccaca gaggacgtcc cccacaggcc caaaaaacat gcagacctct ggccggctga 120

gcaatgtggc ccccccctgc attctccgga agaatcctcc atcagcccga aatggcggcc 180

atgagactga tgcccaaatt cttgaactca accaacagct ggtggacttg aagctgacag 240

tggatgggct ggagaaggaa cgtgacttct acttcagcaa acttcgtgac atcgagctca 300

tctgccagga gcatgaaagt gaaaacagcc ctgttatctc aggcatcatt ggcatcctct 360

atgccacaga ggaaggattc gcacccc 387

<210> 129
<211> 412
<212> DNA
<213> Homo sapiens

<400> 129
aatctgatga aagctatact ccaagcaaga tctcagtcag agtaggaaat aattttcaca 60

acctacaaga aattcggcaa cttgagttgg tggaaccaag tggctggatt catgttccct 120

taactgacaa tcataagaag ccaactcgtt cattcatgat acagattgct gttctagcca 180

atcacctgaa tggaagagac acccatatga gacaaattaa aatatacaca ccagtagaag 240

agagctccat tggtaaattt cctagatgta caactataga tttcatgatg tatcgttcaa 300

taaggtgact ttaaaatgag acgaaaatca ttaaacgtat ctttgtttta tcctgtattc 360

aaataatata tcatgtacct ttattgaaca aggcatccgt tatatctaat tt 412

<210> 130
<211> 407
<212> DNA
<213> Homo sapiens

<400> 130
gaaaggatta caaccctctg ctggcgcggc agggccagga cgtagcgcca cctcctaacc 60

caggtgatca gatcttcaac aaatccaaga aactcattgg cacagcagtt ccacagagga 120

cgtcccccac aggcccaaaa aacatgcaga cctctggccg gctgagcaat gtggcccccc 180

cctgcattct ccggaagaat cctccatcag cccgaaatgg cggccatgag actgatgccc 240

aaattcttga actcaaccaa cagctggtgg acttgaagct gacagtggat gggctggaga 300

aggaacgtga cttctacttc agcaaacttc gtgacatcga gctcatctgc caggagcatg 360

aaagtgaaaa cagccctgtt atctcaggca tcattggcat cctctat 407

<210> 131
<211> 566
<212> DNA
<213> Homo sapiens

<220>
<221> misc_feature
<222> (330)..(330)
<223> n is a, c, g, or t

<220>
<221> misc_feature
<222> (398)..(398)
<223> n is a, c, g, or t

<220>
<221> misc_feature
<222> (476)..(476)
<223> n is a, c, g, or t

<220>
<221> misc_feature
<222> (538)..(538)
<223> n is a, c, g, or t

<400> 131
gctttccaat tgggatatcc tgtgatatga ttggcaggct gtccgagtag ttacacttaa 60

taaaatttgg gttcgaaagc agattcctgg aagttcaaaa ataactaaaa cagccccgga 120

taggttagaa agtgaactta aatcattctc agaattagaa aataagaagt tattttttat 180

cttcaaagat aaaaaggggg ccgggcacgg tggctcacgc ctgtaatccc agcactttgg 240

aaagccgagg caagtggatc acgaggtcga gagattgaga ccatcctggc caacatggtg 300

gaaccccgtc tctactaaaa ctacaaaaan ttagccaaga tcacgccatt gcactccagc 360

ctggagacag tgagacgccg tctcaaaaaa aaaaaaanaa agataaaaag agttttgagc 420

aagctcaaag acccgtgtgc cagcagtcag atgccccgtt gggtgcacag gctaangcag 480

cccggcccaa tctggaaacc agtcaggctt ttgccagccc ctaatctaac tgattggnac 540

atgttggtgg ataccccccaa ctttgg 566

<210> 132
<211> 531
<212> DNA
<213> Homo sapiens

<400> 132
ggctgaaagt gttaactgtt ccatactttt agcacaatgt gctgcataag gttacctgtg 60

tacagagttt tactttagat taactaaata ttgcctgggt tcagttttta tttccattct 120

gaaatgcttc ctttttattg tttgaaactg aaaataaaca attgttgaac ccttttgatt 180

ttacctcatt ttaaaactgt tttaatttat tatttggctt gttcttaata ttagtcacta 240

aaagcagtgg gagcattgtc ttatgaaatg cttaggaatc attttatata gtacatgtac 300

aacattaaac gtgtttaaaa aagaaaaagg taccagcgat cacttgtccc ttgccatttt 360

ttcttgtaat tatgttagac aaatcttggc ggcgggggga tcaaaacata attgttttaa 420

ttctacagct gtaggagctt tgtattgctg aactttcatc tggaaaagtt tcacagtgac 480

atttttaaaa gagaattttt ttatctgccg aattctacca gtgtaacctt t 531

<210> 133
<211> 439
<212> DNA
<213> Homo sapiens

<400> 133
tagaatgggt gagtcctacc ccagccccca gccatgggga agggaagcca agccaggcac 60

actgcgccgg agcccgtgct gtcagctctg gaaaagaaca ggtaaagtgc aaggagaatc 120

cagtaagtaa aaatatacca atgactttgt caaatacaca gctgctggct gtcaggagca 180

ggtggctggc tggggcgggt ggcagcagca ccctgggaag tgttgggcga cacagcagag 240

agacaacgga aataaatagg gtgggtgtgg acaggggtcc caccaactgg gccagcacca 300

cccaggacgg gcttctgcct gctttccggc cccatgcccc caagcccccc tcgcctggct 360

ccccagtgct gcagcactga cacaaagcac ccggccgacc gtgttaggaa aggactataa 420

tgtgggagtg gagcaggga 439

<210> 134
<211> 537
<212> DNA
<213> Homo sapiens

<400> 134
tgagcctgtg cgttttgcat actgggttgg tttgctgggg ctgcggtgac agcatatgcc 60

gcgagctggg ctttaacaga gatgtgtgct ctcacagctt tgcaggcggg ggtctgagat 120

cagggtgtcg cgggtggggg gtcactgctg aggccgtgag gggaatctgc tcaggcctgt 180

ccctggcttc tgggggctgc tggtggtatt ttcagttcct tggtgtgtgg atacttcgcc 240

ccatctctgc cttcacctgt gtcctccctg tgtgggtgct ggtgtccaaa atttcccctt 300

ttcgtagtga caccagctgt gttggattgg ggcccaccct gctccagcat ggcctaatct 360

taactaatta catttgcaag gatcttatgt ccacaaaagt cacagtctga ggtgctgggg 420

gttaggactt caatatataa attttgcggt tacacaattc aatccatgac agaatccaaa 480

ggtttactct ggttataaaa acagtacaat aaaatattgt ttatagcctt ccctgta 537

<210> 135
<211> 303
<212> DNA
<213> Homo sapiens

<220>
<221> misc_feature
<222> (39)..(42)
<223> n is a, c, g, or t

<220>
<221> misc_feature
<222> (44)..(44)
<223> n is a, c, g, or t

<220>
<221> misc_feature
<222> (46)..(46)
<223> n is a, c, g, or t

<220>
<221> misc_feature
<222> (48)..(48)
<223> n is a, c, g, or t

<220>
<221> misc_feature
<222> (54)..(54)
<223> n is a, c, g, or t

<220>
<221> misc_feature
<222> (60)..(60)
<223> n is a, c, g, or t

<220>
<221> misc_feature
<222> (64)..(64)
<223> n is a, c, g, or t

<220>
<221> misc_feature
<222> (67)..(67)
<223> n is a, c, g, or t

<220>
<221> misc_feature
<222> (69)..(69)
<223> n is a, c, g, or t

<220>
<221> misc_feature
<222> (71)..(71)
<223> n is a, c, g, or t

<220>
<221> misc_feature
<222> (74)..(74)
<223> n is a, c, g, or t

<220>
<221> misc_feature
<222> (83)..(83)
<223> n is a, c, g, or t

<220>
<221> misc_feature
<222> (102)..(102)
<223> n is a, c, g, or t

<220>
<221> misc_feature
<222> (153)..(153)
<223> n is a, c, g, or t

<220>
<221> misc_feature
<222> (162)..(162)
<223> n is a, c, g, or t

<400> 135
ggtttccagt gtttaacact gtatacaaca gtgacgacnn nntntntntg gggnccccn 60

cggncancng naanactatt tgngcagagt ttgccatcct gngaatgctg ctgcagagct 120

cggaggggcg ctgtgtgtac atcaccccca tgnaggccct gncagagcag gtatgacgtg 180

gcgctgtgtc atgtgaattt cccaagaagc atttcatctg tgattccgta tgaaggcttt 240

ctaagccctg aaatttgcag ggtcatttcc tcagtttgtg tattaaagaa aagctgcccc 300

agc 303

<210> 136
<211> 444
<212> DNA
<213> Homo sapiens

<400> 136
cttcccccag tgattgctgg ttgaattctt atatggacag gtttcccttc ccccagtatc 60

gcacatggtt ctagttagaa tcctgttaga tagtgagcat ctgctgttag ctagacactg 120

ttgaatcaat acagtgaaac agacaggtaa gcaggtggtt ttaatgcctc attctaagtg 180

ctatttaaat tggatcttga aagatgaata aggcctgcct taagttctgc taaaggcaat 240

gttgttatta aggacgggtg aggaaggacc aagaagtctg gctcctgctt ttctacatgc 300

ctgtgaagga gacttttcat gaaactgaat aaaagaaatc ctttccgaga tagaggagat 360

ttgtaggtga atgcagaagt gtatccagct ttgaaccatg cattccatct atattcctcc 420

tgtctgaact catgctcttt tgat 444

<210> 137
<211> 327
<212> DNA
<213> Homo sapiens

<400> 137
tctttttca agtcccttgt gggcaaggat gtggtcgtgg aactaaagaa tgacctgagc 60

atctgtggaa ccctccattc tgtggatcag tatctcaaca tcaaactaac tgacatcagt 120

gtcacagacc ctgagaaata ccctcacatg ttatcagtga agaactgctt cattcggggc 180

tcagtggtcc gatacgtgca gctgccagca gatgaggtcg acacacagtt gctacaggat 240

gcggcaagga aggaagccct gcagcagaaa cagtgatggc tcctcctcct cttcccctcc 300

ctctttcatt ggtgacccat aacccca 327

<210> 138
<211> 199
<212> DNA
<213> Homo sapiens

<400> 138
agggcactaa ggcacagtat ctggcagcca aggccctaaa gaagcagtca tggcgattcc 60

acaccaagta catgatgtgg ttccagaggc acgaggagcc caagaccatc actgacgagt 120

ttgagcaggg cacctacatc tactttgact acgagaagtg ggccagcgg aagaaggaag 180

gcttcacctt tgagtaccg 199

<210> 139
<211> 307
<212> DNA
<213> Homo sapiens

<400> 139
tcatcctgtg aaagtggttt ctctatggaa agctttgttt gcttcctaca aatacatgct 60

tattccttaa gggatgtgtt agagttactg tggatttctc tgttttctgt cttacaagaa 120

acttgtctat gtaccttaat actttgttta ggatgaggag tctttgtgtc cctgtacagt 180

agtctgacgt atttcccctt ctgtccccta gtaagcccag ttgctgtatc tgaacagttt 240

gagctctttt tgtaatatac tctaaacctg ttatttctgt gctaataaac gagatgcaga 300

acccttg 307

<210> 140
<211> 280
<212> DNA
<213> Homo sapiens

<400> 140
aggcaagagc tcagtgatcc aggtgttcca gcagctgggc tgtgcggtga ttgacgtgga 60

cgtgatggcc cggcacgtcg tgcagccagg ataccctgcc caccggcgca tcgtagaggt 120

cttcggcact gaggtcttgc tggagaacgg cgacataaat cgcaaggtcc tgggggacct 180

gatctttaac cagcctgacc ggcggcagct gctcaacgcc atcacccacc ccgagattcg 240

caaggagatg atgaaggaga cgttcaagta cttcctccgg 280

<210> 141
<211> 499
<212> DNA
<213> Homo sapiens

<220>
<221> misc_feature
<222> (116)..(116)
<223> n is a, c, g, or t

<220>

```
<221>  misc_feature
<222>  (119)..(119)
<223>  n is a, c, g, or t

<220>
<221>  misc_feature
<222>  (126)..(127)
<223>  n is a, c, g, or t

<220>
<221>  misc_feature
<222>  (129)..(129)
<223>  n is a, c, g, or t

<220>
<221>  misc_feature
<222>  (131)..(131)
<223>  n is a, c, g, or t

<220>
<221>  misc_feature
<222>  (134)..(135)
<223>  n is a, c, g, or t

<220>
<221>  misc_feature
<222>  (144)..(144)
<223>  n is a, c, g, or t

<220>
<221>  misc_feature
<222>  (151)..(151)
<223>  n is a, c, g, or t

<220>
<221>  misc_feature
<222>  (156)..(156)
<223>  n is a, c, g, or t

<220>
<221>  misc_feature
<222>  (158)..(158)
<223>  n is a, c, g, or t

<220>
<221>  misc_feature
<222>  (162)..(162)
<223>  n is a, c, g, or t

<220>
<221>  misc_feature
<222>  (172)..(172)
<223>  n is a, c, g, or t

<220>
<221>  misc_feature
<222>  (175)..(175)
<223>  n is a, c, g, or t

<220>
<221>  misc_feature
<222>  (177)..(177)
<223>  n is a, c, g, or t

<220>
```

<221> misc_feature
<222> (179)..(179)
<223> n is a, c, g, or t

<220>
<221> misc_feature
<222> (181)..(181)
<223> n is a, c, g, or t

<220>
<221> misc_feature
<222> (183)..(183)
<223> n is a, c, g, or t

<220>
<221> misc_feature
<222> (185)..(185)
<223> n is a, c, g, or t

<220>
<221> misc_feature
<222> (188)..(188)
<223> n is a, c, g, or t

<220>
<221> misc_feature
<222> (191)..(192)
<223> n is a, c, g, or t

<220>
<221> misc_feature
<222> (197)..(197)
<223> n is a, c, g, or t

<220>
<221> misc_feature
<222> (200)..(200)
<223> n is a, c, g, or t

<400> 141
aatattgttt tgtgtgactg aatgtgttag ctcatgcctg taattccagc actttgggag 60

gctgaggcag gaggatcact tgaggctggg agttcaagac cagcctgggc aacatngcna 120

gacccnntnt ntannaaaaa aggnattatg nggtgnantt antccaatgt gnggnantnt 180

ntngntgnga nncctgntcn tcccgttgat caccagggtt gaatttatta aattaggcat 240

attaattcat tgaagttgac tttttagtat cctgctttaa gttgggagtt atgagttgca 300

gaaacgaatt gctgaaatgg aaactcaaaa ggaaaaaatt catgaagata ccaaagaaat 360

taatgagaag agcaatatac tatcaaatga aatgaaagct aagaataaag atgtaaaaga 420

tacagaaaag aaactgaata aaattacaaa atttattgag gagaataaag aaaaatttac 480

acagctagat ttggaagat 499

<210> 142
<211> 345
<212> DNA
<213> Homo sapiens

<400> 142

agttaggtca gtgtctcagc tgctctatcg ggcctatagg tttcttaggc atgtttaaga 60

agctgccact aaggatatgg atatgctgaa cagcttagag cttctcattg aaatttaacc 120

ctaacagctc cactcttgtc tgttttattt ggtttatggc tttataaaac atcatttgac 180

ttttcagttt tcactgcaaa aaaactcaaa agtttgaaaa tcagaactga tgcctttggt 240

ttcatcatct ttaaaatatc tgattctttc caacatccta actataaaaa cagaaggact 300

ttattgccaa tagtctaaat taaaaatcca cctagtaaat accag 345

<210> 143
<211> 515
<212> DNA
<213> Homo sapiens

<400> 143
agctttaatt cgtgccttgt ttcagaacac agaaagaaga gcagctgccc ttgctaaaat 60

taaatagctc catcttctta agaaagctat gtcttgaata tgtggattct tcccttggca 120

taattactcc cttaaagact tctttgaatc gcccattggt tttggtgaac cagtacatct 180

tggaagtttg actttacaga agaacgtcta cctcctggcc tgtacgaggc tttgtttaag 240

aactgtttat taagataaat tgtcaagtaa agcacctcaa ttcattgact ttctagccat 300

cttcctttga ttagctaaca aactgtcagg cagcattatt tcatgctgct tccagagcct 360

ctgggagcta tatacattgt aaatgcaggc cctagctttg gaacgaggaa ttgggagatt 420

ccaggagtca gggtagagaa tttctgagca aatcggagat attttagggg tgtggaggag 480

gggaagggag gaatgggcca ccatatttgg cttac 515

<210> 144
<211> 433
<212> DNA
<213> Homo sapiens

<220>
<221> misc_feature
<222> (26)..(26)
<223> n is a, c, g, or t

<220>
<221> misc_feature
<222> (41)..(41)
<223> n is a, c, g, or t

<220>
<221> misc_feature
<222> (52)..(52)
<223> n is a, c, g, or t

<220>
<221> misc_feature
<222> (370)..(370)
<223> n is a, c, g, or t

<400> 144

acacccagcc agtaatcttt agcttntaga aacaatcaga nttccttaag anttttgcaa 60

ttaggtttag aggagctcac ttgctttttc aaatatgggt tggatatgtc caaaaaaaga 120

gttttgcctc tacaatgaaa aatgattttt accagattat ggaaaggaag attcataaaa 180

atgtgccttt aaaaagcttt tttaaagtgc aagttcacca atttgacttt tgaaaatacg 240

ttcattttag gaattgattt tttaaggaaa gttgtaaaca cagcaaagca cagaattaca 300

ctggttcaga cttgcatctt agcaccaaaa tccactatac aaatgcagac agcataaagt 360

aacctagttn cgctttaaat ttgttgctta ttttattctc ccttataaat gtcattggtc 420

ttttgttaga aca 433

<210> 145
<211> 413
<212> DNA
<213> Homo sapiens

<400> 145
tcacctctag aatagccacc caaagacctt cctgaggctg cctcagaagc accacttgct 60

gttttgaatg actctactag tatgagaagg atgtgaaggt ggttggctgg ttgggcttta 120

acttcctggg attcataatt tttaagcttg gaagatagct gctgttccca tgatgggcac 180

atttcctgag aagcttgaat gactgatgag catagagcac ccctgccttc ctcaggaaac 240

ctgaccggca ggggctctct ggcttcctga aagcttcacc tcttccctcg tttatatctc 300

aactgtaagg gcattttcaa gcttctgttc atggaatgag caactcagac tgtctggagc 360

ttgctgagta caaacacacc accactaagt ttcagaactt tccttaggac ttg 413

<210> 146
<211> 476
<212> DNA
<213> Homo sapiens

<400> 146
gtgccgagaa ctaggacctc gtccccttct ctatcccttc ccctgatcca tctcgtctct 60

gttctccttc ccttttccgt tcccgaagcc ggaaggagcc gaggtaccca cggaaaaagt 120

cgaagctgtc cccggagagt gaggccttca cgaagcggtg gcggaaggag ccgaagttct 180

ctgatcggag gcgtgagcaa gtggcatctc cggaaagagc cgaaacacgg actcgagctt 240

aatcccagga ggggcccgag ggcgagatcc ggaaatctcc ggaaagagcc gaaggccggg 300

acggttagga ttgtcggaag tggccgattg cttggacagg gccggcggag aagatcggag 360

caagtccgtg gaagaagcca aagactggga cggattgaat tgttggaaga acccgaactc 420

gcagagggga ctgggcgcag tggcacacga ggacacacgg aaacctccga acgttg 476

<210> 147
<211> 504
<212> DNA
<213> Homo sapiens

<400> 147
acccggtcta aatccaaatg cttctccagc catccaggag tggctgtcct tttcagtctt 60

gtcttttata taggtagctg agggggaaga tttagaagcc ttgcactcac taaatagatt 120

aaacagagca ggcttgtttg ttgaattgct ccaaagtcca acagacacac actgagcagg 180

tgttttacac tcacattccc tttttgcccc ttaaatagaa agtgcaggta aaggtttata 240

caacaagaaa gcacattgaa aataatttga tactctaaca atccattaac atgtgtaggg 300

gttacggtga ggatcatgtg ttgtattcga aaaacgggga gagggatgct taattggccc 360

tcgcttgcta ttttttttctc atttcttcac aataggaccg tctttggcag cagcaaaatg 420

tatttcagta tggcagtctt tcctctctta cattattggt aagattatac taacaaaatg 480

tttccccttg tacaattatg ctgt 504

<210> 148
<211> 499
<212> DNA
<213> Homo sapiens

<220>
<221> misc_feature
<222> (27)..(27)
<223> n is a, c, g, or t

<220>
<221> misc_feature
<222> (30)..(30)
<223> n is a, c, g, or t

<220>
<221> misc_feature
<222> (34)..(34)
<223> n is a, c, g, or t

<220>
<221> misc_feature
<222> (38)..(38)
<223> n is a, c, g, or t

<220>
<221> misc_feature
<222> (43)..(43)
<223> n is a, c, g, or t

<220>
<221> misc_feature
<222> (46)..(46)
<223> n is a, c, g, or t

<220>
<221> misc_feature
<222> (54)..(54)
<223> n is a, c, g, or t

<220>
<221> misc_feature
<222> (60)..(60)

<223> n is a, c, g, or t

<220>
<221> misc_feature
<222> (137)..(137)
<223> n is a, c, g, or t

<220>
<221> misc_feature
<222> (145)..(145)
<223> n is a, c, g, or t

<220>
<221> misc_feature
<222> (158)..(158)
<223> n is a, c, g, or t

<220>
<221> misc_feature
<222> (168)..(168)
<223> n is a, c, g, or t

<400> 148
gtctggtatt tcaggctgga ctccctngcn gctngctngc acngtnaacc catngttccn 60

gtaagtccca caccaacact ctgcggcctg ctgttcccac aatcagccgg tctccagaca 120

ctgagagggt atatacnttt tcagnctgag agaaggtncc agcattanca aggagttctg 180

ggatcccaca gtttaactgt ctgatcccaa cttccagtga ccatcacatt cacttctgga 240

cagtattcaa cacatctgat aggggcatca tgggtcccaa caagattttc ttgatcagtg 300

ttcaaatcat gcattttcaa ttgatgatct agtcctccac tccaggcatg cgttggatcg 360

tagaaggcgc agtccaggac ggcgccggtg tgctggtact tgagccgcat ggagttggcc 420

ggcacatcgt agagacgcac ggacgtgtcc caggaggaga caagcaggaa ctgggaggtg 480

ttggggctga acttcacgg 499

<210> 149
<211> 529
<212> DNA
<213> Homo sapiens

<220>
<221> misc_feature
<222> (147)..(147)
<223> n is a, c, g, or t

<220>
<221> misc_feature
<222> (150)..(150)
<223> n is a, c, g, or t

<220>
<221> misc_feature
<222> (252)..(252)
<223> n is a, c, g, or t

<400> 149
ttcaagtatt cctattgtac tggagaagct gggaaggaga ttgttaactc tgaggagtgt 60

```
attataaatg agataactgg ggaagaatct gtgaaaaaac ctcaaaccct catggagctg      120

catcaagaaa aactgaaaga ggaaaanaan aagaagaaaa agaaaaagaa gaagcatcga      180

aagagcagtt cagatagtga tgatgaagaa aagaagcatg aaaaattgaa aaaggcactg      240

aacgcagagg angcccgcct tcttcatgtc aaggagacca tgcagattga tgagaggaag      300

cggccttaca atagcatgta tgaaactcga gaacctactg aagaggaaat ggaggcatat      360

agaatgaaac gtcagaggcc agatgacccc atggcctctt tccttggaca gtagcaacta      420

gtcagaagac catccaagat agatgcagct gatacattct tttcagcttc ttattgatga      480

ttgtagatag aaaaatcctt gtttattctt cttgctgcct ggctttaat                  529


<210>  150
<211>  314
<212>  DNA
<213>  Homo sapiens


<220>
<221>  misc_feature
<222>  (133)..(133)
<223>  n is a, c, g, or t

<220>
<221>  misc_feature
<222>  (141)..(141)
<223>  n is a, c, g, or t

<220>
<221>  misc_feature
<222>  (151)..(151)
<223>  n is a, c, g, or t

<400>  150
aaaatgaatg tgaatctgca gatacacaga aagtaagcta ggatgaagaa aaggtgttag       60

ggaagttaac agctgagatg ccaccagagc caagtgtttg agagttttgt gtctcaatac      120

ctaggctcac atntgctttt nagtcattgt ncttccttat tcagcagctg ggagaggtag      180

gaaattagag ccaggaggaa tgtttgacgt tacgtcatta atctcagaca tcttgtagat      240

taggaaaagt gacttggtcg aggtcataca cctatatagt gacaagtaag aagctagagg      300

agtgccgagc gcag                                                        314


<210>  151
<211>  507
<212>  DNA
<213>  Homo sapiens


<220>
<221>  misc_feature
<222>  (48)..(49)
<223>  n is a, c, g, or t

<220>
```

<221> misc_feature
<222> (56)..(56)
<223> n is a, c, g, or t

<220>
<221> misc_feature
<222> (58)..(58)
<223> n is a, c, g, or t

<220>
<221> misc_feature
<222> (70)..(71)
<223> n is a, c, g, or t

<220>
<221> misc_feature
<222> (133)..(133)
<223> n is a, c, g, or t

<400> 151
tatcctaata tttatagcca gcagctgtta ctttgtttgt aaaatttnna aaaaancnaa 60

acctgaagtn ngtttattga aagaattata aggatagctt ctagggatat tagtaataga 120

ttgccatgat ccngaagcct cttctcagaa cactgtgaag ataatactga cagagtagaa 180

tcagtgaaaa cagcctaaca aaacctctga catcagtata tcaactttta acagcccact 240

cactggcttt tctaaactac caaaaaccac ctgtaggata ctgcattaag tcagcatctc 300

agaaacagtg gtgtgagtaa ctgagtcaaa ttgtcaagta ctttggatat acatagtgtc 360

gagctttaga ttggtatgaa attaaacagc aatgcaaata gatttaattc ctatttactt 420

taagaatgaa aaacaatgtt cattttccta cctaaacagg gtggttcaac ataaaggctg 480

atgttgtgtc cgttttgtat tttatct 507

<210> 152
<211> 362
<212> DNA
<213> Homo sapiens

<400> 152
ggataaacta acctgttgat tcaatattat ttgactcctt ttttactacc gatgaccaaa 60

tgaagcagtg taattaatgg aaatagttga gtggactttt tctcagtggt taacatgccc 120

attttaaaga gtaatactta cctttaagaa gaatgttgtt gaactctttg catgttattt 180

agtatgatgt gcagaaaaca cttaagaacg tacctggtct tcatgaattc ctctttggaa 240

ctgggaaaga gatccctgtg gctattaaaa agggggagg gttcttacac accattaatt 300

atgaagcaaa aggtttattt gcttaaaatg tcatttaaag atacttaaac tgcatgcaaa 360

ct 362

<210> 153
<211> 429
<212> DNA
<213> Homo sapiens

<400> 153
aaagcaaata tagttcatga cctctagcaa ctgttgaaaa ctgctcttta gggatgacat 60

gctggccctt ttttttttgt tgttgccaag gctgaagtgc agtggcacca tcacagctca 120

ctgcagcctc gaactcccag gttcaaccct tcctcctgcc tcagccttcc cagtagctgg 180

gactacagat gtacaccatc atgcctagct catttttaaa aaaatttttt atggcattgt 240

atttatcttc tctttataac caggggttga ccagccacag aacttgtaaa gtttttttata 300

tttttaaaag gttgtaagaa atagtagtag ttggctggtc cccgctgctc tcctgcatta 360

tagtatactt ctgttcacct agtttgctag agagaggcag tatagtgtgt atagtgattt 420

ccaaacttt 429

<210> 154
<211> 539
<212> DNA
<213> Homo sapiens

<400> 154
tgttcgaaaa cgtcggcagg ttctaaaaga tctagttaaa gttattcaac aggagtctta 60

cacatataaa gacccaatta cagaatttgt tgaatgttta tatgttaact ttgactttga 120

tggggctcag aaaaagctga gggaatgtga atcagtgctt gtgaatgact tcttcttggt 180

ggcttgtctt gaggatttca ttgaaaatgc ccgtctcttc atatttgaga ctttctgtcg 240

catccaccag tgtatcagca ttaacatgtt ggcagataaa ttgaacatga ctccagaaga 300

agctgaaagg tggattgtaa atttgattag aaatgcaaga ctggatgcca agattgattc 360

taaattaggt catgtggtta tgggtaacaa tgcagtctca ccctatcagc aagtgattga 420

aaagaccaaa agcctttcct ttagaagcca gatgttggcc atgaatattg agaagaaact 480

taatcagaat agcaggtcag aggctcctaa ctgggcaact caagattctg gcttctact 539

<210> 155
<211> 162
<212> DNA
<213> Homo sapiens

<220>
<221> misc_feature
<222> (38)..(38)
<223> n is a, c, g, or t

<400> 155
agcttgcatg gccttgtact gcattggggt gatgtgcntc aaagcctggg ggtggcacgt 60

cccagtattt acggaccttc ttcttcttct cgtggcgggg ggaacgaatc agtccaccgt 120

gctcctcttt agcgcctctg gtcaaaggtt tgctgcgtcg tc 162

<210> 156
<211> 305
<212> DNA

<213> Homo sapiens

<400> 156
tccgtgtttc ctaacgtgga gtgtatgggt ctaagagagc ctgctgtcct ccctgccttc 60

caccttggag aggaggctgg acgcatcagc agtggccagg gcaggtcgca aaatctccca 120

gcctagagac cacacctgaa acggctgaag ccagcttgca caagggctgc tgtccctctg 180

cggcaggcag agctggtggg ggcaggggtc acagagcagt catagacacc atggaccagg 240

gcaggagaag ggcagatggc acatgggcac aacagggcct tgtccttaga gcactggggg 300

gtcat 305

<210> 157
<211> 371
<212> DNA
<213> Homo sapiens

<400> 157
gaaagttcct gccagcagtt tgacaagctg cggaagcggg atgccttcct cgagcagttc 60

cgtaaggagg acatgttcaa ggacaacttt gatgagatgg acaggtctag ggaggttgtt 120

caggagctca ttgatgagta ccatgcggcc acccagccag actacatttc ctggggcacc 180

caggagcagt gatttccctc cccactactc cttctccttc tagatggtaa ccacagcctc 240

gaccatgcct gctccctctg acccagcttc acctcatgga caacccttct tggttcatct 300

ccagcccgtg agctggtcct gcttcctccc ttccatgccc taacttttaa tatgcttgtt 360

cagctctaat a 371

<210> 158
<211> 479
<212> DNA
<213> Homo sapiens

<400> 158
gcagaagaaa gtcttcccac aaccccattt tatttcatat tgggaaaaca caggcaacag 60

caggatgaaa aactaaacga aactttagag aatgagctgg tacaactacc cttaacagaa 120

aacatacccg caattagtga gcttcttcac actccagccc atgtcctgcc atctgctgct 180

ttcctgtgct ccatgtttgt aaattcattg ctgctgtcta aagagactaa gagtgctaag 240

gaaattcctg aagatgtaga tatggaagaa gaaaaagaaa gtgaagattc agatgaagaa 300

aatgatttta ccgaaaaagt ccaggataca agtaacacag gtttaggaga agacattata 360

catcagttgt caaaatctga agaaaaagaa ctgagaaaat ttaggaaaat agactacagc 420

tggatagctg ccctttaagc cttggagatg ggaggatcc ttggactttg tgtttttga 479

<210> 159
<211> 567
<212> DNA
<213> Homo sapiens

<400> 159
ggatgcccct gaagaggttc gtaaccgtga cttcaggaga gagttggaag aaagagagag 60

agctgctgca agagagaaaa atagggatcg tccaacccga gaacatacaa cctcctcttc 120

agtgtcaaaa aagccacggt tagaccagat tcctgccgcc aaccttgatg cagatgaccc 180

tctaacagat gaggaagatg aagattttga agaagaaagt gatgatgatg atactgcagc 240

tcttcttgca gaactggaaa aaattaaaaa agaaagagct gaagagcagg ccaggaagga 300

acaagaacaa aaagctgaag aagagaggat tcgtatggaa aacattctga gcggaaaccc 360

tctccttaat ctcactggcc catcccagcc tcaggccaac ttcaaagtta aaagaaggtg 420

ggatgatgac gttgtcttca agaactgtgc aaaaggtgta gatgaccaga agaaagacaa 480

aagatttgta aatgacacac tgcgatctga atttcacaaa aagttcatgg agaaatatat 540

taaatagtac agttttatgt gcttaat 567

<210> 160
<211> 437
<212> DNA
<213> Homo sapiens

<400> 160
gttggctcag tgtatgctgg ggacaaagaa aaactaacaa gccgacctgc ctttatgata 60

aattctagtg tgcttacaag ggatgacttc ctgaggtgtg atctgtccac cttgaagaac 120

tccacaactg aagaagggga gctgtgagaa cgtggattgt tctacaactt gcacagggta 180

acagaggaag tggctgaggc ctagagtcac gttttccagt tcccttcgca aactatattt 240

cttggaacgc gaaaggaagc tttacctatt tcatagaaga cctggaatcc ataacctcag 300

aaggcaatat tattgataga aaatgtggaa ggatcaggaa gttcttagat tcttggatga 360

cagatgcatg ttgatgccct atggagatgt ccttgtgttt tgaggtcact gaggtaggaa 420

gacctgtcta ctcttgg 437

<210> 161
<211> 351
<212> DNA
<213> Homo sapiens

<400> 161
cacagcaggg cggggtagga ccccagcccc tcccaaaaca gcctctcctt ctcccataga 60

ccccttctt ctccccttcc ccacggtagg aacatagcgt gtttatattt tatggccaaa 120

ctattttgaa ttttgttgtc cggccctcag tgccctgccc tctcccttac caggaccaca 180

gctctgttcc ttcggcctct ggtcctctct ggtcccctcc tgggtttctt acgtagttga 240

tttttcctct ttagtctccc ccgacctgcg cccagccccg tggcccctgc ccctctccta 300

ctctctgtgg cagtttcata tttgctaaga cgaatttgct cattaaacat t 351

<210> 162

<211> 255
<212> DNA
<213> Homo sapiens

<400> 162
attgtcctgc agaggttcat tcccctgacc ctttccccac attggtaaga gtagctgggt 60

tttctaagcc actctctgga atctctttgt gttagggtct cgatttgagg acattcattt 120

cttcagcagc ccattagcaa ctgagagccc agggatgtcc tacaggatag tttcatagtg 180

acaggtggca cttggctaat agaatatggc tgatattgtc attaatcatt ttgtaccttg 240

acatgggttg tctaa 255

<210> 163
<211> 357
<212> DNA
<213> Homo sapiens

<220>
<221> misc_feature
<222> (83)..(84)
<223> n is a, c, g, or t

<220>
<221> misc_feature
<222> (86)..(87)
<223> n is a, c, g, or t

<400> 163
atagtgaatt gttcccaatg ttgaaatgga cgtgtaagcc tttgagctag cttggagtcg 60

aatacactat ttttcactca cannanntat tcatctttgt atttaatact atagctctgt 120

caatatcaca tgaggcagtt tttcaaatac gtataaacag aggttgctta ttattaaagg 180

aaagacaaag tgggactctt tatgatgtca tgaccatgat aactaagcac ctaagaaaat 240

tatttaaaat agttatgtgg taggcagaaa gacaaataat ttagtttttt acttttcacc 300

agcatgtatc ttagctacct aaactgaaac atgggaggct gggcttaatt caaaata 357

<210> 164
<211> 385
<212> DNA
<213> Homo sapiens

<400> 164
gcttgtattc aggttcattg gcttttgctg gatgatccac ctaaagaagt tacctaattt 60

ggccttttaa aaaaggtgtt agtgtttatt atagctactt tcaaggaaag tttgaatatg 120

attctagtct ctaaagttct tcacgttttc tgacattccc tggagggtga ctggggaaga 180

attgctccag ggtagaagaa ccaggcccaa gactttacca ttctgatcta gagacaaagg 240

atactcaatg aggagctttt ttcccctctt ggaacaggta aaatgctttt tcttattaat 300

ataattataa aacagtattt tatgtaacag ctattcccat attctaggag tggcctaaga 360

aatgcgtgtt tcagtgacta gatta 385

<210> 165
<211> 481
<212> DNA
<213> Homo sapiens

<220>
<221> misc_feature
<222> (169)..(172)
<223> n is a, c, g, or t

<220>
<221> misc_feature
<222> (349)..(349)
<223> n is a, c, g, or t

<220>
<221> misc_feature
<222> (379)..(379)
<223> n is a, c, g, or t

<220>
<221> misc_feature
<222> (414)..(414)
<223> n is a, c, g, or t

<220>
<221> misc_feature
<222> (426)..(426)
<223> n is a, c, g, or t

<220>
<221> misc_feature
<222> (431)..(431)
<223> n is a, c, g, or t

<220>
<221> misc_feature
<222> (434)..(434)
<223> n is a, c, g, or t

<220>
<221> misc_feature
<222> (449)..(449)
<223> n is a, c, g, or t

<220>
<221> misc_feature
<222> (452)..(454)
<223> n is a, c, g, or t

<400> 165
gtaaaaagga actccaggtc tctctttttc aaacactggt gctgctaatg tttaatgagg 60

gagaggagtt cagtttagaa gagatcaagc aggcaactgg aatagaggat ggagagttaa 120

ggagaacact gcagtcatta gcctgtggca aagctagagt tctggcgann nntccaaagg 180

gcaaagacat tgaagatggt gacaagttca tttgtaatga tgatttcaaa cataaacttt 240

tcaggataaa gatcaatcaa atccagatga aagaaacggt tgaagaacaa gcaagcacta 300

cagaaagagt atttcaagac agacagtatc aaattgatgc tgcaattgnt cgaattatga 360

agatgagaaa gacacttanc cacaatctcc ttgtttcaga agtgtacaac cagntgaaat 420

ttccangtaa ngcntgctga tcttaagang annntagaat ctttaattga ccgggactac 480

a 481

<210> 166
<211> 508
<212> DNA
<213> Homo sapiens

<400> 166
gcagaagatg ggcctccaga actcctgttt attcatggag gacacactgc taagatttca 60

gattttagct ggaaccccaa tgagccttgg gtcatttgct cagtgtctga ggataacatc 120

atgcagatat ggcaaatggc tgaaaatatt tacaatgatg aagagtcaga tgtcacgaca 180

tccgaactgg agggacaagg atcttaaacc caaagtacga gaaatgtttc tgttgaatgt 240

aatgctacat gaatgcttga tttatcaagc gccaaaaagg cattgtatag taggaaatgt 300

aagtggggtg gcttatggct tctttatcct ctgattctag cactttcaag tgagctgttg 360

cgtactgtat catattgtag ctattaggga agagaagaat gttgcttaag aaagaacatc 420

accattgatt ttaaatacaa gtagcagggt attgcctttg attcaactgt tttaagtcct 480

cattttctca aactaagtgc ttgctgtt 508

<210> 167
<211> 328
<212> DNA
<213> Homo sapiens

<220>
<221> misc_feature
<222> (124)..(124)
<223> n is a, c, g, or t

<220>
<221> misc_feature
<222> (205)..(205)
<223> n is a, c, g, or t

<220>
<221> misc_feature
<222> (225)..(225)
<223> n is a, c, g, or t

<220>
<221> misc_feature
<222> (234)..(234)
<223> n is a, c, g, or t

<400> 167
tggggtgtgg gattttccca gtatgtgtcc ctgcaccagg ctgtgggctc tgctgccgag 60

ggaccttgat ggcccccact tcacctccag gtcccagcac tcagcagggc aggggctcag 120

tgcngaaact attttttttg aatgggcttc tcaagttcta atactgggaa attcctgctg 180

cttgcaaaca ctctggaacc aaccnacctg ggtttcagcc cagtncagct gggngactct 240

aggcaagtca ctcgaacctc tgtgtctcaa ttaacttatc tgtaaaaatg gggggaagac 300

cacctaccta atgcagttgt tatgaaga 328

<210> 168
<211> 544
<212> DNA
<213> Homo sapiens

<400> 168
atgtttcctt gggtcagtgg ttttgaggtc cagtagctag gcttttctct tttgtccttc 60

ctgttggaat gaaaacattt cgattttcct tcatctgtga ctggtgccat agacacaggt 120

ttatagtttt aacttacagt attgtttgaa atttacctgt ttttcttgtc aaacctgagc 180

actcctcctg ctgaagtttc ttatttaatt ccagagtact gtcctctact ctaaggcatt 240

acttttaagg gtattatgaa ggcagttttt caaaggatat gaccagttgg ggtaattcaa 300

attaaaaagg aaaagatttg tttggagtaa ctggtgtctc taagggggat ttttagtgtc 360

aagtatggcg gctctttcac ccctccattg agagcccttg ttattcagag ctccaagact 420

agacctggct aacaaacata ggagacaaag ttaggaaaca ttgatacaag ctttgtacag 480

agatttgtac atttgtgtaa taggcctttt catgctttat gtgtagcttt ttacctgtaa 540

cctt 544

<210> 169
<211> 199
<212> DNA
<213> Homo sapiens

<400> 169
agttcacccc tcaaatcctg ggtgcaccc cccaactccc atgcccccaa tgctgaggcc 60

cccacttccc tccgaaggcc cagggaacat acctccccct cccccaacca actgagaagc 120

tgctccctcc cccagcaagc ccagcgccag gtgctcttgc cttttcccac tgagagaagg 180

ctgctctttt gtactgccc 199

<210> 170
<211> 503
<212> DNA
<213> Homo sapiens

<400> 170
agattgaagc caatgctggg caactgtacc tgacaggggt ggtggtactg cacaaggatg 60

tcaacgtggt agtagtggaa gggggcccca aggcccagaa gaaatttaag cgtcttatgc 120

tgcatcggat aaagtgggat gaacagacat ctaacacaaa gggagatgat gatgaggagt 180

ctgatgagga agctgtgaag aaaaccaaca aatgtgtact agtctgggag ggtacagcca 240

aagaccggag ctttggagag atgaagttta aacagtgtcc tacagagaac atggctcgtg 300

agcatttcaa aaagcatggg gctgaacact actgggacct tgcgctgagt gaatctgtgt 360

tagagtccac tgattgagac tactgcaagc ccttgcctct cctcccttgc ctttgtctct 420

tcagtcctct cacttattct atttcccaac cccctcccac ttgtttgtgt gatctcagaa 480

ctgtgccaag cagacactgg gac 503

<210> 171
<211> 458
<212> DNA
<213> Homo sapiens

<220>
<221> misc_feature
<222> (77)..(77)
<223> n is a, c, g, or t

<400> 171
ggaatgagga acacccatct ccttggccct ctccaccctg aagagttagt tattaaaata 60

attggcaagc tcttgcnaat gtcagtcatc cattgttcag aatggaatag caataataca 120

tccctggctg ccctgggctt ggccaggatt actcactgaa ggcctcaggg ttactggcac 180

acactttctt ttcctaataa tcccatcccc tcagctttcc taaggctaga gtgaatttcg 240

tgttccttta gtttacataa gatggtgaac ttggcaaaag ctatcattaa acagaagcta 300

agagaaagcc tatgtcgtgg aatccagaat gggtattgcc attcactgct gtccacagaa 360

gctgtcttga atttctttct gtgtcttttc tttttttttc tttaagactg ttgtttacca 420

gactgggctc tgtggaacac aggtgtcctg ggagatgg 458

<210> 172
<211> 201
<212> DNA
<213> Homo sapiens

<400> 172
ggctggcatc cctgtagagc cagaggggcc acccagggca gtgacattcc agatatgttg 60

ggctcacctc atccttgctg tgagactgga gttccatggg gacatgaagt cagtacaccg 120

cagagctgct cagctgctct acctctcgct gactttttttg ttgcacatat acattttctt 180

tcaattagca tttatttcag c 201

<210> 173
<211> 334
<212> DNA
<213> Homo sapiens

<400> 173
ccctgtccag actcgaggca gtggtaacac tgcacagtgc tatgtggctt ctctttgagg 60

atttttgggt tttgtaacta aattcttgct gccctcatac tttttatgta ttagaatcat 120

attcgtattg ccctttttaaa acattgggat cctccaaagg cctgccccat gtatttaaca 180

gtaatacagg aagcatggca ggcaccatgc aaaccaagga tggatggtgc agtccctgtg 240

tcagtgggcg gtggtttcct gctggcctgg aatcactcat cacctgattg attggctctg 300

tggtcctggg caggtgcctc ataggtgtgt ggat 334

<210> 174
<211> 251
<212> DNA
<213> Homo sapiens

<400> 174
ggcctattct aggtagttcc aaatagtatt tttgttgtca aactttaaaa tttatattaa 60

tttgcaaatg tatgtctctg agtaggactt ggacctttcc tgagatttat tttatccgtg 120

atgtattttt tttaattctt ttgatacaga gaagggtctt ttttttttta agtatttcag 180

tgaaaacttg gtgtaagtct gaacccatct tttgaaatgt attttcttca ttgcaggtcc 240

acctaatcat c 251

<210> 175
<211> 550
<212> DNA
<213> Homo sapiens

<400> 175
gaagaccctt taaagcagtg aatctgaaac aattttcaca cccttaagtg gttgatacgt 60

acctatttta ggtattttga ggtatttacc ataaactaaa tttagaaatt ttttagattc 120

acttgaagta aacattacaa acattggata cggtggggtt ttctttagat tttacttgag 180

agaaggtgag tacaaagcaa tttgcagttg ttgtaatgac aagattactg cgcaagtgtg 240

aatccaaaca gtatagcttt taaattttaa agcatttggt aaattatcgc tgagtttttt 300

tctgttgcca atagcaaact gcttttccat taatggagaa ttcatgcctt tcaagcattt 360

taaatatgac aatatttata aatgtatggt ttggaggaat cgtttaaatt ctctttccta 420

attttctttc ttttgaagat agattctttc aacaagtaat ttgtagtaat gactgtgttg 480

acttcaattt tggagcgcag tagctatgtt aaagatgaac tatttggtct cattgaagcc 540

aacacagaac 550

<210> 176
<211> 130
<212> DNA
<213> Homo sapiens

<400> 176
ctccgaaggc ccagggaaca tacctccccc tcccccaacc aactgagaag ctgctccctc 60

ccccagcaag cccagcgcca ggtgctcttg ccttttccca ctgagagaag gctgctcttt 120

tgtactgccc 130

<210> 177
<211> 501
<212> DNA
<213> Homo sapiens

<400> 177
gcagcttgga gtgctaactg gaagatcaaa atcatgtctc ttgctgataa cctaagagca 60

ttaagctctg agtccagtga ggtcatagca gtgtatgctc cttgccctta cggagtgtct 120

gtttttagaa atttagattt taaaaggcgt gggacatacg accaggccct atagccacat 180

aaatataaat ttcatagaaa aagaataaaa gcggagatat attttttgac acagaggcac 240

ccaaagaaat acaaacattg cctagctgct gccatttctg taactgccca gaagggtgac 300

agatttctga aggggaaagg attcagatat gaccttttct ttagtcccaa ccactagttt 360

taacaaatgt gaattattga aatgtgaagg gacaaaaaga atcatacatt taaactgtct 420

tgttcagcat accaatattg tatgttacaa atcatcattt ctaaatctgg attgattctg 480

ttgtgttttt gactgtttct a 501

<210> 178
<211> 516
<212> DNA
<213> Homo sapiens

<220>
<221> misc_feature
<222> (26)..(26)
<223> n is a, c, g, or t

<220>
<221> misc_feature
<222> (260)..(260)
<223> n is a, c, g, or t

<400> 178
aggctcgtga gccatttgtt tctttngctg gttatagttg ctaattctaa agctgcttca 60

gactgcttca tgaggaggtt aatctacaat taaacaatat ttcctcttgg ccgtccatta 120

ttttctgaag cagatggttc atcatttcct gggctgttaa acaaagcgag gttaaggtta 180

gactcttggg aatcagctag ttttcaatct tattagggtg cagaaggaaa actaataaga 240

aaacctccta atatcatttn tgtgactgta aacaattatt tattagcaaa caattgatcc 300

cagaagggca aattgtttga gtcagtaatg agctgagaaa agacagagca tatctgtgta 360

tttggaaaaa taattgtaac gtaattgcag tgcatttaga caggcatcta tttggacctg 420

tttctatctc taaatgaatt tttggaaaca ttaatgaggt ttacatattt ctctgacatt 480

tatatagttc ttatgtccat ttcagttgac cagccg 516

<210> 179
<211> 328
<212> DNA
<213> Homo sapiens

<400> 179
gcctgcacta gggcaaaggc cagtaggaat agattggagg tgttaaggtg tgaactgtta 60

aggtaagatg ataacttaat gactgattat tggatgtgga gggtgactga gaggatagaa 120

tgagtaccca tgaatagcca tgattcctac cctgtcccag tcatctcttt ccttatccat 180

ctctgaaaca atctgcttac atcctcctca gcaactggaa ttcctcaagt tagttagaca 240

ttctgtgtgc tgtgtggtct ctcactgccc ccccactccc cacccctcca caagccattg 300

attcattcat ccagttcaat aaatcttg 328

<210> 180
<211> 511
<212> DNA
<213> Homo sapiens

<400> 180
ggcagacgtg gaaggacttt gaggtccggc atggcaatga ggacaccatc aaggaaatgc 60

tgcgtatccg gcgcagcgtg caggccacgt acaacacgca ggtcaacttc atggcctcgc 120

agatgctcaa ggtctcgggc atcccgggca ccgtgtctga cctggcccct gggcagagtg 180

gcatggacga catgaagctg ctggaacagc gggcagagca gctggcggct gaggcggagc 240

gtgaccagcc cttgcgcgcc cagagcaaga tcctgttcgt gaggagtgac gcctcccggg 300

aggagctggc agagctggca cagcaggtca accccgagga gatccagctg ggcgaggacg 360

aggacgagga cgagatggac ctggagccca acgaggttcg gctggagcag cagagcgtgc 420

cagccgcagt gtttgggagc ctgaaggaag actgacccgt ccctccccca tccccccctcc 480

ccaccccccat ccccaataca gctacgtttg t 511

<210> 181
<211> 285
<212> DNA
<213> Homo sapiens

<220>
<221> misc_feature
<222> (236)..(236)
<223> n is a, c, g, or t

<400> 181
aaatgatctc agtatcctct ttcttgtatt tttcttaatt caggatctgg gccttttggg 60

ttctgcagcc gtatgttgca aatattttcc tacttttact tgacttttga cttaggatgt 120

cttgttagag aagttcctaa tttaggtgca gtccaatgaa tcaatcttct tctttattgt 180

agcccttttg atgtttaaat ttcccctatt ccagggttat aaactcctat gttgtnttct 240

acaattgcag tgttcaatat agtaccagta actacaggtg gctac 285

<210> 182
<211> 537

<212> DNA
<213> Homo sapiens

<220>
<221> misc_feature
<222> (77)..(77)
<223> n is a, c, g, or t

<220>
<221> misc_feature
<222> (80)..(81)
<223> n is a, c, g, or t

<220>
<221> misc_feature
<222> (87)..(87)
<223> n is a, c, g, or t

<220>
<221> misc_feature
<222> (91)..(91)
<223> n is a, c, g, or t

<220>
<221> misc_feature
<222> (100)..(100)
<223> n is a, c, g, or t

<220>
<221> misc_feature
<222> (102)..(102)
<223> n is a, c, g, or t

<220>
<221> misc_feature
<222> (104)..(104)
<223> n is a, c, g, or t

<220>
<221> misc_feature
<222> (109)..(109)
<223> n is a, c, g, or t

<220>
<221> misc_feature
<222> (111)..(111)
<223> n is a, c, g, or t

<220>
<221> misc_feature
<222> (114)..(114)
<223> n is a, c, g, or t

<220>
<221> misc_feature
<222> (117)..(117)
<223> n is a, c, g, or t

<220>
<221> misc_feature
<222> (119)..(119)
<223> n is a, c, g, or t

<220>

<221> misc_feature
<222> (137)..(137)
<223> n is a, c, g, or t

<220>
<221> misc_feature
<222> (139)..(139)
<223> n is a, c, g, or t

<220>
<221> misc_feature
<222> (142)..(142)
<223> n is a, c, g, or t

<220>
<221> misc_feature
<222> (144)..(144)
<223> n is a, c, g, or t

<220>
<221> misc_feature
<222> (146)..(146)
<223> n is a, c, g, or t

<220>
<221> misc_feature
<222> (148)..(148)
<223> n is a, c, g, or t

<220>
<221> misc_feature
<222> (151)..(151)
<223> n is a, c, g, or t

<220>
<221> misc_feature
<222> (381)..(381)
<223> n is a, c, g, or t

<400> 182
ttcacaaatg atatcacctc cttgggaaac tgttagttaa taccttacct ttagaaaagg 60

catagtaatc atagccngtn ngttttntga ngttgggcan tngntatanc ntgnggnanc 120

cacatttgga agtcctntnc anantntnct nactttaact tcattatgaa ggacacctgt 180

aagtggcatg tttaataaaa gataccagat taaaaggcaa tgtactatct tggaaagagc 240

cagacatctg agttttaatc tcagttttag ccctctgatg tagaactatt gagggttata 300

gactggtata taatgttctt ggtaagaagt acttgataaa tagtattggt tataactaac 360

aaacctgaac aaactgcttt ncttacccac aaggaaaaag aaagtattgg tctttggtta 420

ttcactaagg caagtggatg agtttttcat cagtaagctt aaattattag ggctgtttga 480

tcagtatcca tatttcataa gccttactgt ataagaaact gtattacatc tacttat 537

<210> 183
<211> 444
<212> DNA
<213> Homo sapiens

<400> 183

tgctcactcg tgctgagggt cacatgggct ctgaacctcg ggatcaggat gctgagaaga 60

agaagaagcc ccgcgatgtg gcccgcagag acttggcctt tgaagtccct gagcggggca 120

gtcgacctgc ctccccagct gccaagctgc ccgcctcacc ctcaggctca gagggtctct 180

ccagtgtgtc cagcagcccc acctccagtc ccaagaccaa agtgaccaca gtgacctctg 240

cccagaagtc cagtcagatt ggaagttctc agctgctgaa gagacatgtg cagcggacag 300

aagctgtgct gacccacaaa caagctcaag ttcccatttc atcagaacca ccagaggaag 360

gagagaaaga ggatcttagg gttcagctga agcgacacca tccctcgagt cccccttcctg 420

tctactcttg gtttcaccac taga 444

<210> 184
<211> 281
<212> DNA
<213> Homo sapiens

<220>
<221> misc_feature
<222> (34)..(34)
<223> n is a, c, g, or t

<400> 184
gcctggcaca gcgttcaggt gttccgtgtc cccntctcct ttccctctcc ccatctcacc 60

cctggtctgg gtgtgggggt gcagctgtga gtagcacaga caggacccct gccccgtggc 120

gtggacattc ttgttggggc cgggtcaaag agacagtcaa caggtgaact ctgtcctgcg 180

tctagcggtg ctaagtcaac accaagaaga aaaagaaagg gggtggcggt gaggcagcat 240

taggtgctga tttaactaag gcacgtggat actcgggggg c 281

<210> 185
<211> 558
<212> DNA
<213> Homo sapiens

<400> 185
ccggctccat tttacttgtt tgatgaaatt gaccaggctc tggatgctca gcacagaaag 60

gctgtgtcag atatgattat ggaacttgct gtacatgctc agtttattac aactactttt 120

aggcctgaac tgcttgagtc agctgacaaa ttctatggtg taaagttcag aaataaggtt 180

agtcatattg atgtgatcac agcagagatg gccaaagact ttgtagaaga tgataccaca 240

catggttaat tggaaaatac tacctactgg tttgggagat gtatatagta atatgattct 300

catacccagg aactgtaaat ttaaacctaa atatttggcc aatagttttc agacttaaag 360

catcatagtc cttttatatt tgtctttgta ttttataaga tactctgtaa tgtcatgttt 420

gtactgatag tttaagaatt taatttcctg tacaactttt tgtaaaatgt tctgctccta 480

ttttaaatgt tttgaaacat gctaaatatt ctttcctaat tattttatca cttatactac 540

cttttttata gcttcaat 558

<210> 186
<211> 489
<212> DNA
<213> Homo sapiens

<220>
<221> misc_feature
<222> (345)..(348)
<223> n is a, c, g, or t

<400> 186
agaagagtct gatgctgtta ctttgcgatc ctgctcttgg tttccactgt ccaggccata 60

caactctgtc tactcccatt agtatttacc aacaggtgtt aaaggaaagc tgaacaagca 120

gcctactcac cgatctgcta ggtggtcttc catgttcatg gtggcttctg cagtttcatt 180

gagagtaaca ttgctgttaa tacagataga tccagtagta gagacatcat cggtccaaaa 240

gactttgcat ctcattagtt gtccatagtg actaccagga ggtggctggc atctataacg 300

tgtgggacgt ttttatgtaa agaactgcct gtgttttttt ttttnnnnaa ccaaagacac 360

tgaccataga ttgacttata cttttataag tctaattgaa atacttgggg tactatgaaa 420

ggactgttcc ttgagtggct acgattagaa aatcaggact aacaaacttt tctttgtctg 480

gaagcatat 489

<210> 187
<211> 160
<212> DNA
<213> Homo sapiens

<400> 187
gtatcgcagt gttgggtaag actttgaaca agcttgttct caggctttga taagtagtgc 60

tgtttgccag ctgtatatta tccctaaaaa taagtaataa ggtatatatg gtacatattt 120

tgacatgcat atacatattt gcatcctgac taggctgccc 160

<210> 188
<211> 469
<212> DNA
<213> Homo sapiens

<400> 188
aaatcatgtc gtgacacgtt tccctgtctg tgtcttttca gaggctggct agtatttcac 60

tgagtagatg tatgaagatg tatttcacca ccttctgtta ggtagatagc tgggttctat 120

tttggttttt gatattttaa ataatgctct ggtgaacact tgtttattct tgtgtacctc 180

tgggagcatt tctccaggcc agagaataag tagaggatgt gtacctctaa tatctgggca 240

gctgctcact aactgccctt caaaaagttt ttgctacttt acaattctag ccacggtgat 300

gaaaatatgt ttcatatttt gtggaaatca aaattttaac tttattaatc tgggtgaaaa 360

atgaaattgt tttaatgtgc attttcctga ttacttaaga agttttatgt cttttatgaa 420

atgtttgtca tactctttga attatagtgg ctttgtatat tcagatgtt 469

<210> 189
<211> 556
<212> DNA
<213> Homo sapiens

<400> 189
gggagttagg accaccaatg gattgtggaa aatggagcca tgacaagaac aaagccactg 60

actgagatgg agtgagctga gacagataag agaatacctt gtctcaccta tcctgccctc 120

acatcttcca ccagcacctt actgcccagg cctatctgga agccacctca ccaaggacct 180

tggaagagca agggacagtg aggcaggaga agaacaagaa atggatgtaa gcctggccca 240

taatgtgaac ataagtaatc actaatgctc aacaatttat ccattcaatc atttattcat 300

tgggttgtca gatagtctat gtatgtgtaa aacaatctgt tttggcttta tgtgcaaaat 360

ctgttatagc tttaaaatat atctggaact ttttagatta ttccaagcct tattttgagt 420

aaatatttgt tacttttagt tctataagtg aggaagagtt tatggcaaag atttttggca 480

ctttgttttc aagatggtgt tatcttttga attcttgata aatgactgtt tttttctgcc 540

taatagtaac tggtta 556

<210> 190
<211> 517
<212> DNA
<213> Homo sapiens

<400> 190
ttctctcgtg ggtaacgctg tagccaggca ggaccagcct cttctgggag acccctctcc 60

ccacgtaggg tttgtgtagt gctcccacat cctgcttatt gcctgccacc cctgcttctc 120

gcctggacct cttggtattc cgtgtacacc atccttgctg tttcttgcct ctgtgccttc 180

aacctgctgc tcctggcctg ggataccttt tattttcttt tatttcttct tccttttttt 240

tttttttttt tttttttaac ctacaactag ctctcagttc aggcactgtc taaagcccca 300

ggctgggtta ggtggtctag gataccaact ccccctcaac attttacctt aatccaacag 360

tacagtaatt agccgaaact tgcctggttt ttcctactag actgtaagcc ctctagggac 420

agggacagtg tcttattcat ctctgtgttc ttagtgcaga gcctggcccg ctgttggtgt 480

ttaataaagt gtgttgatct gaaatgcccc agaccaa 517

<210> 191
<211> 358
<212> DNA
<213> Homo sapiens

<400> 191
gctgcacaga cgtgctcagc agggctacag gtgctcaagc cctattgggg atgggtgggg 60

agtggggcag cggcgtttgc caccttaaga attggggcca aagccactga tgtttatttg 120

acagtgacac tgcactgggt acttaaagaa attatttccc gttgtaatta taattactgc 180

ttattaagga aaatatggga attttagaaa gaatcaagtt tgccacccaa atgctaccac 240

tgttaatctt ttggtgttaa atgttccccc tagacatttc tgtgcataga tttttggtgt 300

gtttacatag tcgttattct gtatatacaa ttttatgtcc cttttgtact taacgtat 358

<210> 192
<211> 573
<212> DNA
<213> Homo sapiens

<400> 192
gttaacacgt attctcatgg tgagggaggt acatgaagaa cttgccaaag ccaaatctga 60

agactctgat gttgaattat cagattaaaa cggaagtgag gttcttattt tcatacatat 120

tggtatgcac caaactgtga atgcatccag ctgttggaaa atgatgtata agtctaagtc 180

ctcttgactt gaccataaga tcatggaaaa cagatgactt gtgaacccca cagtgtggat 240

gtgcaaatga aaattgaagg aaagaatatg aactgagaaa tgttctttgg cagtgatata 300

gttcttagac atcttcagaa tgactaattt ctccgagtgg tgcataatct tattttgttt 360

gggagtaaca aatcgtggaa tatttttaag gaaaactgtt gtataaaact ttaccatagt 420

aaccttagac cttagagagg tagctttgga gtgaaacttt ggctgcaata ggctactttg 480

gcaagccctc cgtaaaagtc agaggagaga tcagtacaga gctaagagtg acatcaaatg 540

aggactgtgg gacccagatt tgaagaccca ata 573

<210> 193
<211> 409
<212> DNA
<213> Homo sapiens

<400> 193
gtagatccct tcagttcaaa acataatgtg attgtgggca gaaatggatc tggaaaaagt 60

aacttttttt atgcaattca gtttgttctc agtgatgagt ttagtcatct tcgtccagaa 120

cagcggttgg ctttattgca tgaaggtact ggtcctcgtg ttatttctgc ttttgtggag 180

attatttttg ataattcaga caaccggtta ccaatcgata aagaggaagt ttcacttcga 240

agagttattg gtgccaaaaa ggatcagtat ttcttagaca agaagatggt cacgaaaaat 300

gatgtgatga acctccttga aagcgctggt tttctcgaaa gcaatcctta ttatattgtt 360

aaacaaggaa agatcaacca gatggcaaca gcaccagatt ctcagagat 409

<210> 194
<211> 534
<212> DNA
<213> Homo sapiens

<400> 194
gacatctcct catgtaattc aggcagttat gggagcccta gagggcctga gagttgctat 60

tggaccatgt agaatgttgc aatattgttt acagggtctg tttcacccag cccggaaagt 120

cagagatgta tattggaaaa tttacaactc catctacatt ggttcccagg acgctctcat 180

agcacattac ccaagaatct acaacgatga taagaacacc tatattcgtt atgaacttga 240

ctatatctta taattttatt gtttattttg tgtttaatgc acagctactt cacaccttaa 300

acttgctttg atttggtgat gtaaactttt aaacattgca gttcagtgta gaactggtca 360

tagaggaaga gctagaaatc cagtagcatg atttttaaat aacctgtctt tgtttttgat 420

gttaaacagt aaatgccagt agtgaccaag aacacagtga ttatatacac tatactggag 480

ggatttcatt tttaattcat ctttatgaag atttagaact cattccttgt gttt 534

<210> 195
<211> 455
<212> DNA
<213> Homo sapiens

<400> 195
atctgattcc agaattgccc tgaagtctgg ctatggaaaa tatcttggta taaattcaga 60

tggacttgtt gttgggcgtt cagatgcaat tggaccaaga gaacaatggg aaccagtctt 120

tcaaaatggg aaaatggctt tgttggcctc aaatagctgc tttattagat gcaatgaagc 180

aggggacata gaagcaaaaa gtaaaacagc aggagaagaa gaaatgatca agattagatc 240

ctgtgctgaa agagaaacca agaaaaaaga tgacattcca gaagaagaca aaggaaatgt 300

aaaacaatgt gaaatcaatt atgtaaagaa atttcagagc ttccaagacc acaaacttaa 360

aataagtaaa gaagacagta aaattcttaa aaaggctcgg aaagatggat tttgcatga 420

gacgcttctg gacaggagag ccaaattgaa agccg 455

<210> 196
<211> 522
<212> DNA
<213> Homo sapiens

<400> 196
gaccttgttg ccccttgaaac ttgaaaatag ggattctggg gtgaggatac aaagacattg 60

tcttgcatat ccataagcag gtcttagagc attattccaa actctagctg tttcagtagt 120

tctatgagga ttgcaagtca taggtgtgtg tggcatatca gtccatctcc ctcatctcca 180

ttctcagttt cttccccaca aaatttggaa tcaaagcttt tatgacgttt gccaattgca 240

gaacttcttc agctaaggtt aatttgacgc tatgataaaa ctgagagatg tcaaaaagcc 300

tcttagaaat tttaatcttg aaagactttt cagggtatct catttttag gtgggggtgg 360

caggtgtatt tctttttaa caaataaaag gcatttaagt aaaactaaaa tgaaaaaagt 420

aggccttctg acattgtgta cttggtggtt ctgtccctct gcctgtaaca aatctcattt 480

ttgttaccaa gaactgtatg aaagaagtaa atccaccccg at 522

<210> 197
<211> 225
<212> DNA
<213> Homo sapiens

<400> 197
acaaggtata tttctctcac ataacccacc ccaccattta tatcactcaa cttgagttat 60

ggaaattatc acttctgtat cccttctgtg gattgttcat tatgtcgttt tgtaatttga 120

gagattttcc ccctcaacaa gaaaacatcg attattttcc ctggttttaa atgtgatatg 180

tgctcagtgc aaaaatttcc agggtttgaa gctgaattta ctagt 225

<210> 198
<211> 570
<212> DNA
<213> Homo sapiens

<400> 198
ccttcgcaac gttgactgtc tttagctttc taatagaagt ttaagaaaag tttccgtttg 60

cacaagaaaa taacgcttgg gcattaaatg aatgccttta tagatagtca cttgtttcta 120

caattcagta tttgatgtgg tcgtgtaaat atgtacaata ttgtaaatac ataaaaaata 180

tacaaatttt tggctgctgt gaagatgtaa ttttatcttt taacatttat aattatatga 240

ggaaatttga cctcagtgat cacgagaaga aagccatgac cgaccaatat gttgacatac 300

tgatcctcta ctctgagtgg ggctaaataa gttattttct ctgaccgcct actggaaata 360

tttttaagtg gaaccaaaat aggcatcctt acaaatcagg aagactgact tgacacgttt 420

gtaaatggta gaacggtggc tactgtgagt ggggagcaga accgcaccac tgttatactg 480

ggataacaat ttttttgaga aggataaagt ggcattattt tattttacaa ggtgcccaga 540

tcccagttat ccttgtatcc atgtaatttc 570

<210> 199
<211> 517
<212> DNA
<213> Homo sapiens

<220>
<221> misc_feature
<222> (77)..(77)
<223> n is a, c, g, or t

<400> 199
gtttctgggt ccttgaaatc acctgttccc agatcagata ttccagagcc tgaaagggaa 60

caaaaacgcc gcaaaantga tactcaccct tctccatcac attcctccac agtaaaggac 120

agtctcatcg aactcaagga atcttcagca aagctctaca ttaatcatac tcctccacca 180

ctgtccaaga gtaaggagag agaaatggac aagaaagatt tggacaagtc aagggaaaga 240

tccagagaaa gagagaaaaa agatgaaaag gacaggaaag agcggaaaag ggatcactca 300

aacaacgacc gtgaagtgcc accggactta accaagagac gtaaagagga gaatggaaca 360

atgggggttt caaaacataa aagtgaaagt ccttgtgaat ctccttatcc aaatgagaaa 420

gacaaggaaa aaaataagtc aaaatcttca ggcaaagaaa aaggcagtga ttcatttaaa 480

tctgagaaga tggataaaat ctcctccggt ggcaaaa 517

<210> 200
<211> 545
<212> DNA
<213> Homo sapiens

<400> 200
ttactatttg tgtcatacct gccttctaat gagaaaacgc ctttacatag ccttgtttca 60

atagtggagg agtgacccac tttcttttct ttccccgcac ataaacttta attggttgta 120

gccatagaca cctatttgat ttagcatgtg ggggataggg tgcacatcca tcatctgtta 180

gttgtcgtaa agatagattg taggtagaat tgaggtccct tttgtaccct tcccagtgct 240

cttcaacttc tttaattagg gaattaaatt cacaaattca gttttctttt ttttgagact 300

gaggcttgca ctccagcctg ggggacagag caagactctg tctcaaacaa aaaaaaaaaa 360

aaggaaaaga taagaaaaac aagtgtttat atgaagtatt ttctcagaaa aagagtaacc 420

caaatacttt tcaagtccat gtggcttgat taatctttaa taaataagaa tattggtgat 480

ttagtaaaaa tggatattct acctagcttt gttaatcaaa taaactcata ttatctctgt 540

tacaa 545

<210> 201
<211> 315
<212> DNA
<213> Homo sapiens

<400> 201
ttgggctgtg ttggaatggg cctgcagccc aacaaacaag ggaactagga ccgacagtga 60

cttcaccagc ttgctaggtc agaatgagag actggtgggt ctgtctacct gtttcttcta 120

caagatccct atttgactgt aaaagtagct aatactcaca tgttctccaa tcccaggtag 180

ccatggtaga gttgggtaga gttgagcagc cgccccagga tccaaatgtg gtgtctgaaa 240

tggaaagaac taaggcaacc aggaaggcac tgatctgcct tataagcaca gtcatctgaa 300

agtcaggcct gctgc 315

<210> 202
<211> 339
<212> DNA
<213> Homo sapiens

<400> 202
atgaccgtcg ccgtgagagg gatgaccggc gtgatctaag agaaagacga gatctaagag 60

acgacaggga ccgaagagga cctccactca gatcagaacg tgaagaagta agttcttgga 120

gacgtgctga tgacaggaaa gatgaccggg tggaagagcg ggaccctcct cgtcgagttc 180

ctcccccagc tctttcaaga gaccgagaaa gagaccgaga ccgagaaaga gaaggtgaaa 240

aagagaaggc ctcatggaga gctgagaaag atagggaatc tctccgtcgt actaaaaatg 300

agactgatga agatggatgg accacagtac gacgttaag 339

<210> 203
<211> 332
<212> DNA
<213> Homo sapiens

<400> 203
gttctgaccc ctggaaagac accaattggc acaccagcca tgaacatggc tacccctact 60

ccaggtcaca taatgagtat gactcctgaa cagcttcagg cttggcggtg ggaaagagaa 120

attgatgaga gaaatcgccc actttctgat gaggaattag atgctatgtt cccagaagga 180

tataaggtac ttcctcctcc agctggttat gttcctattc gaactccagc tcgaaagctg 240

acagctactc caacaccttt gggtggtatg actggtttcc acatgcaaac tgaagatcga 300

actatgaaaa gtgttaatga ccagccatct gg 332

<210> 204
<211> 340
<212> DNA
<213> Homo sapiens

<400> 204
aaaatcaacg tctcttagtg gcccttagag agcttgggga aaccagagaa agagaagaac 60

aagaaacaac ttcatccaaa atcactgagc ttcagctcaa acttgagagt gcccttactg 120

aactagaaca actccgcaaa tcacgacagc atcaaatgca gcttgttgat tccatagttc 180

gtcagcgtga tatgtaccgt attttattgt cacaaacaac aggagttgcc attccattac 240

atgcttcaag cttagatgat gtttctcttg catcaactcc aaaacgtcca agtacatcac 300

agactgtttc cactcctgct ccagtacctg ttattgaatc 340

<210> 205
<211> 487
<212> DNA
<213> Homo sapiens

<220>
<221> misc_feature
<222> (110)..(110)
<223> n is a, c, g, or t

<220>
<221> misc_feature
<222> (128)..(128)
<223> n is a, c, g, or t

<220>
<221> misc_feature

<222> (191)..(191)
<223> n is a, c, g, or t

<400> 205
tacaaactaa ccaaggcatc ggtacattgc cttgaaacag gcgaatatac tcacatcagg 60

aatatcttga ttgtgctaac aaaaatactt ccttggtacc caaaagtttn gaatctgggt 120

caagcttngg aaagaagagt acacaaaatc tgccaagaag aaaaagagaa gaggccagat 180

ctatatgcat nggctatggg ctactctggg cagttgaaaa gtagaaagtc atacatgata 240

cctgaaaatg agtttcatca caaagacccc cctccgagga atgcagttgc cagtgtgcaa 300

aatgggcctg gtggtgggcc ttcttcatca tcaataggaa gtgcatctaa atcggatgaa 360

agcagtactg aggagactga taaatcaagg gagagatctc agtgtggtgt gaaagctgtt 420

aataaagctt ctagtaccac acctaaaggg aattcaagca atggaaatag tggctctaac 480

agcaaca 487

<210> 206
<211> 392
<212> DNA
<213> Homo sapiens

<220>
<221> misc_feature
<222> (262)..(262)
<223> n is a, c, g, or t

<400> 206
cagccagttt aattgttcct ttgtcatcaa tcaagagatt ttgaggtttt aagtctctgt 60

gaagaactct tctagagtga caaaacacaa tcccctgtag gatttggtat aaataactct 120

taacaagtga agaatccatg tactgaccag gagggataga atccaagtat ttcttcagat 180

ccatggaaag aaactcaaag atgagatata acctggaatc ctgcataagc acatcctgaa 240

gactgactat atttggatga cnaagttcct ttaatagaga aatttcccga attgcagtac 300

taggaacccc ttcctcttca ctttctagtc tgattttttt catggctacc acttgacctg 360

tagttttgtg tctaccctta tacacaactc ca 392

<210> 207
<211> 326
<212> DNA
<213> Homo sapiens

<220>
<221> misc_feature
<222> (236)..(236)
<223> n is a, c, g, or t

<400> 207
ggcaacatgg caacaggaat ggaagaggca gcaggagcta caatgcagaa aagccatgga 60

ttaataggaa ctgaagcgcc gggagccatg aagctgcagg acccatgagg cagaaaaagc 120

catgggctag catcgagggg ggcagaaaga agttagtcag tagcagtagg aggagtataa 180

atacagccag aaaggagttg agtcaccaat ttgggaagca ctagagaagg gagcancaga 240

tgcctgcagc tgagggggtg acaagataag ccaggctcta gagctgcttt ggatcatgaa 300

ccattttcaa gtttctgttc ttccat 326

<210> 208
<211> 448
<212> DNA
<213> Homo sapiens

<400> 208
gtttacaggg tctgtttcac ccagcccgga aagtcagaga tgtatattgg aaaatttaca 60

actccatcta cattggttcc caggacgctc tcatagcaca ttacccaaga atctacaacg 120

atgataagaa cacctatatt cgttatgaac ttgactatat cttataattt tattgtttat 180

tttgtgttta atgcacagct acttcacacc ttaaacttgc tttgatttgg tgatgtaaac 240

ttttaaacat tgcagatcag tgtagaactg gtcatagagg aagagctaga aatccagtag 300

catgattttt aaataacctg tctttgtttt tgatgttaaa cagtaaatgc cagtagtgac 360

caagaacaca gtgattatat acactatact ggagggattt catttttaat tcatctttat 420

gaagatttag aactcattcc ttgtgttt 448

<210> 209
<211> 458
<212> DNA
<213> Homo sapiens

<400> 209
ggaggatatc gaacgccaag ttagagaatt gaaaacaaaa atttcagcta tgaaagaaga 60

aaaagaacag cttagtgctg aaagacaaga gcagattaag cagaggacta agttggagct 120

taaagccaag gatttacaag atgaactagc aggcaatagt gaacaaagga aacgtttatt 180

aaaagagagg cagaagctgc ttgaaaaaat agaagaaaag cagaaagaac tggcagaaac 240

agaacccaaa ttcaacagtg tgaaagagaa agaagaacga ggaattgcta gattggctca 300

agctacccag gaaagaacgg atctttatgc aaagcagggt cgaggaagcc agtttacatc 360

aaaagaagaa agggataagt ggattaaaaa ggaactcaag tctttagatc aggctattaa 420

tgacaagaaa agacagattg ctgctataca taaggatt 458

<210> 210
<211> 535
<212> DNA
<213> Homo sapiens

<220>
<221> misc_feature
<222> (425)..(425)

<223> n is a, c, g, or t

<400> 210
aatgctttac tctgactatg tgctattggg ttttatttcc agaaaatata gttctccttt 60

tttctgcatg aaggatacat cgtggtgcca catgctttaa gcaatttaaa caagagagat 120

aagaggaaaa tgcaaccacc acatctgact tgcccaatgt agactttcct ctattagatt 180

gaagtacaca acctaatatg atatattatt ttgtagtatc tcagactttg taaataaata 240

ccattatttt tatatggaaa ttttatagaa gagctatttc tgtatacgta attactcctg 300

attttctgaa attgcttctg gtagataaca gacaagtcct aagcagtgtt ccactaaggg 360

tggttccagg cctgcctgcc gtggagttga ctgggggaat tttacagttt tgcgatccta 420

ggatncgtcc cagacgctca gtcagaagtg ctggaggtgg ggcctgggaa gctgtatttg 480

taatgaactc tggtgttttt tgtccattaa agtgtatctt tgtccatcct ataag 535

<210> 211
<211> 540
<212> DNA
<213> Homo sapiens

<400> 211
tctttccttt cttatcagga gacaatcagc attacaacac ctccttgctt gccgctgcag 60

cagcagcagt ttcagatgat caagacctcc tacactcgtc tcggtttttc ccgtatacct 120

cctcacagat gtttcttgat cagttaagtg caggaggcag tacttctctg ccaaccacca 180

atggaagcag tagtggcagt aacagcagcc tggtttcttc caacagccta agggaaagcc 240

atagccacac cgtcacaaac aggagcagca cggacacggc atccatcttt ggcatcatac 300

cagacattat ttcattggac tgattcccag gccctgctgc tcccatcccc accccagatc 360

gaatgaactt ggcagaaaga agagaacttt gtgctctgtt ttaccttact ctgtttagaa 420

aagtatacaa gcgtgttttt tttccttttt ttagggaaaa aattaaaaga aatgtacaga 480

gaacaaaact atattttcag ttttactttt gtatataaat ctaagactgc ctgtgtgata 540

<210> 212
<211> 499
<212> DNA
<213> Homo sapiens

<400> 212
aagtcgtcta tttctttcta gttgcatcaa cttgcacact ggtgtctaac tatatgtggt 60

attcggaact tgtaattctt attgagcagc cattgcctat attttctcag ctcctcactg 120

atttctttat atgagctcag ctgacaagca aatgcagcta ttttgtttac cctctcttca 180

taccggacca tcaacaacag tgttctaact catccctgat ggtctgtgag atgtagacta 240

ttacacaaga ttcaagttac tcgtaagcaa cttttagata gagttggtcc tgttaggagg 300

agactcttga tgtcaccttc agtatcttga aagcgggtcc cctccccgag gctcttaatt 360

ctttgaaaac ttgatgctgt ttcagctgaa aaattagcaa gactgttaaa aaaaaaaagt 420

tgaggggagc tgtttaagaa actgaaaagt aattgcaaac tacattggat aattgtgact 480

ttcagttgtt gttctgtat 499

<210> 213
<211> 399
<212> DNA
<213> Homo sapiens

<400> 213
ttcttactcc ataccttgtt cgatatggag gacaaataat tggattgtct gataagtctg 60

ccaataaact atccagaaat agcaagtgta atagtcccca ctatacgaat tttatggttt 120

gtataaacac taacattttc cccttctgta gttgtatgaa aaaacaaata ttgttagcat 180

agtagataaa ttgttatgaa ataccagaaa aaaaaatctg tatcttttac tgagaacacc 240

caatacccag ataaatgact gtatcaggat ttcatttgca tgttagtcca cagagttgcc 300

cagaacccta aatttattca taagagaaaa tattgattaa ttattggtca ttcctcataa 360

gtgtagctgt tgatgtgtgc gtctgattat tgcttttttt 399

<210> 214
<211> 396
<212> DNA
<213> Homo sapiens

<220>
<221> misc_feature
<222> (71)..(74)
<223> n is a, c, g, or t

<220>
<221> misc_feature
<222> (76)..(77)
<223> n is a, c, g, or t

<220>
<221> misc_feature
<222> (79)..(84)
<223> n is a, c, g, or t

<400> 214
tggatgggct gagactcctc gaacagatcg aggtggagat tctattggtg aaacaccgac 60

tcctggagcc nnnnanngnn nnnnacggtg ggatgaaaca ccagctagtc agatgggtgg 120

aagcactcca gttctgaccc ctggaaagac accaattggc acaccagcca tgaacatggc 180

tacccctact ccaggtcaca taatgagtat gactcctgaa cagcttcagg cttggcggtg 240

ggaaagagaa attgatgaga gaaatcgccc actttctgat gaggaattag atgctatgtt 300

cccagaagga tataaggtac ttcctcctcc agctggttat gttcctattc gaactccagc 360

tcgaaagctg acagctactc caacaccttt gggtgg 396

<210> 215
<211> 500
<212> DNA
<213> Homo sapiens

<220>
<221> misc_feature
<222> (446)..(446)
<223> n is a, c, g, or t

<400> 215
ttaatagctg acctcccaaa tctgacagga tagacactgc cacgtgcaag gcctgccagc 60

ccctcagacg cacaaaatgc gtaaaacaaa tgcatccttt cctggctaag cgagtattac 120

tctcttagcc ctgcaccaaa cctccaatct agccacattt aactcttcat ttcttagacc 180

cgcagagtgt cttcctgcct ctgagctgtg agtgttgttc cctttgcccg ggatgctctt 240

gtttttaata ccagttcaag tcccactctc tcagtgaagc actcccttcc ccactatagc 300

ctttagtgaa ccctcgtttc ttgcttcttt attatctgta ctgttgtcca cttggcaatt 360

gttcaggcct ctgtgttgtt actgattttt gtatgtatat atatatatat gtcttgtttt 420

tccaactaga ttgtgagctc cttaanggca gagccatgaa ttatacctct ttgtatcccc 480

agtgccttgc atacagtaag 500

<210> 216
<211> 515
<212> DNA
<213> Homo sapiens

<220>
<221> misc_feature
<222> (82)..(82)
<223> n is a, c, g, or t

<400> 216
agtggttttg gtcatgtgtc cacaggagaa actaaccatt cagttgtctt aattttagtt 60

cgttctaccc tgtgaggagt tngtttccat cagttgttga ctttccaaaa tgttgcatta 120

agtaatagtt gtcactctgt tggtctcatg gtcaatatca atcagacttt catgatctct 180

actaattatt agtagagtcc tgtactatgt ctgtaactac taagtttaaa gaaaagcaca 240

tagtcacttc atctcttttt ttcttagcct acgctcactc cccaacccat cccaacattg 300

acatgctatc tgtggacaaa tagcagttct cagaatctag tcaagttgcc atcatccccc 360

ttgccttggc cgttcatagt aggtatgcat atgtttgttt ctgtacagta ctgtgtgtgt 420

gtgtgtatat atatatacat ctgtatgcac acatctttga taaaatagct atttgactag 480

cagggttaaa gtggctttta attacttcgt gagtg 515

<210> 217
<211> 545
<212> DNA

```
<213>  Homo sapiens


<220>
<221>  misc_feature
<222>  (27)..(27)
<223>  n is a, c, g, or t

<220>
<221>  misc_feature
<222>  (29)..(29)
<223>  n is a, c, g, or t

<400>  217
gaaaaatcgg attctttggc agatttncnt ttgagtcaag tgtctgaaat ggagtgaaaa        60

tatatcctaa ctaaattaat gtggaaagag cattttttta gacaatttca attttaaaca       120

cataaaactt tcaagatctt caggactttt taaagcacat ttgaaattat tttagtaaga       180

attttgtttt atcaatagat gttgaattct gtttttaat taaatacaaa gcttagattt        240

cagaaagaga gggaaaatag ctggtggtcc cagagtgtgc tgctgttaat tgtttaacaa       300

aggggaaaat gtacataaac agataaagtt accataaatt ccatgaactt aaatctgtga       360

ttcattgcct taaaactttc tctcttagaa tttccatacc gcatgccaaa ccagtaaaat       420

ggcttttaaa aatgtatagt agacaatgtc agtttgtata aaagtaccaa gtgaaaatat       480

ttattacatg cattggaaaa aaattgttta cctattgaat gttacctgtt tatgtagagc       540

tcttt                                                                   545
```